# EUROPÄISCHE PATENTSCHRIFT

(11) **EP 2 608 752 B1**
(45) Veröffentlichungstag und Bekanntmachung des Hinweises auf die Patenterteilung: **28.01.2015**
(21) Anmeldenummer: 11751550.2
(22) Anmeldetag: 26.08.2011
(51) Int. Cl.: A61F 2/90, A61F 2/852, A61B 17/12

(54) **MEDIZINISCHE VORRICHTUNG UND SYSTEM MIT EINER DERARTIGEN VORRICHTUNG**
MEDICAL DEVICE AND SYSTEM HAVING SUCH A DEVICE
DISPOSITIF MÉDICAL ET SYSTÈME COMPORTANT UN TEL DISPOSITIF

(30) Priorität: 26.08.2010 DE 102010035543
(43) Veröffentlichungstag der Anmeldung: 03.07.2013
(73) Patentinhaber: Acandis GmbH & Co. KG, 76327 Pfinztal (DE)
(72) Erfinder: CATTANEO, Giorgio, 76199 Karlsruhe (DE); BAIDINGER, Otto, 75175 Pforzheim (DE)
(74) Vertreter: Kilchert, Jochen
(86) Internationale Anmeldenummer: PCT/EP2011/004309
(87) Internationale Veröffentlichungsnummer: WO 2012/025247

(56) Entgegenhaltungen:
- EP-A1- 1 645 246
- WO-A1-2011/049823
- DE-A1- 10 301 600
- US-A- 5 064 435
- US-A1- 2007 168 019
- US-B1- 6 348 066

## Beschreibung

Die Erfindung betrifft eine medizinische Vorrichtung gemäß dem Oberbegriff des Patentanspruchs 1. Ferner betrifft die Erfindung ein System mit einer derartigen Vorrichtung. Eine Vorrichtung der eingangs genannten Art ist beispielsweise aus DE 601 28 588 T2 oder der US 2007/0168019 bekannt.

DE 601 28 588 T2 offenbart einen Stent, dessen rohrförmige Struktur durch mehrere Schichten gebildet ist. Die einzelnen Schichten umfassen jeweils ein Drahtgeflecht, wobei die Drahtgeflechte miteinander verflochten sind. Die Drahtgeflechte der einzelnen Schichten sind also mit dem Drahtgeflecht einer benachbarten Schicht jeweils verflochten und bilden somit eine großflächige Verbindung zwischen den Schichten. Insgesamt ergibt sich auf diese Weise eine vergleichsweise komplexe Flechtstruktur der Wandung des Stents.

Durch die komplexe Flechtstruktur wird die Feinmaschigkeit des bekannten Stents erhöht, was zu Vorteilen bei der Behandlung von Aneurysmen führen soll. Konkret wird der Stent dazu eingesetzt, den Blutfluss in ein Aneurysma zu behindern, indem der Stent in einem Blutgefäß im Bereich eines Aneurysmas eingesetzt wird. Dazu wird der Stent in an sich üblicher Weise über ein Zuführsystem an den Behandlungsort geführt. Innerhalb des Zuführsystems liegt der Stent in einem komprimierten Zustand vor. Mit anderen Worten weist der Stent im Zuführsystem einen minimalen Querschnittsdurchmesser auf. Im Bereich des Behandlungsortes wird der Stent aus dem Zuführsystem entlassen. Der Stent wird insbesondere am Behandlungsort expandiert bzw. aufgeweitet, so dass sich der Stent an der Gefäßwand des Blutgefäßes abstützt. Die Aufweitung kann einerseits selbsttätig erfolgen (selbstexpandierbare Stents) oder durch einen Ballon des Zuführsystems (ballonexpandierbare Stents).

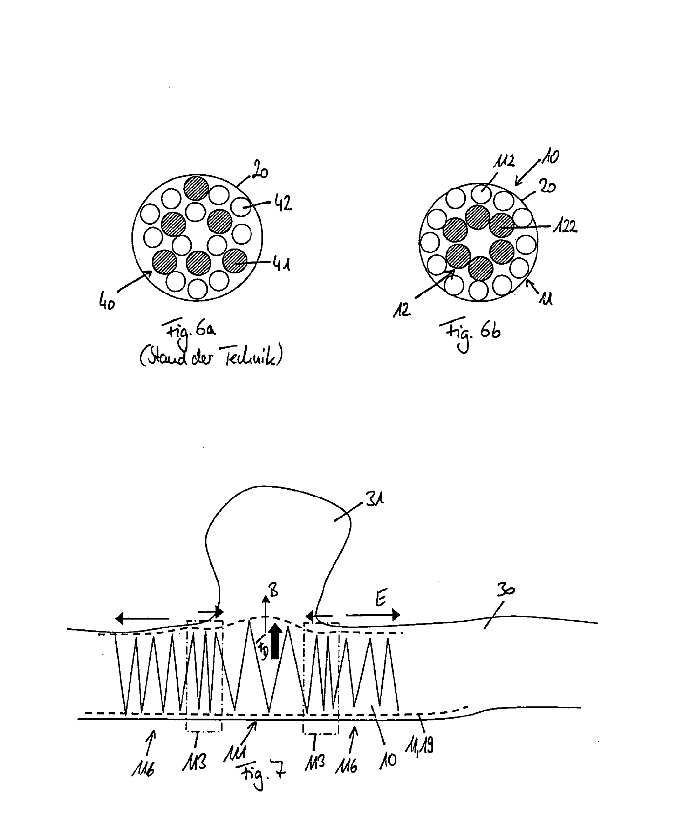

Der bekannte Stent weist Nachteile auf. Durch die komplexe Flechtstruktur, wobei die einzelnen Drahtelemente über mehrere Schichten der Wandungen miteinander verflochten sind, ergibt sich im komprimierten Zustand des bekannten Stents eine Anordnung der einzelnen Drähte innerhalb des Zuführsystems, die einen großen Raumbedarf erfordert. Diese Anordnung zeigt sich insbesondere im Querschnitt des komprimierten, bekannten Stents, der beispielhaft in Figur 6a dargestellt ist. Dabei weisen die ersten Drähte 41 einer ersten Flechtschicht einen größeren Querschnittsdurchmesser als die zweiten Drähte 42 einer zweiten Flechtschicht auf. Die ersten Drähte 41 der ersten Flechtschicht sind mit den zweiten Drähten 42 der zweiten Flechtschicht verflochten. Daraus ergibt sich im komprimierten Zustand die Raum beanspruchende Anordnung gemäß Figur 6a. Zwischen den ersten und zweiten Drähten 41, 42 bleiben vergleichsweise große Freiräume ungenutzt, so dass der bekannte Stent im komprimierten Zustand einen relativ großen Gesamtquerschnittsdurchmesser umfasst. Das beeinflusst auch den kleinstmöglichen Querschnittsdurchmesser für das Zuführsystem. Eine Zufuhr des bekannten Stents in kleinere Gefäße ist somit erschwert.

Durch die Verflechtung der einzelnen Schichten miteinander wird bei dem bekannten Stent überdies die Flexibilität negativ beeinflusst. Insbesondere blockieren sich die miteinander verflochtenen Drähte gegenseitig, so dass der bekannte Stent eine vergleichsweise hohe Steifigkeit bzw. niedrige Flexibilität aufweist.

Im Allgemeinen wirken auf Aneurysmen in Blutgefäßen unterschiedliche physikalische Phänomene, die zu einer Vergrößerung oder sogar einer Ruptur des Aneurysmas führen können. Diese aufgrund der Physiologie des Herzkreislaufsystem bzw. Gefäßsystems bewirkten physikalischen Phänomene umfassen einerseits die Druckübertragung des Blutdrucks in das Aneurysma, andererseits auftretende Schubspannungen, die durch eine Blutströmung innerhalb des Aneurysmas bewirkt sind, und ferner lokale Belastungen der Aneurysmenwand bzw. des Aneurysmenhalses durch lagebedingte, direkte Anströmung einzelner Bereiche des Aneurysmas.

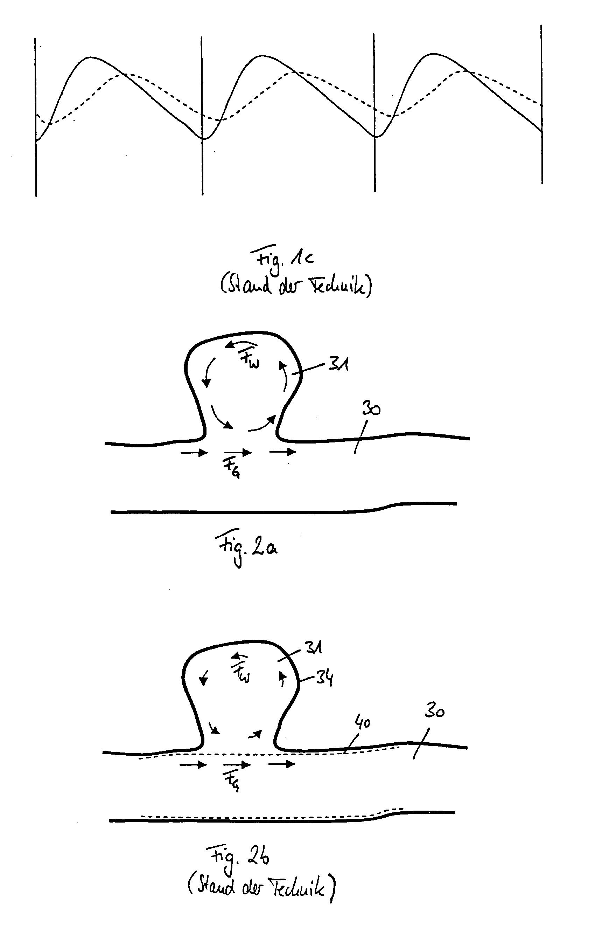

Aneurysmen bilden sich meistens in Arterien, also Blutgefäßen, die vom Herzen wegführen. Die Blutströmung in Arterien ist aufgrund des pulsierenden Pumpverhaltens des Herzens erhöhten Druckschwankungen unterworfen. In der Auswurfphase des Herzens, der Systole, treten die höchsten Druckspitzen im arteriellen Gefäßsystem auf. Ein Druckminimum wird in der Diastole, der Füllungsphase der Herzkammern, erreicht. Die Höhe des lokalen Drucks in begrenzten Gefäßabschnitten bestimmt sich unter anderem durch die Compliance, d.h. die Elastizität der Gefäßwand. Die Druckschwankungen im Gefäß übertragen sich über den Aneurysmenhals in das Aneurysma. Unbehandelt überträgt sich der Druck innerhalb des Blutgefäßes im Wesentlichen vollständig in das Aneurysma, was zu einer erhöhten Belastung der bereits geschwächten Aneurysmenwand führt. Es droht die Gefahr einer Ruptur des Aneurysmas. Durch den Einsatz bekannter Stents, beispielsweise des eingangs genannten Stents gemäß DE 601 28 588 T2, wird der Blutfluss vom Blutgefäß in das Aneurysma behindert. Somit entsteht ein Durchflusswiderstand, der die Strömungsgeschwindigkeit des Blutes in das Aneurysma reduziert. Der Druck innerhalb des Aneurysmas steigt daher während der Systole langsamer und geringer an als innerhalb des Blutgefäßes. Mit anderen Worten erfolgt die Druckübertragung vom Blutgefäß in das Aneurysma durch die Maschen des Stents zeitversetzt und nicht in voller Höhe. Figur 1c zeigt beispielhaft den Druckverlauf des Blutdrucks im Blutgefäß (durchgezogene Linie) und die Druckkurve des Blutdrucks innerhalb des Aneurysmas (gestrichelte Kurve).

Aus der Praxis ist bekannt, dass es einige Stunden oder Tage nach der Behandlung eines Aneurysmas mit bekannten Stents, die die Druckübertragung in das Aneurysma behindern, Risse in der Aneurysmenwand auftreten können, die zu einer Blutung führen. Bei der Behandlung von Aneurysmen mit den bekannten Stents besteht also weiterhin die Gefahr einer Ruptur des Aneurysmas.

Es wird davon ausgegangen, dass durch die Abdeckung des Aneurysmas mit bekannten Stents, die den Blutdruck im Aneurysma beeinflussen, die Zellen der Aneurysmenwand, die unter anderem auch Muskelzellen im Bereich der Muskelschicht (Media) der Gefäßwand, umfassen, durch die nachlassende Beanspruchung degenerieren. Mit anderen Worten sind die Zellen der Aneurysmenwand an die erhöhte Druckbelastung gewöhnt. Durch Wegfall der erhöhten Druckbelastung können sich abbauende Prozesse einstellen, wodurch sich die mechanischen Eigenschaften der Gefäßwand negativ verändern können. Somit steigt die Gefahr einer Ruptur der Gefäßwand im Aneurysmenbereich.

Die Blutströmung innerhalb eines Aneurysmas unterliegt einem weiteren physikalischen Phänomen. Durch das am Aneurysmenhals vorbeiströmende Blut im Blutgefäß werden Scherkräfte an der Grenzfläche zwischen dem Blut im Blutgefäß und dem Blut innerhalb des Aneurysmas wirksam. Die dadurch entstehenden Schubspannungen bewirken eine Verwirbelung des Blutes innerhalb des Aneurysmas. Im Aneurysma bildet sich somit ein Strömungswirbel aus. Die Wirbelbildung im Aneurysma verhindert die Blutgerinnung innerhalb des Aneurysmas. Insbesondere wird durch die Wirbelbildung vermieden, dass sich Staubereiche bilden, die als eine Voraussetzung für die Agglomeration in Form der sogenannten Geldrollenbildung angesehen werden. Der Begriff Geldrollenbildung, auch *Rouleau-Bildung* oder *Pseudoagglutination* genannt, bezeichnet die reversible Bildung von kettenartigen Stapeln roter Blutkörperchen. Hinsichtlich der zuvor genannten Degeneration von Zellen der Aneurysmenwand wird die Behinderung einer tangentialen Blutströmung, die aufgrund von Schubspannungen zu einer Verwirbelung der Blutströmung innerhalb des Aneurysmas führt, als nachteilig angesehen. Bis zu einem gewissen Grad ist allerdings die Verringerung der Schubspannung erforderlich, damit das Blut gerinnen kann. Wenn die Verringerung aber zu stark ist, erfolgt die Thrombenentstehung zu schnell. Der durch den Blutstau entstehende frische Thrombus nimmt stark an Volumen zu, was zu Rissen in der Aneurysmawand führen kann.

Aneurysmen in gekrümmten Blutgefäßabschnitten weisen eine weitere Besonderheit hinsichtlich ihrer Beeinflussung durch die Blutströmung im Blutgefäß auf. Durch die gekrümmte Gefäßform wird das Blut im Blutgefäß in eine gekrümmte Strömungsbahn gelenkt. Bei Ausbildung eines Aneurysmas im Scheitelpunkt der Krümmung ergibt sich eine Strömungskomponente der Blutströmung, die im Wesentlichen direkt in das Aneurysma, insbesondere auf den Aneurysmenhals, gerichtet ist. Das Blut aus dem Blutgefäß strömt also direkt in das Aneurysma hinein. Sobald das einströmende Blut auf die Aneurysmenwand, insbesondere im Aneurysmenkopf, trifft, wird die Blutströmung umgelenkt, wodurch die kinetische Strömungsenergie des Blutes in einen lokalen Druck umgewandelt wird, der die Aneurysmenwand lokal beansprucht. Die lokale Anströmung bzw. der daraus resultierende lokale Druck kann in Verbindung mit der physiologischen Druckwelle, die durch die Systole und die Diastole ausgelöst ist, die Ursache für die Entstehung des Aneurysmas bilden. Eine Verminderung der Druckwelle kann positiv sein, um zu verhindern, dass das Aneurysma weiter wächst, oder um sogar ein Schrumpfen des Aneurysmas zu erreichen. Andererseits kann sich die Reduzierung nachteilig auf die Degeneration der Zellen der Aneurysmawand auswirken.

Durch bekannte Stents, die den Aneurysmenhals abdecken, wird der lokale Druck durch die direkte Einströmung des Blutes in das Aneurysma verringert bzw. vermieden, da die Blutströmung durch den im Gefäß platzierten Stent in die vorgegebene gekrümmte Strömungsbahn gelenkt wird. Somit wird der Anteil der direkt in das Aneurysma gerichteten Strömungskomponente reduziert. Gleichzeitig wird durch die bekannten Stents die Übertragung einer Druckwelle in das Aneurysma vermieden, wodurch die Degenerierung der Zellen der Aneurysmenwand begünstigt wird.

Eine weitere Möglichkeit, die direkte Anströmung eines Aneurysmas zu vermeiden, besteht darin, die Gefäßkrümmung zu beeinflussen. Beispielsweise kann durch einen geeigneten Stent der Krümmungsradius des Gefäßes im Bereich des Aneurysmas reduziert werden. Voraussetzung dafür ist eine Stentstruktur, die eine ausreichend hohe Steifigkeit bzw. Radialkraft aufweist, so dass die Stentstruktur das Blutgefäß in eine gestrecktere bzw. weniger gekrümmte Form zwingt.

Den zuvor beschriebenen medizinischen Anforderungen an die Behandlung von Aneurysmen wird in der Praxis auf unterschiedliche Weise technisch begegnet. Einerseits wird davon ausgegangen, dass eine hohe Feinmaschigkeit, also eine möglichst geringe Maschengröße, eines Aneurysmenstents eine effiziente Behandlung ermöglicht. Die hohe Feinmaschigkeit wird üblicherweise durch eine hohe Anzahl von Drähten erreicht. Um eine geeignete Crimpbarkeit zu erreichen, so dass der Aneurysmenstent in kleine Blutgefäße einführbar ist, weisen die einzelnen Drähte einen vergleichsweise kleinen Querschnittsdurchmesser auf. Derartige Stents bilden daher eine vergleichsweise geringe Radialkraft aus. Das führt dazu, dass derartige Stents keine Beeinflussung der Krümmung eines Blutgefäßes ermöglichen. Ferner weisen Stents mit einer geringen Radialkraft eine geringe Stabilität auf, wodurch die Gefahr besteht, dass sich der Stent unter Einfluss der Blutströmung bzw. des Pulsschlags innerhalb des Blutgefäßes aus seiner ursprünglichen Position entfernt. Es besteht also die Gefahr einer Dislokation des Stents. Insbesondere sind aus der Praxis Fälle bekannt, bei welchen die eingesetzten Stents aus dem Blutgefäß in das Aneurysma gewandert sind und dort weitere Schäden verursacht haben. Aufgrund der vergleichsweise geringen Radialkraft weisen derartige Stents auch eine vergleichsweise kleine Rückstellkraft auf. Reibungskräfte zwischen dem Stent und der Gefäßwand können dazu führen, dass eine Anpassung des Querschnittsdurchmessers des Stents an den Querschnittsdurchmesser des Blutgefäßes, insbesondere unter Einfluss von Systole und Diastole, nicht gewährleistet ist. Bei großen Flechtwinkeln lässt sich die Gitterstruktur leicht stauchen. Dadurch werden die Zellen kleiner und die Gitterstruktur wird dichter. Die Permeabilität sinkt und der Durchflusswiderstand steigt, so dass es zu einer Beeinträchtigung der Strömungsverhältnisse und einem Verschluss von Seitenästen der Gefäße kommen kann.

Bei den bekannten Stents wird die vergleichsweise hohe Feinmaschigkeit auch durch einen hohen Flechtwinkel erzielt. Ein großer Flechtwinkel bewirkt auch eine Erhöhung des Foreshortening. Als Foreshortening wird ein Phänomen verstanden, wobei sich die Gitterstruktur des Stents bei der Expansion, also beim Übergang vom komprimierten in den expandierten Zustand, in axialer Richtung verkürzt. Ein großer Flechtwinkel bewirkt eine vergleichsweise große Verkürzung des Stents bei der Expansion. Die Positionierung derartiger Stents ist erschwert. Es besteht die Gefahr einer Fehlpositionierung des Stents. Der Effekt des Foreshortening ist auch im Zusammenhang mit der Querschnittsänderung des Blutgefäßes während der Systole und Diastole wirksam. Bereits bei vergleichsweise kleinen Durchmesseränderungen kann sich der Stent mit einem großen Flechtwinkel stark verlängern oder verkürzen. Dadurch ändert sich auch die Zellkonfiguration bzw. die Maschengröße des Stents. Die Reproduzierbarkeit der Behandlung ist somit erschwert. Durch einen großen Flechtwinkel wird bei dem bekannten Stent eine hohe Flexibilität bereitgestellt. Die Streckung eines gekrümmten Blutgefäßes zur Verringerung des Impulses auf die Aneurysmawand ist mit derart flexiblen Stents jedoch nicht möglich.

Ein weiterer Nachteil bei aus der Praxis bekannten Stents besteht darin, dass sich die Stents bei einer Streckung zumindest abschnittsweise verengen. Eine derartige Streckung des Stents in axialer Richtung kann durch die Abfolge von Systole und Diastole bewirkt sein. Während der Systole wird einerseits der Gefäßdurchmesser in Abhängigkeit der Gefäßcompliance erweitert. Andererseits erfolgt gleichzeitig eine axiale Streckung des Blutgefäßes. Die axialen Enden eines innerhalb des Blutgefäßes positionierten Stents, der sich gegen die Gefäßwand abstützt, entfernen sich der Streckung des Gefäßes voneinander. Gemäß dem Effekt des Foreshortening bewirkt die Streckung des Stents zumindest abschnittsweise eine Verringerung des Stentdurchmessers. Dabei kann sich die Stentstruktur von dem Aneurysma bzw. vom Aneurysmenhals abheben, wodurch der Effekt der Strömungsbeeinflussung reduziert wird. Außerdem kann durch eine Verjüngung des Stentdurchmessers, ausgelöst durch eine Streckung des Stents, der Kontakt zwischen Stent und Gefäßwand reduziert werden, so dass die Gefahr einer Dislokation des Stents besteht.

Der Erfindung liegt die Aufgabe zugrunde, eine medizinische Vorrichtung anzugeben, die eine effiziente Behandlung von Aneurysmen ermöglicht und eine verbesserte Crimpbarkeit aufweist. Insbesondere soll mit der Vorrichtung eine postoperative Ruptur des Aneurysmas bzw. eine postoperative Schwächung der Aneurysmenwand vermieden werden. Eine weitere Aufgabe der Erfindung besteht darin, ein System mit einer derartigen Vorrichtung anzugeben.

Erfindungsgemäß wird diese Aufgabe im Hinblick auf die Vorrichtung durch den Gegenstand des Patentanspruchs 1 und im Hinblick auf das System durch den Gegenstand des Patentanspruchs 15 gelöst.

Die Erfindung beruht auf dem Gedanken, eine medizinische Vorrichtung mit einem zumindest abschnittsweise rohrförmigen Körper anzugeben, die von einem komprimierten Zustand in einen expandierten Zustand überführbar ist und eine Umfangswandung mit wenigstens einer ersten Gitterstruktur und einer zweiten Gitterstruktur umfasst. Die erste Gitterstruktur und die zweite Gitterstruktur bilden separate Schichten der Umfangswandung. Die separaten Schichten der Umfangswandung sind koaxial ineinander angeordnet. Ferner sind die separaten Schichten der Umfangswandung zumindest punktuell miteinander derart verbunden, dass die erste Gitterstruktur und die zweite Gitterstruktur wenigstens abschnittsweise relativ zueinander bewegbar sind.

Erfindungsgemäß bilden die erste Gitterstruktur und die zweite Gitterstruktur separate Schichten der Umfangswandung. Die Gitterstrukturen sind also nicht wie beim Stand der Technik großflächig miteinander verbunden. Vielmehr erfolgt die Verbindung zwischen den Gitterstrukturen punktuell, so dass zwischen den Schichten bzw. Gitterstrukturen eine Relativbewegung ermöglicht ist.

Die punktuelle Verbindung bedeutet, dass der lose aufeinander angeordneten Bereich der beiden Gitterstrukturen flächenmäßig größer ist als der wenigstens eine Verbindungsbereich bzw. die punktuellen Verbindungsbereiche zwischen den beiden Gitterstrukturen derart, dass eine Relativbewegung zwischen den beiden Gitterstrukturen möglich ist. Der wenigstens eine Verbindungsbereich bzw. die punktuellen Verbindungsbereiche bilden keine kontinuierliche Gitterstruktur. Der Verbindungsbereich ist vielmehr lokal begrenzt. Bspw. kann der Verbindungsbereich jeweils einzelne Zellen bzw. Maschen der beiden Gitterstrukturen umfassen, in deren Bereich die mechanische Verbindung besteht. Der punktuelle Verbindungsbereich kann auf höchstens 4 Zellen oder Maschen der ersten und/oder zweiten Gitterstruktur begrenzt sein, wobei entweder 4 oder weniger Zellen der ersten Gitterstruktur mit einer beliebigen Anzahl von Zellen, insbesondere mit mehr als 4 Zellen der zweiten Gitterstruktur verbunden sind. Dasselbe gilt umgekehrt für die zweite Gitterstruktur. Es ist auch möglich, dass beide Gitterstrukturen jeweils im Bereich von höchstens 4 Zellen miteinander verbunden sind. Die Verbindung mit 3 oder 2 Zellen wird explizit offenbart. Die punktuelle Verbindung kann bspw. auch die Verbindung einzelner Gitterelemente der beiden Gitterstrukturen umfassen, insbesondere Gitterfilamente aus Kunststoff oder Metall, wie bspw. Gitterdrähte und/oder Stränge aus mehreren Filamenten, bzw. aus Drähten, die verdrillt oder parallel nebeneinander, d.h. unverdrillt, sein können..

Unter einer punktuellen Verbindung wird somit eine auf einen Teilbereich bzw. eine Teilfläche der Gitterstruktur begrenzte Verbindung verstanden, wobei insbesondere das Verhältnis zwischen der Fläche der verbundenen Gitterstrukturen und der Fläche der freien Gitterstrukturen so ausgebildet ist, dass die Gitterstrukturen sich im freien Bereich relativ zueinander, insbesondere ungehindert relativ zueinander bewegen können. Die Fläche der verbundenen Gitterstrukturen ist kleiner als die Fläche der freien Gitterstrukturen. Die wenigstens eine punktuelle Verbindung kann innerhalb der Gitterstruktur angeordnet sein. Die punktuelle Verbindung hat eine flächige Ausdehnung (ein oder mehrere Verbindungspunkte) oder eine linienförmige Ausdehnung (eine oder mehrere Verbindungslinien) und ist allseitig oder zumindest beidseitig, insbesondere bei der linienförmigen Ausdehnung, von lose aufeinander angeordneten Gitterstrukturen umgeben. Die punktuelle Verbindung kann somit wenigstens einen, insbesondere mehrere einzelne Verbindungspunkte jeweils mit einer flächigen Ausdehnung und/oder wenigstens eine, insbesondere mehrere einzelne Verbindungslinien umfassen. Dabei kann eine Verbindungslinie aus mehreren in einer Reihe, insbesondere in Umfangsrichtung angeordneten einzelnen Verbindungspunkten gebildet sein. Der Begriff Verbindungspunkt ist nicht streng mathematisch zu verstehen.

Die punktuelle Verbindung kann im Randbereich, insbesondere am Rand einer Gitterstruktur angeordnet sein. Der Randbereich kann insgesamt die punktuelle Verbindung bilden. Der Randbereich bildet einen in axialer Richtung der Vorrichtung angeordneten Außenbereich, der zumindest außerhalb der ersten Überkreuzung bzw. des ersten Zellsegments der Gitterstruktur angeordnet ist. Der Außenbereich kann bspw. der Schlaufenbereich eines geflochtenen Stents sein. Bei einem wieder einziehbaren Geflecht mit einer schräg zulaufenden Spitze, wie in DE 10 2009 056 450 beschrieben, bildet der Bereich der schräg zulaufenden Spitze bis zu dem im Querschnitt zylindrisch geschlossenen Mantelbereich den Randbereich, in welchem die Gitterstrukturen verbunden sind. Es ist auch möglich die Gitterstrukturen nur an der schrägen Kante der Spitze zu verbinden, bspw. durch Verdrillung der Drähte.

Wenn die punktuelle Verbindung am Rand einer Gitterstruktur angeordnet ist, bildet der Rand eine Begrenzung der Verbindung. Die übrigen Seiten der Verbindung grenzen an lose aufeinander angeordnete Gitterstrukturen an. Dabei können beide Gitterstrukturen jeweils am Rand oder eine Gitterstruktur am Rand und die andere Gitterstruktur vom Rand beabstandet, bspw. im mittleren Bereich verbunden sein. Dies gilt sowohl für die erste als auch für die zweite Gitterstruktur.

Es ist auch möglich, dass die wenigstens eine punktuelle Verbindung außerhalb der Gitterstruktur angeordnet ist, bspw. durch Verbindungsstränge oder-filamente bzw. -drähte, die über die Gitterstrukturen hinausragen und außerhalb der Gitterstrukturen verbunden sind.

Die punktuelle Verbindung kann unterschiedliche geometrische Formen aufweisen. Bspw. kann die Form der Verbindung der Form einer Zelle oder mehrerer zusammenhängender Zellen entsprechen. Generell kann die punktuelle Verbindung aus einzelnen Unterverbindungen gebildet sein, die ihrerseits punktuelle Verbindungen darstellen, bspw. in der Form von punktuell miteinander verbundenen Einzeldrähten oder Strängen. Die übergeordnete punktuelle Verbindung ist dabei zumindest teilweise von lose aufeinander angeordneten Gitterstrukturen umgeben derart, dass im nicht verbundenen Bereich der Gitterstrukturen eine Relativbewegung der Gitterstrukturen möglich ist. Dies gilt sowohl für flächige als auch für linienförmige punktuelle Verbindungen.

Die linienförmige Verbindung kann sich in Umfangsrichtung und/oder in Längsrichtung und/oder schräg zur Längsachse der Vorrichtung erstrecken. Die Erstreckung nur in Umfangsrichtung oder nur in Längsrichtung ist bevorzugt.

Die Länge der linienförmigen Verbindung beträgt höchstens 30%, insbesondere höchstens 25%, insbesondere höchstens 20%, insbesondere höchstens 15%, insbesondere höchstens 10%, insbesondere höchstens 5%, insbesondere höchstens 4%, insbesondere höchstens 3%, insbesondere höchstens 2%, insbesondere höchstens 1% der Gesamtlänge der Vorrichtung in Längsrichtung oder des Umfangs der Vorrichtung.

Es ist möglich, dass die punktuelle Verbindung am Rand oder sogar außerhalb der beiden Gitterstrukturen angeordnet ist. Bspw. können die beiden Gitterstrukturen durch einen gemeinsamen Strang oder Führungsdraht verbunden sein, durch den die Vorrichtung betätigbar bzw. in einem Zuführsystem bewegbar ist. In diesem Fall sind die beiden Gitterstrukturen über die gesamte Fläche der Vorrichtung lose aufeinander angeordnet und nur am axialen Ende, wo die beiden Gitterstrukturen mit dem Strang bzw. dem Führungsdraht verbunden sind punktuell fixiert.

Die beiden Schichten bzw. Gitterstrukturen sind koaxial ineinander angeordnet. Dadurch wird erreicht, dass der rohrförmige Körper in einem komprimierten Zustand einen gegenüber dem Stand der Technik kleineren Querschnittsdurchmesser aufweist. Konkret erfolgt bei der Erfindung eine regelmäßige Anordnung der einzelnen Drähte bzw. Stege der Gitterstruktur, wodurch die Anzahl an ungenutzten Freiräumen zwischen den einzelnen Drähten bzw. Stegen reduziert ist. Die Crimpbarkeit bzw. Komprimierbarkeit des rohrförmigen Körpers wird somit erhöht.

Durch die doppelwandige Struktur bzw. den mehrschichtigen Aufbau der Umfangswandung aus zueinander beweglichen Schichten können unterschiedliche Einsatzmöglichkeiten abgedeckt werden. Dabei kann bei der erfindungsgemäßen Vorrichtung eine Funktionstrennung erfolgen, wobei eine der Gitterstrukturen beispielsweise eine tragende bzw. stützende Funktion und die andere bzw. eine weitere Gitterstruktur die Funktion der Strömungsbeeinflussung im Bereich eines Aneurysmas aufweist.

Die Gitterstrukturen bzw. separaten Schichten sind punktuell miteinander verbunden. Durch die punktuelle Verbindung zwischen den Gitterstrukturen ist gewährleistet, dass die Gitterstrukturen relativ zueinander im Wesentlichen ihre Position beibehalten. Insbesondere behalten die Gitterstrukturen ihre Relativposition unabhängig von einem komprimierten oder expandierten Zustand bei. Dabei können sich Teile bzw. Abschnitte der Gitterstrukturen relativ zueinander bewegen. Eine vollständige Verschiebung der beiden Gitterstrukturen zueinander wird jedoch durch die punktuelle Verbindung verhindert. Auf diese Weise wird das Risiko einer Dislokation der medizinischen Vorrichtung vermindert.

Die erste Gitterstruktur und/oder die zweite Gitterstruktur ist jeweils aus miteinander verflochtenen Drähten gebildet. Vorzugsweise weisen beide Gitterstrukturen bzw. allgemein die Gitterstrukturen der Umfangswandung jeweils ein Drahtgeflecht auf. Die einzelnen Drahtgeflechte bzw. Gitterstrukturen sind also vorteilhaft aus jeweils mehreren Drähten gebildet, die sich spiralförmig um eine Längsachse des rohrförmigen Körpers erstrecken. Dabei sind gegenläufige Drahtspiralen vorgesehen, die miteinander verflochten sind. Die einzelnen Schichten der Umfangswandung sind also durch Drahtgeflechte bzw. miteinander verflochtene Drähte oder Bänder gebildet. Die Verflechtung besteht jedoch ausschließlich innerhalb einer einzelnen Schicht. Untereinander sind die einzelnen Schichten punktuell miteinander verbunden, so dass zwischen den Schichten eine Relativbewegung ermöglicht ist.

In diesem Zusammenhang wird darauf hingewiesen, dass im Rahmen der Anmeldung nicht nur Gitterstrukturen offenbart werden, die ein Drahtgeflecht umfassen. Vielmehr umfasst die Beschreibung auch Gitterstrukturen, die auf Basis von Gitterstegen gebildet sind. Derartige Gitterstrukturen können beispielsweise durch Laserschneiden oder Gasphasenabscheideverfahren hergestellt sein.

Im Rahmen der Erfindung ist ferner vorgesehen, dass jedes einzelne Drahtelement einer Gitterstruktur bzw. einer Schicht eine einzige Stelle umfasst, an der der Draht o0der die Drähte mit einem Draht oder mit Drähten einer benachbarten Schicht verbunden ist. Somit wird erreicht, dass zwischen den Schichten eine punktuelle Verbindung besteht.

Die erste Gitterstruktur kann ein proximales Ende aufweisen, das mit einem proximalen Ende der zweiten Gitterstruktur verbunden ist, so dass jeweils den proximalen Enden gegenüber angeordnete distale Enden der ersten und zweiten Gitterstruktur relativ zueinander bewegbar sind. Diese Ausführungsform geht auf die Idee zurück, die Gitterstrukturen der separaten Schichten an jeweils einem axialen Ende, insbesondere an den proximalen Enden, miteinander zu verbinden. Somit ist die gesamte Gitterstruktur dazwischen bewegbar. Die distalen Enden der ersten und zweiten Gitterstruktur sind hingegen frei angeordnet, so dass sich die distalen Enden der Gitterstruktur relativ zueinander bewegbar sind. Die zumindest abschnittsweise vorgesehene relative Beweglichkeit der Gitterstrukturen zueinander ermöglicht besonders vorteilhaft eine Funktionstrennung. Insbesondere können die erste und zweite Gitterstruktur unterschiedlich geometrisch gestaltet sein, so dass mit der ersten und der zweiten Gitterstruktur jeweils verschiedene Funktionen erfüllbar sind. Die Verbindung der proximalen Enden der Gitterstrukturen miteinander ist besonders vorteilhaft, da der relativ zueinander bewegbare Bereich der Gitterstrukturen vergleichsweise groß ist. Auf diese Weise können unterschiedliche Eigenschaften der Gitterstrukturen miteinander über einen vergleichsweise großen Bereich oder den gesamten Bereich des rohrförmigen Körpers wirksam werden. Alternativ zur Verbindung der proximalen Enden der Gitterstrukturen ist es auch möglich, dass die distalen Enden der ersten und zweiten Gitterstruktur miteinander verbunden sind. Ferner können die erste und die zweite Gitterstruktur in einen mittleren Bereich des rohrförmigen Körpers miteinander punktuell verbunden sein. Durch die freien Enden der Gitterstrukturen am distalen und/oder proximalen Ende der Vorrichtung wird bewirkt, dass die beiden Gitterstrukturen sich unabhängig voneinander bei der Expansion im Gefäß verkürzen können (foreshortening), bspw. wenn die beiden Gitterstrukturen unterschiedliche Flechtwinkel aufweisen.

Allgemein gilt, dass durch die nur punktuelle Verbindung der Gitterstrukturen eine Funktionstrennung der beiden Strukturen ermöglicht wird.

Gemäß einer weiteren bevorzugten Ausführungsform weisen die erste Gitterstruktur und die zweite Gitterstruktur in einem Herstellzustand zumindest abschnittsweise gleiche oder voneinander verschiedene Flechtwinkel auf. Durch unterschiedliche Flechtwinkel zwischen der ersten Gitterstruktur und der zweiten Gitterstruktur bzw. zwischen den separaten Schichten des rohrförmigen Körpers wird erreicht, dass sich die Gitterstrukturen bei der Expansion des rohrförmigen Körpers unterschiedlich stark verkürzen. Auch bei einer Querschnittsänderung des Körperhohlorgans, in dem die medizinische Vorrichtung eingesetzt ist, verhalten sich die Gitterstrukturen mit unterschiedlichen Flechtwinkel verschieden. Die unterschiedliche Verkürzung der Gitterstrukturen kann vorteilhaft für eine genaue Positionierung der medizinischen Vorrichtung eingesetzt werden. Beispielsweise kann die zweite Gitterstruktur derart ausgebildet sein, dass der Effekt des Foreshortening reduziert ist. Die zweite Gitterstruktur kann somit relativ exakt positioniert werden. Da die erste Gitterstruktur punktuell mit der zweiten Gitterstruktur verbunden ist, ermöglicht eine genaue Positionierung der zweiten Gitterstruktur gleichzeitig eine relativ genaue Positionierung der ersten Gitterstruktur bzw. allgemein des rohrförmigen Körpers.

Vorzugsweise beträgt der Flechtwinkel der ersten Gitterstruktur und/oder der zweiten Gitterstruktur höchsten 70°, insbesondere höchstens 65°, insbesondere höchstens 60°, insbesondere höchstens 59°, insbesondere höchstens 57°, insbesondere höchstens 55°, insbesondere höchstens 52°, insbesondere höchstens 50°. Ein derartiger Flechtwinkel stellt sicher, dass einerseits eine ausreichende Flexibilität der Gitterstrukturen bereitgestellt wird. Andererseits bewirkt ein derartiger Flechtwinkel eine Begrenzung des Foreshortening-Effekts. Ferner wird die Stauchbarkeit verringert, so dass der vorbestimmte Durchflusswiderstand nicht beeinträchtigt wird..

Zwischen der ersten Gitterstruktur und der zweiten Gitterstruktur kann in einem radial expandierten Zustand des rohrförmigen Körpers zumindest abschnittsweise ein Spalt ausgebildet sein. Insbesondere in Verbindung mit unterschiedlichen Flechtwinkeln für die erste Gitterstruktur und die zweite Gitterstruktur wird ermöglicht, dass sich bei einer Querschnitts- oder Längenänderung des Körperhohlorgans, in dem die medizinische Vorrichtung angeordnet ist, die beiden Gitterstrukturen voneinander abheben. Dadurch wird zwischen den Gitterstrukturen ein Spalt, insbesondere Ringspalt gebildet.

Bspw. kann der Spalt dadurch erzeugt werden, dass die äußere, zweite Gitterstruktur bzw. das außen angeordnete Netz in Längsrichtung zwei voneinander beabstandete punktuelle Verbindungen mit der inneren Gitterstruktur aufweist, bspw. zwei in Umfangsrichtung verlaufende Verbindungslinien oder einzelne Verbindungspunkte entlang zweier in Umfangsrichtung verlaufender Linien. Die Verbindungslinien können an den axialen Enden der Gitterstruktur oder von einem oder beiden Enden axial nach innen versetzt angeordnet sein. Die beiden Gitterstrukturen weisen unterschiedliche Flechtwinkel auf. Die äußeren zweite Gitterstruktur weist einen kleineren Flechtwinkel und somit eine geringeres Foreshortening als die innere erste Gitterstruktur auf. Im expandierten Zustand ist die innere Gitterstruktur stärker verkürzt als die äußere Gitterstruktur. Dadurch kommt es zu einer Auswölbung der zweiten Gitterstruktur und somit zu einem Spalt zwischen beiden Gitterstrukturen. Der Flechtwinkelunterschied kann mindestens 1°, insbesondere mindestens 2°, mindestens 3°, mindestens 4°, mindestens 5°, mindestens 10°, mindestens 15°, mindestens 20°, mindestens 25°, mindestens 30° betragen. Die Obergrenze für den Bereich des Flechtwinkelunterschieds beträgt höchstens 30°, insbesondere höchstens 25°, höchstens 20°, höchstens 15°, höchstens 10°, höchstens 5°, höchstens 4°, höchstens 3°, höchstens 2°, höchstens 1°. Die vorstehend genannten Ober- und Untergrenzen können jeweils miteinander kombiniert werden.

Generell kann die Auswölbung zwischen einzelnen axial beabstandeten Verbindungspunkten, insbesondere zwischen Paaren von axial beabstandeten Verbindungspunkten erfolgen. Es können zwei in Längsrichtung beabstandete Verbindungslinien aus mehreren in Umfangsrichtung in Reihe angeordneten Verbindungspunkten vorgesehen sein. Es können auch mehr als zwei derartige Verbindungslinien vorgesehen sein, zwischen denen jeweils eine Auswölbung gebildet ist, so dass mehrere Auswölbungen hintereinander angeordnet sind.

Im Spalt zwischen den Gitterstrukturen entstehen Strömungswirbel, die eine Art Polster bilden. Das Polster führt zu einem erwünschten Energieverlust, wobei sich die Strömungsgeschwindigkeit verlangsamt. Die Schubspannungen im Hauptgefäß wirken somit zunächst im Spalt und erzeugen dort die Wirbel. Die über die Wandung des Netzes bzw. der äußeren Gitterstruktur vom Spalt ins Aneurysma übertragenen Schubspannungen werden dadurch verringert. Die Gerinnung im Aneurysma wird begünstigt. Ferner werden die durch das Einströmen des Blutes in das Aneurysma bewirkten lokalen Druckbeanspruchungen der Aneurysmenwand reduziert.

Vorzugsweise weisen die erste Gitterstruktur und die zweite Gitterstruktur jeweils geschlossene Maschen auf. Die Größe der Maschen der ersten Gitterstruktur ist vorteilhafterweise von der Größe der Maschen der zweiten Gitterstruktur verschieden. Insbesondere kann die erste Gitterstruktur eine kleinere Maschengröße aufweisen als die zweite Gitterstruktur. Mit anderen Worten weist die erste Gitterstruktur vorzugsweise eine gegenüber der zweiten Gitterstruktur erhöhte Feinmaschigkeit auf. Die zweite Gitterstruktur kann beispielsweise eine Tragstruktur für die netzartige Struktur der ersten Gitterstruktur bilden. Auf diese Weise wird die Funktionstrennung zwischen den beiden Gitterstrukturen bzw. Schichten der Umfangswandung sichergestellt. Die zweite Gitterstruktur stützt bzw. fixiert die erste Gitterstruktur im Blutgefäß. Die erste Gitterstruktur kann hingegen derart feinmaschig ausgebildet sein, dass eine effiziente Strömungsbeeinflussung der Blutströmung in das Aneurysma gewährleistet ist. Ferner kann die erste Gitterstruktur derart flexibel sein, dass die erste Gitterstruktur einer Querschnittsänderung des Blutgefäßes gut folgen kann.

Die Drähte der ersten Gitterstruktur weisen einen kleineren Querschnittsdurchmesser als die Drähte der zweiten Gitterstruktur auf. Die Ausweitbarkeit der ersten Gitterstruktur gegenüber der zweiten Gitterstruktur wird somit erhöht. Die erste Gitterstruktur kann ferner eine größere Anzahl an Drähten aufweisen als die zweite Gitterstruktur. Damit ist sichergestellt, dass die erste Gitterstruktur eine höhere Feinmaschigkeit aufweist als die zweite Gitterstruktur. Im Zusammenhang mit einem kleineren Querschnittsdurchmesser der Drähte der ersten Gitterstruktur wird die Ausweitbarkeit der ersten Gitterstruktur gegenüber der zweiten Gitterstruktur weiter erhöht. Die Funktion der Strömungsbeeinflussung einer Blutströmung in das Aneurysma wird verbessert.

Vorzugsweise bildet die erste Gitterstruktur eine Außenschicht und die zweite Gitterstruktur eine Innenschicht des rohrförmigen Körpers. Dabei kann die zweite Gitterstruktur eine Tragstruktur und die erste Gitterstruktur eine netzartige Abdeckstruktur bilden. Die Tragstruktur stützt die Abdeckstruktur von Innen. Dadurch wird vermieden, dass sich die erste Gitterstruktur bzw. die Abdeckstruktur nicht vollständig bei der Expansion entfaltet. Die innere Tragstruktur bzw. die die Innenschicht bildende zweite Gitterstruktur stützt die erste Gitterstruktur über ihre gesamte Länge.

Bei einer weiteren bevorzugten Ausgestaltung der medizinischen Vorrichtung weist die erste Gitterstruktur eine axiale Längserstreckung auf, die kleiner als eine axiale Längserstreckung der zweiten Gitterstruktur ist, so dass die erste Gitterstruktur die zweite Gitterstruktur abschnittsweise, insbesondere um höchstens 98%, höchstens 97%, höchstens 96%, höchstens 95%, höchstens 94%, höchstens 93%, höchstens 92%, höchstens 91%, höchstens 90%, höchstens 85%, höchstens 80%, höchstens 75%, höchstens 70%, höchstens 65%, höchstens 60%, höchstens 55%, höchstens 50%, höchstens 45%, höchstens 40%, höchstens 35%, höchstens 30%, höchstens 25%, höchstens 20%, höchstens 15%, höchstens 10%, höchstens 5% bezogen auf die längere Gitterstruktur überdeckt. Die vorgenannten geometrischen Verhältnisse gelten für den Herstellzustand der medizinischen Vorrichtung. Der Herstellzustand entspricht im Wesentlichen einem kraftunbelasteten Zustand. Das bedeutet, dass die medizinische Vorrichtung keiner äußeren Kraft ausgesetzt ist, die eine Komprimierung des rohrförmigen Körpers bewirkt. Mit anderen Worten ist der rohrförmigen Körper im Herstellzustand vollständig expandiert.

Es ist auch möglich, die Vorrichtung so anzupassen, dass die vorgenannten geometrischen Verhältnisse im komprimierten Zustand vorliegen, wobei die erste Gitterstruktur kürzer als die zweite Gitterstruktur ist. Die vorstehend genannten Werte werden auch im Zusammenhang mit dem komprimierten Zustand offenbart.

Der Längenunterschied zwischen den beiden Gitterstrukturen kann durch eine Anpassung des Foreshortenings durch eine geeignete Wahl der Flechtwinkel beim Expandieren vergrößert, verkleinert oder konstant gehalten werden. Bspw. kann der Längenunterschied um wenigstens 10%, insbesondere wenigstens 20%, insbesondere wenigstens 30%, insbesondere wenigstens 40%, insbesondere wenigstens 50%, insbesondere wenigstens 60%, insbesondere wenigstens 70%, insbesondere wenigstens 80%, insbesondere wenigstens 90% oder um 100% (Längenausgleich) verkürzt werden. Andererseits kann der Längenunterschied um wenigstens 2%, insbesondere wenigstens 5%, insbesondere wenigstens 10%, insbesondere wenigstens 20%, insbesondere wenigstens 30%, insbesondere wenigstens 40%, insbesondere wenigstens 50%, insbesondere wenigstens 60% vergrößert werden. Bei Längengleichheit, also wenn der Ausgangsunterschied 0 mm beträgt, werden die vorstehend genannten Werte auf die Gesamtlänge einer der beiden Gitterstrukturen bezogen.

In absoluten Werten kann der Längenunterschied wie folgt geändert (verringert oder vergrößert) werden: 1mm, 5mm, 10mm, 15mm, 20mm. Im Fall der Verringerung des Längenunterschieds sind diese Werte untere Bereichsgrenzen (wenigstens) und im Fall der Vergrößerung obere Bereichsgrenzen (höchstens).

Vorzugsweise ist die zweite Gitterstruktur zumindest abschnittsweise durch die erste Gitterstruktur bedeckt. Der rohrförmige Körper weist also zumindest einen Abschnitt auf, der einen mehrschichtigen Aufbau aufweist.

Der rohrförmige Körper kann wenigstens eine dritte Gitterstruktur aufweisen. Die dritte Gitterstruktur bildet vorzugsweise gemeinsam mit der ersten Gitterstruktur die Außenschicht des rohrförmigen Körpers bzw. der Umfangswandung des rohrförmigen Körpers. Im Allgemeinen können die einzelnen separaten Schichten der Umfangswandung mehrere Gitterstrukturen aufweisen. Die Gitterstrukturen einzelner Schichten können relativ zueinander bewegbar sein. Wesentlich ist, dass eigenständige Schichten jeweils wenigstens eine Gitterstruktur umfassen, die gegenüber einer Gitterstruktur einer benachbarten Schicht abschnittsweise relativbeweglich ist. Bevorzugt ist es, wenn die Außenschicht mehrere Gitterstrukturen aufweist. Im komprimierten und/oder expandierten Zustand des rohrförmigen Körpers können die Gitterstrukturen der Außenschicht, also die erste und die dritte Gitterstruktur zueinander fluchtend angeordnet sein. Der Querschnittsdurchmesser des rohförmigen Körpers im komprimierten Zustand ist somit reduziert. Alternativ können die Gitterstrukturen der Außensicht, also die erste und die dritte Gitterstruktur, im komprimierten und/oder expandierten Zustand des rohrförmigen Körpers überlappend angeordnet sein. Das ermöglicht eine vergleichsweise großflächige Abdeckung der zweiten Gitterstruktur in einem expandierten Zustand des rohrförmigen Körpers.

Vorzugsweise ist die erste Gitterstruktur an einem proximalen Ende und die dritte Gitterstruktur an einem distalen Ende mit der zweiten Gitterstruktur verbunden, die die Innenschicht des rohrförmigen Körpers bildet. Mit anderen Worten weisen die erste und die dritte Gitterstruktur jeweils ein an der zweiten Gitterstruktur fixiertes Ende und ein freies Ende auf, wobei die freien Enden der ersten und der dritten Gitterstruktur einander zugewandt bzw. benachbart angeordnet sind. Im komprimierten Zustand des rohrförmigen Körpers können die freien Enden der ersten und dritten Gitterstruktur fluchtend zueinander bzw. bündig aneinander angeordnet sein. Die freien Enden der ersten und dritten Gitterstruktur können sich auch im komprimierten Zustand des rohrförmigen Körpers überlappen.

Bevorzugt ist vorgesehen, dass sich die erste Gitterstruktur und die dritte Gitterstruktur in einen radial komprimierten Zustand oder einen radial expandierten Zustand zumindest abschnittsweise überlappen. Aufgrund des Foreshortening-Effekts, der nicht nur auf die erste und dritte Gitterstruktur, sondern insbesondere auf die zweite Gitterstruktur wirkt, die die Innenschicht des rohrförmigen Körpers bildet, erfolgt bei der Expansion des rohrförmigen Körpers eine Verkürzung der Innenschicht bzw. zweiten Gitterstruktur. Die beiden Gitterstrukturen, die die Außenschicht bilden, also die erste und dritte Gitterstruktur, nähern sich bei der Expansion des rohrförmigen Körpers einander an. Durch geeignet Auslegung der einzelnen Gitterstrukturen kann erreicht werden, dass sich die erste und dritte Gitterstruktur im expandierte Zustand des rohrförmigen Körpers abschnittsweise überlappen. Bei der Expansion nähern sich also die freien Enden der ersten und zweiten Gitterstuktur an und schieben sich übereinander. Dies ist der Fall, wenn der Effekt des Foreshortenings der äußeren Gitterstruktur kleiner ist als der Effekt des Foreshortenings der inneren Gitterstruktur. Vorzugsweise ist der überlappende Bereich der ersten und dritten Gitterstruktur am Behandlungsort im Bereich des Aneurysmas angeordnet. Dadurch wird bewirkt, dass die Außenschicht des rohrförmigen Körpers im Bereich des Aneurysmas eine erhöhte Ausweitbarkeit aufweist, da die freien Enden der ersten und dritten Gitterstruktur relativ zueinander bewegbar sind. Unter Einfluss der Blutströmung können sich daher die freien Enden der ersten und dritten Gitterstruktur bzw. der Überlappungsbereiche in das Aneurysma wölben, so dass zwischen der Außenschicht und der Innenschicht bzw. der zweiten Gitterstruktur im Bereich des Aneurysmas ein Strömungspolster gebildet ist, in dem die Strömungsenergie des in das Aneurysma strömenden Blutes reduziert wird und somit die Belastung auf die Aneurysmenwand gemindet wird. Es ist ferner möglich, dass sich die erste und dritte Gitterstruktur sowohl im radial komprimierten Zustand, als auch im radial expandierten Zustand abschnittsweise überlappen. Die Gitterstrukturen bzw. die Innenschicht und die Außenschicht können derart ausgebildet sein, dass sich die erste und dritte Gitterstruktur im radial expandierten Zustand des rohrförmigen Körpers nicht überlappen. Mit anderen Worten können die freien Ende der ersten und dritten Gitterstruktur im radial expandierten Zustand bündig aneinander oder beabstandet voneinander angeordnet sein. Die erste und dritter Gitterstruktur können also im radial expandierten Zustand des rohrförmigen Körpers zueinander fluchtend angeordnet sein.

Bei einer weiteren bevorzugten Ausführungsform ist vorgesehen, dass die erste Gitterstruktur und die dritte Gitterstruktur jeweils ein proximales Ende umfassen, das mit der zweiten Gitterstruktur verbunden ist. Dabei kann das proximale Ende der ersten Gitterstruktur von dem proximalen Ende der zweiten Gitterstruktur beabstandet angeordnet sein. Die Außenschicht kann im Allgemeinen mehrere Gitterstrukturen aufweisen, die jeweils einen axialen Abschnitt der Außenschicht bilden. Die Gitterstrukturen, insbesondere die erste und die dritte Gitterstruktur weisen jeweils ein proximales Ende auf, das mit der zweiten Gitterstruktur, also der Innenschicht, punktuell verbunden ist. Das distale Ende der ersten und dritten Gitterstruktur ist frei angeordnet. Die erste und dritte Gitterstruktur können sich überlappen. Auf diese Weise können die erste und dritte Gitterstruktur eine schuppenartige Außenschicht bilden. Insbesondere können die erste und dritte Gitterstruktur eine Klappenfunktion aufweisen, wobei die erste und dritte Gitterstruktur vorteilhaft im Bereich eines Aneurysmas positioniert werden. Die freien Enden der ersten und/oder dritten Gitterstruktur können bezüglich der Innenschicht des rohrförmigen Körpers radial nach außen auslenkbar sein, so dass ein Katheter, beispielsweise ein Katheter zur Positionierung von Coils, in das Aneurysma einführbar ist, wobei der Katheter durch die Maschen der zweiten Gitterstruktur hindurchgeführt wird und wenigstens ein freies Ende der ersten oder dritten Gitterstruktur radial nach außen auslenkt, um einen Zugang zum Aneurysma zu erhalten.

Eine weitere bevorzugte Ausgestaltung der medizinischen Vorrichtung sieht vor, dass die erste Gitterstruktur einen mittleren Abschnitt und zwei dem mittleren Abschnitt begrenzende Randabschnitte umfasst. Im mittleren Abschnitt weist die erste Gitterstruktur einen kleineren Flechtwinkel als in den Randabschnitten auf. Im Allgemeinen kann vorgesehen sein, dass der Flechtwinkel der jeweiligen Gitterstruktur variabel ist. Mit anderen Worten kann sich der Flechtwinkel entlang der Gitterstruktur, insbesondere in Längsrichtung der Gitterstruktur, ändern. Vorzugsweise ändert sich der Flechtwinkel entlang der ersten Gitterstruktur derart, dass im mittleren Abschnitt ein kleinerer Flechtwinkel vorliegt als in den Randabschnitten. Damit wird erreicht, dass die erste Gitterstruktur im mittleren Abschnitt eine gegenüber den Randabschnitten erhöhte radiale Ausweitbarkeit aufweist. Der mittlere Abschnitt der ersten Gitterstruktur ist also in radialer Richtung weiter ausweitbar als die Randabschnitte. Die Ausweitbarkeit ist eine Folge der lokalen Flexibilität im mittleren Abschnitt. Der Begriff Flexibilität wird jedoch in erster Linie für das Biegeverhalten der gesamten Vorrichtung bzw. des gesamten Stents verwendet.

Eine Streckung des Gefäßabschnitts, in dem die medizinische Vorrichtung eingesetzt ist, während der Systole wird durch die Randabschnitte kompensiert. Somit ist sichergestellt, dass sich die axialen Enden der ersten Gitterstruktur, insbesondere ein freies Ende der ersten Gitterstruktur, ihre Lage zumindest nicht signifikant verändern. Die Positionierung der ersten Gitterstruktur wird vielmehr beibehalten. Der mittlere Abschnitt mit dem kleineren Flechtwinkel kann hingegen während der Systole den kleineren Foreshortening-Effekt nutzen. Konkret kann sich der mittlere Abschnitt der ersten Gitterstruktur bei einer Streckung und gleichzeitigen Ausweitung des Gefäßabschnitts verkürzen. Vorzugsweise ist der mittlere Abschnitt auf Höhe des Aneurysmas bzw. des Aneurysmenhalses positioniert. Damit wird erreicht, dass sich der mittlere Abschnitt der ersten Gitterstruktur während der Systole in das Aneurysma bzw. in den Bereich des Aneurysmenhalses hineinwölben kann. Durch den kleinen Flechtwinkel ist die Längenänderung nicht signifikant. Damit trägt der mittlere Abschnitt der ersten Gitterstruktur dazu bei, den systolischen Druck aus dem Blutgefäß in das Aneurysma zumindest bis zu einem gewissen Grad zu übertragen, so dass weiterhin eine mechanische Belastung der Zellen der Aneurysmenwand gegeben ist. Eine Degeneration der Zellen in der Aneurysmenwand wird somit vermieden.

Bei einer bevorzugten Ausführung variiert der Abstand zwischen der Außenschicht und der Innenschicht im expandierten Zustand des Körpers, wobei der Abstand zumindest abschnittsweise abwechselnd ab- und zunimmt. Konkret weist die Außenschicht im expandierten Zustand des Körpers zumindest abschnittsweise eine wellenförmige Kontur auf. Die Wellenförmige Kontur verlangsamt besonders effektiv die Strömung.

Die Außenschicht kann alternierend angeordnete Wellenberge und Wellentäler aufweist, wobei zumindest ein Teil der, insbesondere alle Wellentäler mit der Innenschicht verbunden sind und/oder vorgeformt, insbesondere durch eine Wärmebehandlung vorgeformt sind und/oder einen anderen Flechtwinkel als die Wellenberge aufweisen. Bei der Verbindung, insbesondere der mechanischen Verbindung der Wellentäler mit der Innenschicht kann es vorteilhaft sein, ein einziges Wellental oder mehr als 1 Wellental, insbesondere mehr als 2, mehr als 3, mehr als 4 Wellentäler, insbesondere alle Wellentäler mit der Innenschicht zu verbinden und zu fixieren. Die fixierten Wellentäler sind proximal angeordnet, d.h. auf der selben Seite wie das proximale Ende der Außenschicht. Das distale Ende der Außenschicht sowie etwaige distale, nicht fixierte Wellentäler sind in axialer Richtung beweglich. Bei einer besonders bevorzugten Ausführung ist nur das proximale Ende fixiert, das als halbes Wellental proximal vor dem ersten Wellenberg angesehen werden kann. Alle vollen Wellentäler einschließlich des distalen Endes sind frei und beweglich.

Die Wellenform kann durch mechanische Formgebung vorgeformt bzw. aufgeprägt sein und bildet den Ruhezustand. In der Katheterleitung wird die Wellenform gestreckt und kehrt beim Entlassen in den wellenförmigen Ruhe- bzw. Ausgangszustand zurück. Bei Verwendung eines Formgedächntniswerkstoffes kann die Wellenform unter Ausnützung des Formgedächtniseffektes durch eine geeignete Wärmebehandlung aufgeprägt werden. Durch unterschiedliche Flechtwinkel kann die radiale Stabilität lokal beeinflusst werden, so dass sich Bereiche leicht aufweiten (Wellenberge) und Bereiche weniger leicht aufweiten (Wellentäler). Die vorstehend genannten Optionen zur Ausbildung der Wellenkontur können miteinander einzeln oder insgesamt kombiniert werden.

Gemäß einem nebengeordneten Aspekt beruht die Erfindung auf dem Gedanken, ein System für medizinische Anwendungen mit einer Vorrichtung nach Anspruch 1 und einem Zuführsystem anzugeben, das ein flexibles Zuführelement, insbesondere einen Führungsdraht, umfasst. Das Zuführelement ist mit der Vorrichtung gekoppelt oder koppelbar. Vorzugsweise ist das System derart angepasst, dass die Vorrichtung in das Zuführsystem wieder einziehbar ist.

Die im Zusammenhang mit der medizinischen Vorrichtung beschriebenen Ausführungsbeispiele und Vorteile gelten gleichermaßen für das System mit einer derartigen Vorrichtung.

Die Erfindung wird im Folgenden anhand von Ausführungsbeispielen unter Bezugnahme auf die beigefügten schematischen Zeichnungen näher erläutert. Darin zeigen:
- Figur 1a:: einen Querschnitt durch ein Blutgefäß mit einem Aneurysma, wobei die Druckübertragung in das Aneurysma dargestellt ist;
- Figur 1b:: das Blutgefäß gemäß Figur 1a, wobei ein herkömmlicher Aneurysmenstent eingesetzt;
- Figur 1c:: eine grafische Darstellung des Druckverlaufs im Blutgefäß und im Aneurysma bei Einsatz eines herkömmlichen Aneurysmenstents;
- Figur 2a:: einen Querschnitt durch ein Blutgefäß mit einem Aneurysma, wobei der Einfluss der Schubspannung auf das Aneurysma dargestellt ist;
- Figur 2b:: das Blutgefäß gemäß Figur 2a, wobei ein herkömmlicher Aneurysmenstent eingesetzt ist;
- Figur 3a:: einen Querschnitt durch ein Blutgefäß mit einem Aneurysma, wobei der Einfluss einer direkten Blutströmung in das Aneurysma dargestellt ist;
- Figur 3b:: das Blutgefäß gemäß Figur 3a, wobei ein herkömmlicher Aneurysmenstent mit einer kleinen Maschengröße eingesetzt ist;
- Figur 3c:: das Blutgefäß gemäß Figur 3a, wobei ein herkömmlicher Aneurysmenstent mit einer großen Maschengröße eingesetzt ist;
- Figur 4a:: ein Längsschnitt durch ein Blutgefäß mit einem Aneurysma und einem eingesetzten herkömmlichen Aneurysmenstent, wobei der Einfluss von pulsbedingten Veränderungen des Blutgefäßes auf den Aneurysmenstent dargestellt ist;
- Figur 4b:: das Blutgefäß gemäß Figur 4a, wobei eine erhöhte Blutströmung in das Aneurysma durch die Maschen eines herkömmlichen Aneurysmenstens unter Einfluss eines systolischen Blutdrucks dargestellt ist;
- Figur 5a:: einen Längsschnitt durch ein Blutgefäß mit einem Aneurysma, wobei ein herkömmlicher Aneurysmenstent mit einem großen Flechtwinkel eingesetzt ist;
- Figur 5b:: das Blutgefäß gemäß Figur 5a, wobei ein herkömmlicher Aneurysmenstent mit einem kleinen Flechtwinkel eingesetzt ist;
- Figur 6a:: einen Querschnitt durch einen Aneuyrsmenstent gemäß dem Stand der Technik in einem Zuführsystem, wobei der Aneurysmenstent mehrere, miteinander verflochtene Wandungsschichten aufweist;
- Figur 6b:: einen Querschnitt durch eine erfindungsgemäße medizinische Vorrichtung gemäß einem bevorzugten Ausführungsbeispiel in einem Zuführsystem, wobei die Vorrichtung zwei separate Schichten von Gitterstrukturen aufweist;
- Figur 7:: einen Längsschnitt durch ein Blutgefäß mit einem Aneurysma, wobei eine erfindungsgemäße medizinische Vorrichtung nach einem bevorzugten Ausführungsbeispiel eingesetzt ist, die eine erste Gitterstruktur mit variablen Flechtwinkeln umfasst;
- Figur 8:: eine perspektivische Seitenansicht einer medizinischen Vorrichtung nach einem weiteren erfindungsgemäßen Ausführungsbeispiel;
- Figur 9:: die medizinische Vorrichtung gemäß Figur 8 während der Entlassung aus einem Zuführsystem;
- Figur 10:: die medizinische Vorrichtung gemäß Figur 8 in Anordnung innerhalb eines Blutgefäßes;
- Figur 11:: die medizinische Vorrichtung gemäß Figur 10 unter Einfluss einer Streckung des Blutgefäßes;
- Figur 12:: die medizinische Vorrichtung gemäß Figur 10 unter Einfluss eines systolischen Blutdrucks;
- Figur 13:: eine perspektivische Seitenansicht einer medizinischen Vorrichtung gemäß einem weiteren erfindungsgemäßen Ausführungsbeispiel unter Einfluss einer Streckung des Blutgefäßes, wobei eine Außenschicht der Umfangswandung eine kürzere Gitterstruktur als eine Innenschicht umfasst;
- Figur 14:: eine perspektivische Seitenansicht einer medizinischen Vorrichtung nach einem weiteren erfindungsgemäßen Ausführungsbeispiel unter Einfluss einer Streckung des Blutgefäßes, wobei die Außenschicht der Umfangswandung zweier nacheinander nachgeordneter Gitterstrukturen umfasst;
- Figur 15a:: eine perspektivische Seitenansicht einer medizinischen Vorrichtung gemäß einem weiteren erfindungsgemäßen Ausführungsbeispiel im implantierten Zustand, wobei die Außenschicht der Umfangswandung mit zwei sich im expandierten Zustand der Vorrichtung überlappende Gitterstrukturen umfasst;
- Figur 15b:: die Vorrichtung gemäß Figur 15a in einem komprimierten Zustand innerhalb eines Zuführsystems;
- Figur 16a:: eine perspektivische Seitenansicht einer medizinischen Vorrichtung gemäß einem weiteren erfindungsgemäßen Ausführungsbeispiel, wobei die Außenschicht der Umfangswandung zwei im expandierten Zustand der Vorrichtung fluchtend angeordnete Gitterstrukturen umfasst;
- Figur 16b:: die Vorrichtung gemäß Figur 16a in einem komprimierten Zustand innerhalb eines Zuführsystems;
- Figur 17:: die Vorrichtung gemäß Figur 12, wobei die Reduktion von Verwirbelungen im Aneurysma durch einen Polsterbereich zwischen der ersten und zweiten Gitterstruktur der rohrförmigen Wandung dargestellt ist;
- Figur 18:: die Abwicklung einer Vorrichtung nach einem erfindungsgemäßen Ausführungsbeispiel, bei der zwei Gitterstrukturen überlagert sind;
- Figur 19:: einen Querschnitt durch die Vorrichtung gemäß Figur 18 im Bereich der Verbindungshülse;
- Figur 20:: eine Abwicklung eines Trägers für eine Vorrichtung nach einem weiteren erfindungsgemäßen Ausführungsbeispiel ;
- Figur 21:: die Abwicklung einer Vorrichtung nach einem erfindungsgemäßen Ausführungsbeispiel mit dem Träger gemäß Figur 20;
- Figur 22: eine perspektivische Seitenansicht einer medizinischen Vorrichtung nach einem erfindungsgemäßen Ausführungsbeispiel, das zur Behandlung eines fusiformen Aneurysmas eingesetzt ist;
- Figur 23: eine perspektivische Seitenansicht einer medizinischen Vorrichtung nach einem weiteren erfindungsgemäßen Ausführungsbeispiel, das zur Behandlung eines fusiformen Aneurysmas eingesetzt ist; und
- Figur 24: eine perspektivische Seitenansicht einer medizinischen Vorrichtung nach einem anderen erfindungsgemäßen Ausführungsbeispiel, das zur Behandlung eines fusiformen Aneurysmas eingesetzt ist.

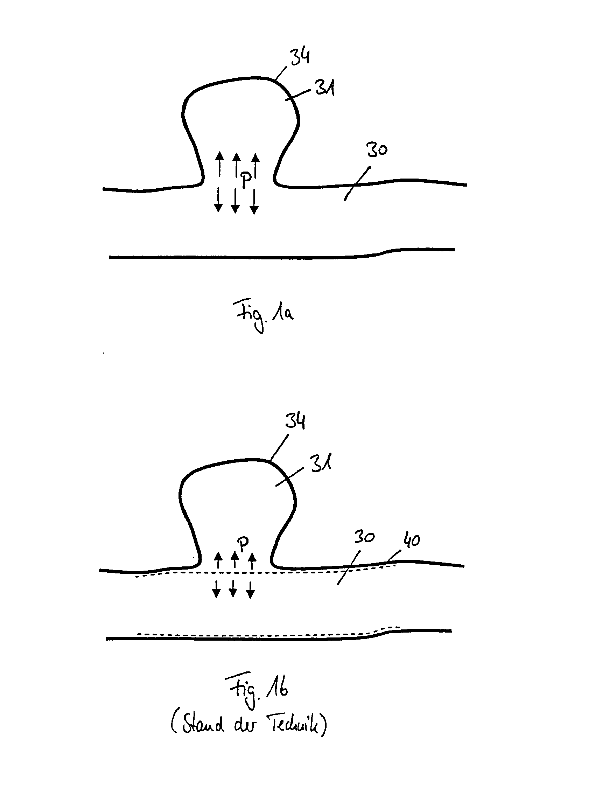

In Figur 1a ist der Einfluss des Blutdrucks auf ein Aneurysma 31 in einem Blutgefäß 30 veranschaulicht. Darin zeigen die Pfeile die Übertragung des Drucks P vom Blutgefäß 30 in das Aneurysma 31 an. Im Allgemeinen unterliegt das Blutgefäß 30 Druckschwankungen, die durch den pulsatielen Blutfluss bzw. die pulsierende Aktivität des Herzens erzeugt werden. Die Druckspitzen treten dabei in der sogenannten Systole, also der Auswurfphase der Herzaktivität auf. Ein Minimum nimmt der Druck in der Diastole an, wenn sich die Herzkammern mit Blut füllen.

Der Einfluss der Druckschwankungen auf das Aneurysma 31 wird gemäß dem Stand der Technik durch den Einsatz eines herkömmlichen feinmaschigen Aneurysmenstents 40 beeinflusst. Dazu ist der herkömmliche Aneurysmenstent 40 im Bereich des Aneurysmas 31 im Blutgefäß 30 implantiert (Figur 1b). Durch die Struktur des herkömmlichen Aneurysmenstents 40 wird der Blutfluss zwischen dem Blutgefäß 30 und dem Aneurysma 31 beeinflusst, so dass der Druck P, der in das Aneurysma 31 übertragen wird einerseits reduziert und andererseits zeitversetzt auf das Aneurysma 31 wirkt. Figur 1c zeigt anschaulich den Druckverlauf im Blutgefäß 30 (durchgezogene Linie) und im Aneurysma 31 (gestrichelte Linie), das durch einen herkömmlichen Aneurysmenstent 40 abgedeckt ist. Aus den dargestellten Druckverläufen ist deutlich zu erkennen, dass der Druck im Aneurysma 31 langsamer ansteigt und abfällt, als im Blutgefäß 30. Die Druckkurve im Aneurysma 31 wird also insgesamt flacher. Der Effekt ist umso wirksamer, je feinmaschiger das Geflecht ist. Die Abschwächung des Drucks ist wünschenswert, soll aber nicht übermäßig ausgeprägt sein.

Dennoch besteht die Möglichkeit, dass die Druckverminderung eine Degeneration der Zellen der Aneurysmenwand 34 fördert. Insbesondere durch die reduzierte mechanische Beanspruchung der Aneurysmenwand 34 besteht die Gefahr, dass die Zellen der Aneurysmenwand 34 degenerieren bzw. sich abbauen, wodurch die Gefahr einer Ruptur selbst nach Einsetzen eines Aneurysmenstents 40 erhöht ist.

Ferner wird ein Aneurysma 31 durch die tangentiale Blutströmung bzw. Gefäßströmung F_{G} beeinflusst, wie in Figur 2a veranschaulicht. Durch die Gefäßströmung F_{G} entstehen an den Grenzflächen zwischen dem Blut innerhalb des Aneurysmas 31 und dem Blut innerhalb des Blutgefäßes 30 Schubspannungen, die zu einer Wirbelströmung F_{W} innerhalb des Aneurysmas 31 führt. Durch den Einsatz eines herkömmlichen Aneurysmenstents 40, wie in Figur 2b dargestellt, wird der Einfluss der durch die Gefäßströmung F_{G} ausgelösten Schubspannung reduziert. Die Wirbelströmung F_{W} innerhalb des Aneurysmas 31 wird somit reduziert. Die Verringerung der Wirbelströmung F_{W} hat einerseits den Vorteil, dass die Gerinnung im Aneurysma verbessert wird. Andererseits kann aber eine übermäßige Verringerung der Wirbelströmung F_{W} die Degenerierung von Zellen der Aneurysmenwand 34 fördern. Außerdem wird ein Blutaustausch zwischen dem Blut im Aneurysma 31 und dem Blutgefäß 30 behindert, so dass eine ausreichende Nährstoffversorgung der Aneurysmenwand 34 nicht unter Umständen nicht gegeben ist. Bei zu starker Strömungsverringerung kann es zur Bildung übermäßig schnell wachsender Thromben kommen.

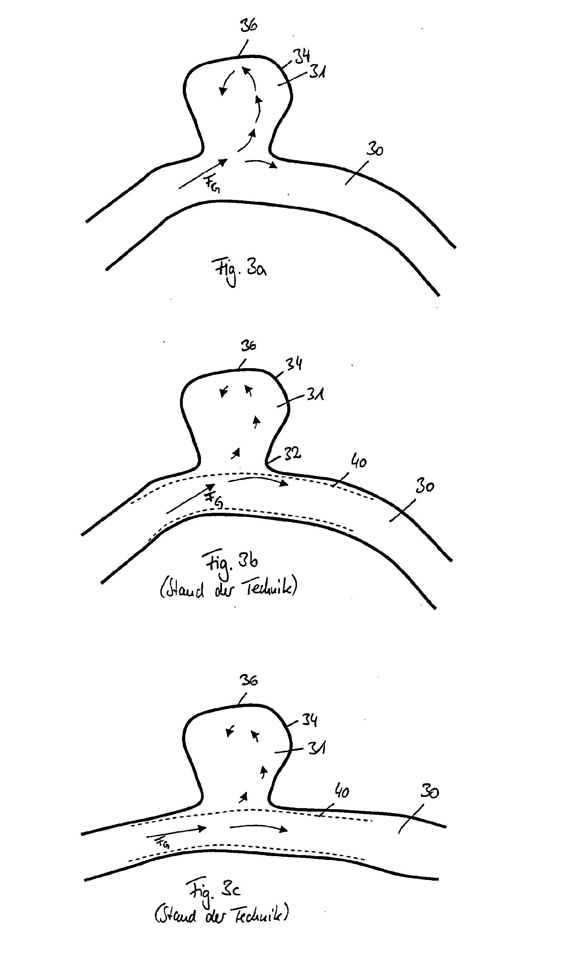

Figur 3a zeigt den Einfluss der Gefäßströmung F_{G} auf ein Aneurysma 31 im unbehandelten Zustand, wenn das Aneurysma 31 in einer Krümmung des Blutgefäßes 30 angeordnet ist. Das Aneurysma 31 wird dabei direkt mit der Gefäßströmung F_{G} beaufschlagt, wobei die Gefäßströmung F_{G} lokal in einem Anströmbereich 36 auf die Aneurysmenwand 34 trifft. Im Anströmbereich 36 wird die Gefäßströmung F_{G} umgelenkt. Die Aneurysmenwand 34 ist im Anströmbereich 36 einer erhöhten Druckbelastung ausgesetzt. Ein in das Blutgefäß 30 eingesetzter herkömmlicher Aneurysmenstent 40 bietet einen Widerstand für die Gefäßströmung F_{G}, so dass der Strömungsanteil bzw. Blutanteil, der in das Aneurysma 31 strömt, reduziert ist. Somit bewirkt ein herkömmlicher Aneurysmenstent 40 eine Verringerung der Strömungsgeschwindigkeit in das Aneurysma. Konkret wird eine Durchgangsströmung F_{D} durch den Aneurysmenhals 32 des Aneurysmas reduziert. Gleichzeitig bewirkt der Widerstand, den der herkömmliche Aneurysmenstent 40 der Blutströmung bzw. Gefäßströmung F_{G} bietet, eine Änderung der Druckwelle bzw. des Druckverlaufs innerhalb des Aneurysmas, wodurch die Degenerierung der Zellen der Aneurysmenwand 34 begünstigt ist. Dies gilt insbesondere für Stents mit einer feinmaschigen Struktur, die zudem eine hohe Biegeflexibilität aufweisen und sich daher gut in die Krümmung des Blutgefäßes 30 einpassen (Figur 3b). Herkömmliche Aneurysmenstents 40 mit einer grobmaschigen Struktur weisen hingegen eine höhere Radialkraft auf, so dass bewirkt wird, dass sich das Blutgefäß 30 streckt, wie in Figur 3c dargestellt. Zwar wird durch die Streckung des Blutgefäßes 30 der Anteil der Gefäßströmung F_{G}, der direkt in das Aneurysma 31 geleitet wird, reduziert. Die grobmaschige Struktur derartiger bekannter Stents 40 lässt jedoch gleichzeitig eine hohe Blutströmung in das Aneurysma 31, insbesondere in Folge von Schubspannungen, zu, so dass die Belastung des Aneurysmas groß ist.

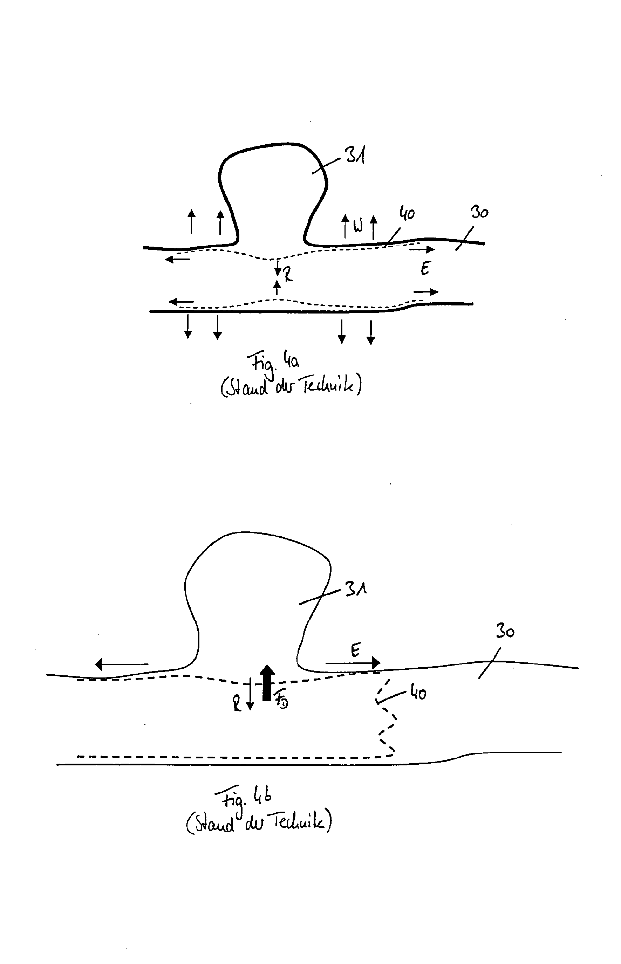

Herkömmliche Aneurysmenstents 40, insbesondere herkömmliche Aneursmenstents 40, die eine feinmaschige Struktur aufweisen, werden durch die periodischen Gefäßänderungen des Blutgefäßes 30 zusätzlich beeinflusst. In Figur 4a ist dargestellt, dass sich das Blutgefäß 30 während der Systole, also während einer Druckspitze im Verlauf des Blutdrucks, einerseits radial aufweitet, also ein Aufweitung W aufweist, und andererseits in Längsrichtung gestreckt wird. Neben der Aufweitung W erfolgt während der Systole auch eine Streckung E des Blutgefäßes 30. Aufgrund des eingangs erläuterten Foreshorteningeffekts bewirkt die Aufweitung W und Streckung E des Blutgefäßes 30 eine Verjüngung R des herkömmlichen Aneurysmenstents 40. Die Verjüngung R zeigt sich insbesondere im Bereich des Aneurysmas 31. Dadurch entfernt sich die Umfangswandung des herkömmlichen Aneurysmenstents 40 vom Aneurysmenhals 32, wie in Figur 4a dargestellt. Die große Gefahr der Dislokation des herkömmlichen Aneurysmenstents 40 wird durch die Ausweitung der Gefäße und die Verjüngung R verst6ärkt. Außerdem wird der Einfluss auf die Wirbelströmung F_{W} im Bereichung des Aneurysmas 31 reduziert. Die Effektivität der Aneurysmenbehandlung 31 wird daher geschmälert. Konkret zeigt die Struktur herkömmlicher Aneurysmenstents 40 während der Systole im Wesentlichen eine inverse Reaktion. Während der Blutdruck in der Systole lokal ein Maximum einnimmt, verjüngt sich gleichzeitig der Aneurysmenstent 40. Mit anderen Worten bewegt sich die Umfangswandung des herkömmlichen Aneurysmenstents 40 gegen den Druckanstieg. Dadurch wird die Durchgangsströmung F_{D} durch die Maschen des herkömmlichen Aneurysmenstents 40 in das Aneurysma 31 erhöht. Dieser Effekt ist in Figur 4b veranschaulicht.

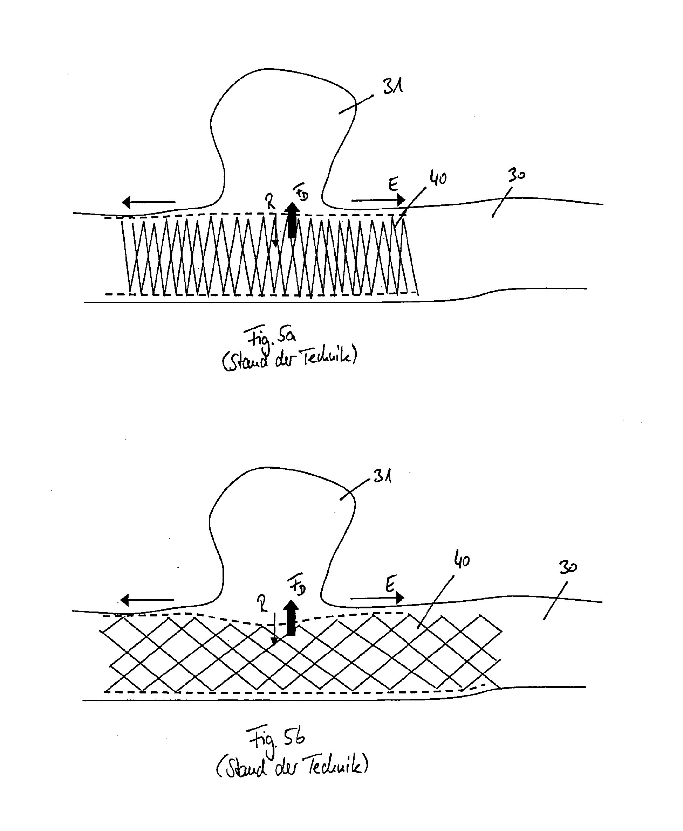

Aus dem Stand der Technik sind herkömmliche Aneurysmenstents 40 bekannt, die einen großen Flechtwinkel aufweisen, wie in Figur 5a dargestellt. Herkömmliche Aneurysmenstents 40 mit einem großen Flechtwinkel haben die Eigenschaft, dass sie einer Streckung E des Blutgefäßes folgen können, ohne eine starke Verjüngung R zu erfahren. Insgesamt ist die Struktur derartiger Aneurysmenstents 40 in radialer Richtung vergleichsweisedimensionsstabil (geringe Compliance), so dass zusätzliche eine starke Beeinflussung der Druckübertragung vom Blutgefäß 30 in das Aneurysma 31 erfolgt. Der Druck P im Aneurysma 31 wird insgesamt reduziert, wie in Figur 1c dargestellt.

Figur 5b zeigt einen herkömmlichen Aneurysmenstent 40, der einen kleinen Flechtwinkel umfasst. Derartige bekannte Stents weisen eine erhöhte Flexibilität auf, die jedoch bewirkt, dass bei einer Streckung E des Blutgefäßes 30 eine Verjüngung R des herkömmlichen Aneurysmenstents 40 auftritt mit den zuvor beschriebenen Nachteilen.

In Figur 6a ist eine Querschnittsansicht des bekannten Aneurysmenstents 40 gemäß DE 601 28 588 T2 im komprimierten Zustand innerhalb eines Zuführsystems 20 gezeigt. Der bekannte Aneurysmenstent 40 weist zwei Lagen von Drahtgeflechten auf, wobei die Drahtgeflechte der beiden Lagen miteinander verflochten sind. Ein erstes Drahtgeflecht weist erste Drähte 41 auf, die einen größeren Querschnittsdurchmesser umfassen als zweite Drähte 42 einer zweiten Lage des herkömmlichen Aneurysmenstents 40. Durch die Verflechtung der ersten und zweiten Drähte 41, 42 ergibt sich im komprimierten Zustand innerhalb des Zuführsystems 20 eine komplexe Anordnung der ersten und zweiten Drähten 41, 42 mit erhöhtem Raumbedarf im komprimierten Zustand.

Demgegenüber ist bei dem erfindungsgemäßen Ausführungsbeispiel vorgesehen, dass die medizinische Vorrichtung einen rohrförmigen Körper 10 umfasst. Die Vorrichtung ist insbesondere als Stent ausgebildet. Der rohrförmige Körper 10 weist eine Umfangswandung auf, die eine erste Gitterstruktur 11 und eine zweite Gitterstruktur 12 umfasst. Die erste und zweite Gitterstruktur 11, 12 bilden jeweils separate Schichten 14, 15 der Umfangswandung. Die Gitterstrukturen 11, 12 des erfindungsgemäßen rohrförmigen Körpers 10 sind also zumindest abschnittsweise, vorzugsweise entlang der gesamten Gitterstruktur voneinander unabhängig. Punktuell sind die Gitterstrukturen 11, 12 miteinander verbunden. Insbesondere kann die Umfangswandung des rohrförmigen Körpers eine Umfangslinie umfassen, in der die erste Gitterstruktur 11 mit der zweiten Gitterstruktur 12 verbunden ist. Insbesondere kann eine einzige Verbindungslinie zwischen der ersten Gitterstruktur 11 und der zweiten Gitterstruktur 12 vorgesehen, die sich in Umfangsrichtung um den rohrförmigen Körper 10 erstreckt. Explizit wird darauf hingewiesen, dass die Verbindung zwischen der ersten Gitterstruktur 11 und der zweiten Gitterstruktur 12 und eventuell weiteren Gitterstrukturen nicht über eine ausgedehnte Fläche, sondern im Wesentlichen linienförmig erfolgt.

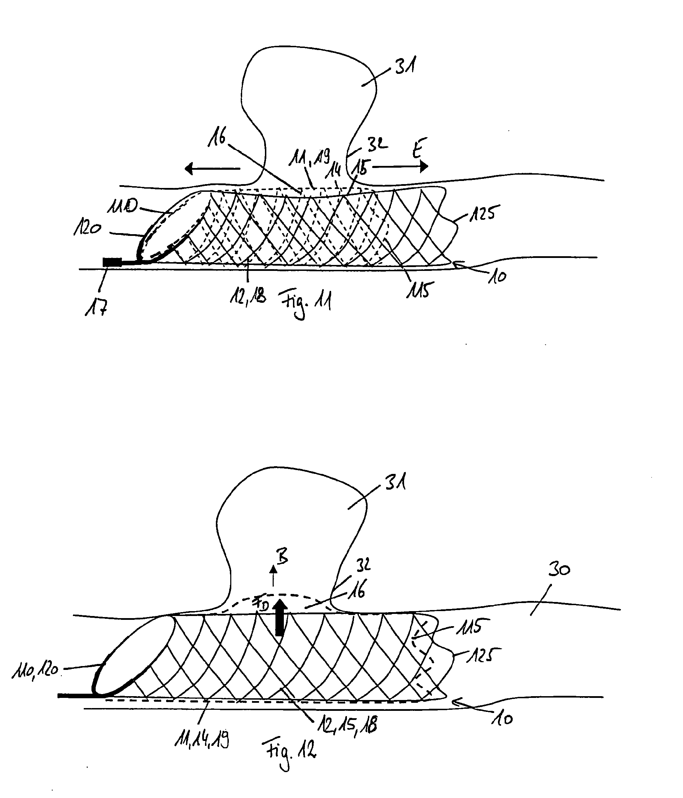

Durch die separaten Schichten, die jeweils eine Gitterstruktur 11, 12 umfassen, wird der Raumbedarf des rohrförmigen Körpers 10 im komprimierten Zustand reduziert, wie in Figur 6b dargestellt. Figur 6b zeigt einen Querschnitt durch den rohrförmigen Körper 10, der im expandierten Zustand zwei hohlzylinderförmig angeordnete Schichten 14, 15 umfasst (s. z.B. Fig. 12), die jeweils eine Gitterstruktur 11, 12 aufweisen. Dabei ist eine Innenschicht 15 vorgesehen, die die zweite Gitterstruktur 12 aufweist. Eine Außenschicht 14, die die Innenschicht 15 umgibt, umfasst die erste Gitterstruktur 11. Die schichtweise bzw. konzentrische Anordnung der beiden Gitterstrukturen 11, 12 bleibt im komprimierten Zustand erhalten, wie in Fig. 6b verdeutlicht.

Die Drähte 112 der ersten äußeren Gitterstruktur 11 weisen einen Querschnittsdurchmesser auf, der kleiner ist als der Querschnittsdurchmesser der Drähte 122 der zweiten inneren Gitterstruktur 12. Der rohrförmige Körper gemäß Figur 6b ist innerhalb eines Zuführsystems 20 angeordnet. Durch den reduzierten Raumbedarf des rohrförmigen Körpers 10 gegenüber herkömmlichen Aneurysmenstens 40 ist die Verwendung eines kleineren Zuführsystems 20 möglich. Die medizinische Vorrichtung ist somit in kleinere Blutgefäße einführbar.

In Figur 6b ist ferner erkennbar, dass die erste Gitterstruktur 11 und die zweite Gitterstruktur 12 koaxial ineinander angeordnet sind. Die erste Gitterstruktur 11 und die zweite Gitterstruktur 12 sind ferner wenigstens abschnittsweise relativ zueinander bewegbar. Konkret sind die erste und zweite Gitterstruktur 11, 12 außerhalb der linienförmigen oder punktuellen Verbindung zwischen der ersten und zweiten Gitterstruktur 11, 12 relativ zueinander bewegbar. Die relative Beweglichkeit bezieht sich insbesondere auf die einzelnen Drähte 112, 122 der ersten und zweiten Gitterstruktur 11, 12. Insbesondere können die Drähte 112 der ersten Gitterstruktur 11 auf den Drähten 122 der zweiten Gitterstruktur 12 gleiten, und umgekehrt.

Bei dem Beispiel gemäß Figur 6b sind die Drähte 112 der ersten Gitterstruktur 11 dicker als die Drähte 122 der zweiten Gitterstruktur 12. Es ist auch möglich, dass die Drähte 112, 122 der Gitterstrukturen 11, 12 denselben Querschnittsdurchmesser aufweisen. Die Drähte 122 der zweiten Gitterstruktur 12 umfassen erfindungsgemäss einen größeren Querschnittsdurchmesser als die Drähte 112 der ersten Gitterstruktur 11.

Für die verbesserte Crimpbarkeit des rohrförmigen Körpers 10 bzw. allgemein der Vorrichtung ist es vorteilhaft, wenn die Innenschicht 15 aus einer relativ kleineren Anzahl dickerer Drähte 122 gebildet ist und die Außenschicht 14 durch eine vergleichsweise höhere Anzahl dünnerer Drähte aufgebaut ist.

Im Allgemeinen hat es sich als vorteilhaft herausgestellt, wenn eine Schicht 14, 15 eine feinmaschigere Struktur aufweist als die andere Schicht 15, 14. Mit anderen Worten kann eine der beiden Schichten 14, 15 mehr Drähte 112, 122 umfassen, als eine andere Schicht 14, 15. Die Drähte 112, 122 können dabei dünner sein als die Drähte 112, 122 der anderen Schicht 15, 14. Es ist möglich, dass die feinmaschigere Schicht 14, 15 einen größeren oder einen kleineren Flechtwinkel als die vergleichsweise grobmaschigere Schicht 15, 14 aufweist. Kombinationen der zuvor genannten Varianten sind möglich.

Besonders bevorzugt ist es, wenn die feinmaschige Schicht die Außenschicht 14 und die grobmaschige Schicht die Innenschicht 15 bildet.

Im Allgemeinen weist die Vorrichtung vorzugsweise einen rohrförmigen Körper 10 auf, der eine Außenschicht 14 und eine Innenschicht 15 umfasst. Die Vorrichtung kann ein Stent sein. Die Außenschicht 14 ist durch die erste Gitterstruktur 11 und die Innenschicht 15 durch die zweite Gitterstruktur 12 gebildet. Die zweite Gitterstruktur 12 weist vorzugsweise ein grobmaschiges Drahtgeflecht auf. Die erste Gitterstruktur 11 weist hingegen ein feinmaschiges Drahtgeflecht auf. Das grobmaschige Drahtgeflecht der zweiten Gitterstruktur 12 bildet somit einen Träger 18, wogegen das feinmaschige Drahtgeflecht der ersten Gitterstruktur 11 ein Netz 19 bildet.

Generell ist es möglich, dass beiden Schichten, also die Außenschicht 14 und die Innenschicht 15, gleichartig ausgebildet sind. Die Schichten 14, 15 können also die gleiche Feinmaschigkeit und/oder die gleiche Drahtanzahl und/oder gleiche Flechtwinkel aufweisen. Alle Kombinationen der vorgenannten Varianten sind möglich. Durch die Verbindung der Gitterstrukturen sind diese zueinander ausgerichtet, bzw. weisen zueinander ausgerichtete Flechtmuster auf.

Ferner wird darauf hingewiesen, dass die Erfindung nicht auf zweischichtige Strukturen eingeschränkt ist. Vielmehr werden im Rahmen der Anmeldung auch rohrförmige Körper 10 bzw. Stents beansprucht und offenbart, die eine Umfangswandung mit drei oder mehr separaten Schichten umfassen. Einige oder alle Schichten können erfindungsgemäß aufgebaut sein.

Nachfolgend werden besondere vorteilhafte Ausführungen des Trägers 18 beschrieben:

Der Träger 18 bzw. die zweite Gitterstruktur 12 weist vorzugsweise höchstens 32, insbesondere höchstens 24, insbesondere höchstens 20, insbesondere höchstens 16, insbesondere höchstens 12, insbesondere höchstens 8, insbesondere höchstens 6, Drähte 122 auf. Die Drähte 122 der zweiten Gitterstruktur 12 bzw. des Trägers 18 umfassen vorzugsweise einen Querschnittsdurchmesser von wenigstens 40 µm, insbesondere wenigstens 50µm, insbesondere wenigstens 60µm, insbesondere wenigstens 68µm, insbesondere wenigstens 75µm, insbesondere wenigstens 84µm, insbesondere wenigstens 100µm. Dies gilt für medizinische Vorrichtungen für den Einsatz in Blutgefäßen 30, die einen Querschnittsdurchmesser von 2 mm bis 6 mm aufweisen. Bei einem Querschnittsdurchmesser des zu behandelnden Blutgefäßes 30, der größer als 6 mm beträgt, weisen die Drähte 122 der zweiten Gitterstruktur 12 bzw. des Trägers 18 vorzugsweise einen Querschnittsdurchmesser von wenigstens 40 µm, insbesondere wenigstens 50 µm, insbesondere wenigstens 60 µm, insbesondere wenigstens 68 µm, insbesondere wenigstens 75 µm, insbesondere wenigstens 84 µm, insbesondere wenigstens 100 µm, insbesondere wenigstens 150 µm, insbesondere wenigstens 200 µm, auf.

Grundsätzlich kann die Umfangswandung des rohrförmigen Körpers 10 mehr als einen Träger 18 umfassen.

Generell weist der Träger 18 bzw. die zweite Gitterstruktur 12 eine hohe Biegeflexibilität auf, wobei die Biegung der zweiten Gitterstruktur 12 bzw. des Trägers 18 entlang einer Längsachse der zweiten Gitterstruktur 12 eine vergleichsweise hohe Biegekraft bzw. ein vergleichsweise hohes Biegemoment erfordert. Das führt dazu, dass der Träger 18 eine Streckung eines gekrümmten Blutgefäßes 30 bewirkt. Somit trägt der Träger 18 zu einer Reduktion der Strömungskomponente direkt in das Aneurysma 31 einströmender Gefäßströmung F_{G} bei. Die direkte Einströmung des Gefäßströmung F_{G} in das Aneurysma 31 wird somit reduziert.

Der Träger 18 kann im weitesten Sinne eine Stützstruktur bzw. Tragstruktur für das Netz 19 sein. Dabei ist es bevorzugt, dass der Träger 18 koaxial innerhalb des Netzes 19 angeordnet ist. Auf diese Weise kann der Träger 18 bzw. die zweite Gitterstruktur 12 zur Stabilisierung des Netzes 19 bzw. der ersten Gitterstruktur 11 eingesetzt werden. Insbesondere kann durch den Träger 18 das Expansionsverhalten des Netzes 19 kontrolliert werden.

Vorzugsweise bildet der Träger 18 die Innenschicht 15 des rohrförmigen Körpers 10. Alternativ kann der Träger 18 die Außenschicht 14 bilden. Damit wird durch Träger 18 eine gute Stabilisierung des Blutgefäßes 30 und eine Expansion des rohrförmigen Körpers 10 bis zu einem zuvor eingestellten Querschnittsdurchmesser erreicht. Insgesamt ermöglicht der Träger 18 eine gute und kontrollierbare Expansion des rohrförmigen Körpers 10 und des Netzes innerhalb des Trägers 18.

Nachfolgend werden bevorzugte Varianten des Netzes 19 bzw. der ersten Gitterstruktur 11 beschrieben:

Vorzugsweise weist das Netz 19 eine höhere Feinmaschigkeit auf als der Träger 18. Das Netz 19 übernimmt somit vorwiegend die Funktion der Strömungsbeeinflussung gegenüber dem Aneurysma 31. Vorzugsweise ist die Feinmaschigkeit des Netzes 19 so begrenzt, dass die Einströmung von Blut in das Aneurysma 31 nicht vollständig verhindert ist. Vielmehr soll mit dem Netz 19 erreicht werden, dass einerseits eine Ruptur des Aneurysmas 31 vermieden und andererseits eine ausreichende Nährstoffversorgung und mechanische Belastung der Aneurysmenwand 34 aufrechterhalten ist, so dass eine Degenerierung der Zellen der Aneurysmenwand 34 vermieden wird. Hinsichtlich der Feinmaschigkeit des Netzes 19 ist es daher vorteilhaft, wenn das Netz 19 höchstens 48, insbesondere höchstens 44, insbesondere höchstens 40, insbesondere höchstens 36, insbesondere höchstens 32, insbesondere höchstens 24, insbesondere höchstens 20, insbesondere höchstens 16, insbesondere höchstens 12, Drähte 112 umfasst. Die Stabilisierung des Netzes 19 erfolgt durch den Träger 18, so dass hinsichtlich der Radialkraft keine besonderen Anforderungen an das Netz 19 bestehen. Um die Crimpbarkeit zu verbessern, ist daher vorteilhaft vorgesehen, den Drahtdurchmesser der Drähte 112 der ersten Gitterstruktur 11 bzw. des Netzes 19 gegenüber dem Querschnittsdurchmesser der Drähte 122 der zweiten Gitterstruktur 12 bzw. des Trägers 18 zu reduzieren. Vorzugsweise weisen die Drähte 112 der ersten Gitterstruktur 11 bzw. des Netzes 19 einen Querschnittsdurchmesser von höchstens 77 µm, insbesondere höchstens 51 µm, insbesondere höchstens 46 µm, insbesondere höchstens 41 µm, insbesondere höchstens 36 µm, insbesondere höchstens 26 µm, insbesondere höchstens 20 µm auf. Dies gilt für den Einsatz des rohrförmigen Körpers 10 bzw. allgemein der medizinischen Vorrichtung in Blutgefäßen 30, die einen Gefäßdurchmesser von 2mm bis 6 mm aufweisen. Bei einem Gefäßdurchmesser von mehr als 6 mm ist es vorteilhaft, wenn die Drähte 112 der ersten Gitterstruktur 11 bzw. des Netzes 19 einen Querschnittsdurchmesser von höchstens 155 µm, insbesondere höchstens 105 µm, insbesondere höchstens 77 µm, insbesondere höchstens 51 µm, insbesondere höchstens 46 µm, insbesondere höchstens 41 µm, insbesondere höchstens 36 µm, insbesondere höchstens 26 µm, insbesondere höchstens 20 µm aufweisen.

Vorzugsweise bildet das Netz 19 die Außenschicht 14 des rohrförmigen Körpers 10. Das hochflexible Netz 19 wird bei der Expansion des rohrförmigen Körpers 10 durch den Träger 18 gestützt bzw. in den expandierten Zustand gedrängt. Durch das Zusammenspiel zwischen Träger 18 und Netz 19 wird vermieden, dass sich das Netz 19 nicht ausreichend im Blutgefäß 30 faltet. Der die Innenschicht 15 bildende Träger 18 stützt das Netz 19 vorzugsweise über die gesamte Länge des Netzes 19.

Die erste Gitterstruktur 11 und die zweite Gitterstruktur 12 sind punktuell miteinander verbunden. Vorzugsweise erfolgt die Verbindung der ersten Gitterstruktur 11 mit der zweiten Gitterstruktur 12 an einem proximalen Ende des rohrförmigen Körper 10. Insbesondere weist die erste Gitterstruktur 11 ein proximales Ende 110 auf, das mit einem proximalen Ende 120 der zweiten Gitterstruktur 12 verbunden ist.

In diesem Zusammenhang wird darauf hingewiesen, dass im Rahmen der Anmeldung proximal angeordnete Elemente näher am Anwender angeordnet sind als distale Elemente.

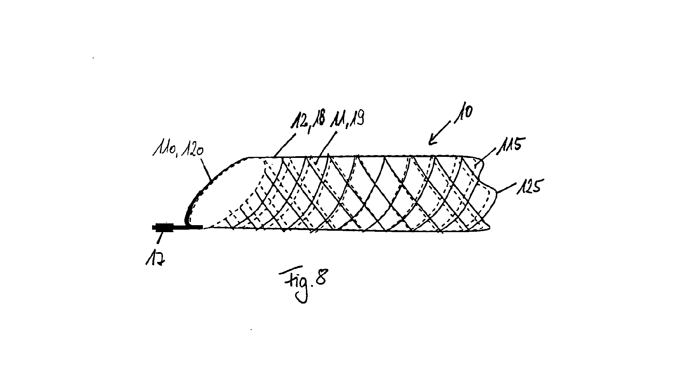

Vorzugsweise weisen die erste Gitterstruktur 11 und die zweite Gitterstruktur 12 jeweils ein schräg zulaufendes proximales Ende 110, 120 auf. Die Drähte 112, 122 der ersten Gitterstruktur 11 und der zweiten Gitterstruktur 12 laufen an den schräg angeordneten proximalen Enden 110, 120 zusammen. Die zusammenlaufenden Drähte 112, 122 sind miteinander verbunden. Eine derartige Verbindung der ersten und zweiten Gitterstruktur 11, 12 ist beispielhaft in Figur 8 dargestellt. Die Verbindung der zusammenlaufenden Drähte 112, 122 der ersten Gitterstruktur 11 und der zweiten Gitterstruktur 12 erfolgt gemäß dem Ausführungsbeispiel nach Figur 8 durch eine Verbindungshülse 17. Die zusammenlaufenden Drähte 112, 122 der ersten und zweiten Gitterstruktur 11, 12 können im Bereich der Verbindungshülse 17 parallel zueinander verlaufen oder miteinander verdrillt sein. Ferner kann vorgesehen sein, dass die Drähte 112 der ersten Gitterstruktur 11 im Bereich der Verbindungshülse 17 die Drähte 122 der zweiten Gitterstruktur 12 vollständig umhüllen. Dies entspricht im Wesentlichen der Anordnung, wie in der Querschnittsansicht gemäß Figur 6b dargestellt. Umgekehrt ist es auch möglich, dass die Drähte 122 der zweiten Gitterstruktur 12 im Bereich der Verbindungshülse 17 die Drähte 112 der ersten Gitterstruktur 11 vollständig umhüllen. Andere Verbindungsarten sind möglich. Beispielsweise können die erste Gitterstruktur 11 und die zweite Gitterstruktur 12 bzw. das Netz 19 und der Träger 18 in einem mittleren Bereich des rohrförmigen Körpers 17 miteinander verbunden sein. Ferner ist eine Verbindung zwischen der ersten und zweiten Gitterstruktur 11, 12 zwischen Netz 19 und dem Träger 18 an einem distalen Ende des rohrförmigen Körpers 10 denkbar, zB. an den Schlaufen der Gitterstruktur. Eine Verbindung im schrägen Bereich ist möglich.

Vorzugsweise weisen die Gitterstrukturen 11, 12 ein Drahtgeflecht auf, das einem proximalen Ende 110, 120 bzw. allgemein an einem axialen Ende der jeweiligen Gitterstruktur 11, 12 einen schrägen Verlauf aufweist. Derartige Drahtgeflechte sind in der nachveröffentlichten deutschen Patentanmeldung Nr. 10 2009 056 450 beschrieben, die auf die Anmelderin zurückgeht.

Um die erfindungsgemäße Doppelfunktion zu erreichen, nämlich einerseits eine ausreichende Fixierung des rohrförmigen Körpers 10 bzw. des Stents im Blutgefäß 30 zu ermöglichen und andererseits die Strömung in ein Aneurysma 31 gezielt zu beeinflussen, ohne eine Degenerierung der Muskelzellen der Aneurysmenwand 34 zu fördern, ist eine gezielte Einstellung der Flechtwinkel der einzelnen Drahtgeflechte bzw. Gitterstrukturen 11, 12 zweckmäßig. Nachfolgend werden bevorzugte Ausführungsbeispiele für unterschiedliche Flechtwinkel beschrieben:

Bei herkömmlichen Aneurysmenstents 40 ist vorgesehen, den Flechtwinkel möglichst groß zu wählen, damit der Aneurysmenhals 32 höchstmöglich verschlossen wird. Der Nachteil derartiger Behandlungsmöglichkeiten besteht in einem erhöhten Risiko einer Blutung, durch Degenerierung der Zellen der Aneurysmenwand und der Entstehung sich vergrößernder frischer Thromben, einem hohen Foreshortening, das eine Positionierung der herkömmlichen Aneurysmenstents 40 erschwert und eine ungenügende Anpassung an variable Gefäßdurchmesser. Im Allgemeinen gilt, dass die Längenänderung eines herkömmlichen Aneurysmenstents bei einer Durchmesseränderung umso größer ist, je größer der Flechtwinkel gewählt wurde. Das erschwert eine reproduzierbare und einstellbare Konfiguration bekannter Aneurysmenstents 40.

Bei der medizinischen Vorrichtung ist daher vorteilhaft vorgesehen, dass der Flechtwinkel begrenzt ist. Insbesondere hat es sich als zweckmäßig erwiesen, wenn der Flechtwinkel höchstens 70°, insbesondere höchstens 67°, insbesondere höchstens 65°, insbesondere höchstens 63°, insbesondere höchstens 60°, beträgt. Dabei bezieht sich der Flechtwinkel auf den spitzen Winkel, der zwischen einem Draht der Gitterstruktur und der Längsachse des rohrförmigen Körpers 10 gebildet ist. Es wurde festgestellt, dass bei einem Flechtwinkel von 60° eine Verkürzung der jeweiligen Gitterstruktur 11, 12 bei der Expansion, d.h. bei der Überführung des rohrförmigen Körpers 10 von einem komprimierten Zustand in einen expandierten Zustand, in Höhe von 50 % erfolgt. Das bedeutet, dass die Gitterstruktur 11, 12 mit einem Flechtwinkel von 60°, die eine Länge im komprimierten Zustand, also innerhalb des Zuführsystems 20, von 40 mm aufweist, im expandierten Zustand, insbesondere im Blutgefäß 30, eine Länge von 20 mm oder etwas mehr als 20mm umfasst.

Vorzugsweise ist ein Flechtwinkel für die erste Gitterstruktur 11 oder die zweite Gitterstruktur 12 vorgesehen, der höchstens 60°, insbesondere höchstens 59°, insbesondere höchstens 58°, insbesondere höchstens 57°, insbesondere höchstens 56°, insbesondere höchstens 55°, insbesondere höchstens 54°, insbesondere höchstens 53°, insbesondere höchstens 52°, insbesondere höchstens 51°, insbesondere höchstens 50°, insbesondere höchstens 45° beträgt. Dadurch wird erreicht, dass sich die Verkürzung des rohrförmigen Körpers 10 bei der Expansion bzw. Freilassung in ein Blutgefäß 30 in einen akzeptablen Bereich bewegt. Der Foreshorteningeffekt wird somit reduziert. Da der rohrförmige Körper 10 üblicherweise überdimensioniert ist, also im Herstellzustand einen größeren Querschnittsdurchmesser aufweist als im implantierten Zustand innerhalb des Blutgefäßes 30, weist die jeweilige Gitterstruktur 11, 12 im Blutgefäß 30 einen Flechtwinkel von etwa 30° bis 50° auf, so dass der rohrförmige Körper 10 eine vergleichsweise gute Flexibilität aufweist.

In diesem Zusammenhang wird darauf hingewiesen, dass sich Dimensionsangaben für die medizinische Vorrichtung, insbesondere den rohrförmigen Körper 10 im Rahmen der Anmeldung grundsätzlich auf den Herstellzustand beziehen, soweit nichts Gegenteiliges angegeben ist.

Die erste Gitterstruktur 11 und die zweite Gitterstruktur 12 bzw. das Netz 19 und der Träger 18 können unterschiedliche Flechtwinkel aufweisen. Dadurch wird erreicht, dass sich bei der Expansion des rohrförmigen Körpers 10 unterschiedliche Foreshorteningeffekte einstellen. Mit anderen Worten zweigen der Träger 18 und das Netz 19, die unterschiedliche Flechtwinkel aufweisen, bei der Entlassung des rohrförmigen Körpers 10 aus einem Zuführsystem 20 ein unterschiedliches Verkürzungsverhalten. Dies gilt auch im implantierten Zustand, wenn auf den rohrförmigen Körper 10 Blutdruckschwankungen in Folge des pulsatielen Blutflusses wirken. Die beiden Schichten 14, 15, also der Träger 18 und das Netz 19, werden also unterschiedlich durch den pulsatielen Blutstrom beeinflusst. Dabei kann in besonders bevorzugter Weise vorgesehen sein, dass sich zwischen dem Träger 18 und dem Netz 19 bzw. zwischen der Außenschicht 14 und der Innenschicht 15, insbesondere zwischen der ersten Gitterstruktur 11 und der zweiten Gitterstruktur 12, ein Spalt 16 ausbildet. Der Spalt 16 kann als Polster wirken. Der Spalt entsteht bspw. dadurch, dass die Geflechte in einem Bereich punktuell verbunden werden und in mindestens einem bereich unterschiedliche Flechtwinkel aufweisen. Der Flechtwinkel kann entlang der Längsachse variieren.

In einer bevorzugten Variante ist der Flechtwinkel des Trägers 18 kleiner als der Flechtwinkel des Netzes 19. Dadurch wird bewirkt, dass sich das Netz 19 bei der Entlassung aus einem Zuführsystem stärker verkürzt als der Träger 18. Dadurch kann die Positionierung des rohrförmigen Körpers 10 insgesamt vereinfacht werden, da der Träger 18 bei der Expansion einen geringfügigen Foreshorteningeffekt zeigt. Im Allgemeinen wird der rohrförmige Körper 10 aus einem Zuführsystem 20 entlassen, in dem das Zuführsystem mit dem komprimierten rohrförmigen Körper 10 an den Behandlungsort geführt wird. Das Zuführsystem 20 wird anschließend in proximale Richtung gezogen, wogegen der rohrförmige Körper 10 ortsfest gehalten wird. Um das Foreshortening zu kompensieren, kann vorgesehen sein, ein proximales Ende des rohrförmigen Körpers 10 gleichzeitig mit der proximalen Bewegung des Zuführsystems 20 leicht in distale Richtung zu schieben. Dadurch wird verhindert, dass die Außenschicht 14 des rohrförmigen Körpers 10 entlang der Gefäßwand 35 des Blutgefäßes 30 gezogen wird und eine Verletzung der Gefäßwand 35 verursacht. Indem der Träger 18 einen kleineren Flechtwinkel aufweist als das Netz 19, wird erreicht, dass bei der Expansion des rohrförmigen Körpers 10 das proximale Ende 120 der zweiten Gitterstruktur 12 bzw. des Trägers 18 nur um einen kleinen Betrag in distale Richtung gegen das Foreshortening geschoben werden muss. Die Positionierung des rohrförmigen Körpers 10 bzw. des mehrlagigen Stents wird somit erleichtert. Das Netz 19 bzw. die erste Gitterstruktur 11 erfährt hingegen eine stärkere Verkürzung, wodurch sich die Feinmaschigkeit des Netzes 19 bei der Expansion erhöht. Auf diese Weise kann durch eine insgesamt kleinere Anzahl von Drähten 112, 122 eine vergleichsweise hohe Feinmaschigkeit erreicht werden.

Außerdem kann das Außenelement, bzw. die äußere Gitterstruktur ein kleineres foreshortening als das Innenelement bzw. die innere Gitterstruktur aufweisen, so dass das Außenelement gut an der Gefäßwand anliegt.

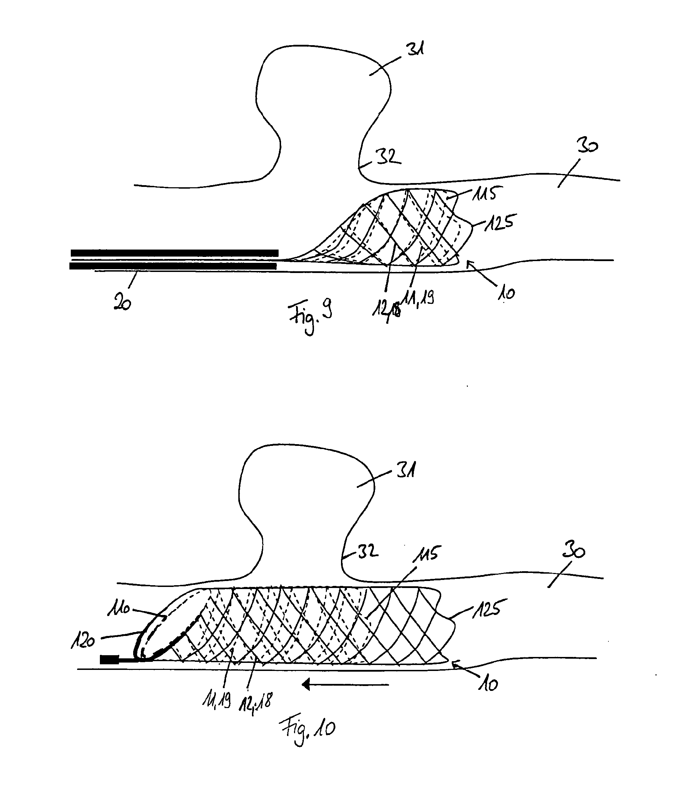

In Figur 9 ist die Freisetzung des rohrförmigen Körpers 10 aus einem Zuführsystem 20 in ein Blutgefäß 30 gezeigt. Bei der Entlassung eines distalen Endes des rohrförmigen Körpers 10 entfalten sich zunächst das distale Ende 115 der ersten Gitterstruktur 11 und das distale Ende 125 der zweiten Gitterstruktur 12 gleichzeitig bzw. auf gleicher Höhe. Durch das weitere Zurückziehen des Zuführsystems 20 in proximale Richtung wird eine Relativbewegung zwischen der ersten Gitterstruktur 11 und der zweiten Gitterstruktur 12 bewirkt, da mit weiterer Entlassung des rohrförmigen Körpers 10 der Foreshorteningeffekt der ersten Gitterstruktur 11 größer ausgeprägt ist als bei der zweiten Gitterstruktur 12. Somit erfolgt eine proximale Verschiebung des Netzes 19 gegenüber dem Träger 18. Diese proximale Verschiebung des Netzes 19 gegenüber dem Träger 18 ist durch den größeren Flechtwinkel des Netzes 19 bedingt. Vorzugsweise ist das Netz 19 außen auf dem Träger 18 angeordnet. Das Netz 19 bildet somit die Außenschicht 14 der Umfangswandung des rohrförmigen Körpers 10. Der Träger 18 bildet hingegen die Innenschicht 15 der Umfangswandung. Alternativ kann vorgesehen sein, dass das Netz 19 die Innenschicht 15 und der Träger 18 die Außenschicht 14 bildet. Das hat den Vorteil, dass das Netz 19 auf Drähten 122 des Trägers 18 bei der Expansion gleiten kann. Das außenangeordnete Netz 19 weist einen kleineren Flechtwinkel als der Träger 18 auf und damit auch einen kleinere Foreshorteningeffekt als der Träger 18.

Wenn das Netz in der bevorzugten Ausgestaltung die Außenschicht 14 bildet, erfolgt der Kontakt zwischen dem rohrförmigen Körper 10 und der Gefäßwand 35 des Blutgefäßes 30 zunächst durch das Netz 19. Zur Kompensation des geringfügigen Foreshortenings des Trägers 18 werden die Gitterstrukturen 11, 12 bzw. das proximale Ende des rohrförmigen Körpers 10 in distale Richtung geschoben. Dabei bewegt sich der innere Träger 18 bzw. die Innenschicht 15 in distale Richtung. Der Träger 18 kann dabei entlang der Gefäßwand 35 gleiten. Da der Träger 18 einen relativ kleinen Flechtwinkel aufweist, ist es möglich, den Träger 18 entlang der Gefäßwand 35 zu schieben, ohne dass der Träger 18 bzw. die zweite Gitterstruktur 12 gestaucht wird. Bei dem vergleichsweise kleinen Flechtwinkel des Trägers 18 weisen die Drähte 122 der zweiten Gitterstruktur 12 bzw. des Trägers 18 eine Richtungskomponente auf, die in Längsrichtung, also parallel zur Längsachse, des rohrförmigen Körpers 10 besonders ausgeprägt ist. Dadurch führt ein Schieben des Trägers 18 bzw. der zweiten Gitterstruktur 12 nicht zwangsläufig zu einer Änderung des Anstellwinkels bzw. Flechtwinkels der Drähte 122 der zweiten Gitterstruktur 12. Somit wird durch den kleinen Flechtwinkel des Trägers 18 ein Stauchen des Trägers 18 bei einer distalen Bewegung des Trägers 18 vermieden. Nach vollständiger Expansion des rohrförmigen Körpers 10 weist der Träger 18 bzw. die zweite Gitterstruktur 12 eine größere Längserstreckung auf, als das Netz 19 bzw. die erste Gitterstruktur 11. Das Netz 19 unterliegt also einem größeren Foreshorteningeffekt als der Träger 18, so dass der Träger 18 im expandierten Zustand des rohrförmigen Körpers 10 nicht vollständig, sondern nur teilweise bzw. abschnittsweise mit dem Netz 19 bedeckt ist (Figur 10). Das Netz 19 ist im komprimierten Zustand länger als der Träger 18. Im expandierten Zustand können sich die Länge des Netzes 19 und des Trägers 18 entsprechen bzw. kann der Längenunterschied sich verringern.

Durch die unterschiedlichen Flechtwinkel zwischen der ersten Gitterstruktur 11 und der zweiten Gitterstruktur 12 weisen die erste Gitterstruktur 11 und die zweite Gitterstruktur 12 bzw. das Netz 19 und der Träger 18 bei Pulsation des Gefäßes unterschiedliches Verhalten auf. Im implantierten Zustand innerhalb des Blutgefäßes 30 kann sich zwischen der ersten Gitterstruktur 11 und der zweiten Gitterstruktur 12 bzw. zwischen dem Netz 19 und dem Träger 18 ein Spalt 16 ausbilden, insbesondere während der Systole. Durch die Streckung E des Blutgefäßes 30 während der Systole bewirkt der Foreshorteningeffekt, dass der Träger 18, der einen vergleichsweise kleinen Flechtwinkel aufweist, zumindest abschnittsweise verjüngt wird bzw. zumindest abschnittsweise eine Verjüngung R zeigt. Das Netz 19 weist hingegen während der Systole und der Streckung E des Blutgefäßes keine oder zumindest eine wesentlich geringere Verjüngung R auf. Somit entsteht zwischen dem Träger 18 und dem Netz 19 ein Spalt 16 bzw. ein Zwischenraum oder Polster. Das Verhalten der Gitterstrukturen 11, 12 bzw. des Träger 18 und des Netzes 19 mit unterschiedlichen Flechtwinkeln bei einer Streckung E des Blutgefäßes ist beispielhaft in Figur 11 dargestellt.

Vorzugsweise ist das Netz 19 derart angepasst, dass das Netz 19 der Pulsation des Blutgefäßes 30 folgen kann. Insbesondere weist das Netz 19 gegenüber dem Träger 18 eine erhöhte Beweglichkeit auf, die beispielsweise durch entsprechend kleine Drahtdurchmesser und/oder entsprechende Einstellung des Flechtwinkels erreichbar ist. Während der Systole, also bei Anstieg des Blutdrucks, wird eine Durchgangsströmung F_{D} bewirkt, wobei Blut aus dem Blutgefäß 30 in ein Aneurysma 31 einströmt und zu einer Volumenerhöhung im Aneurysma 31 führt. Die hohe Ausweitbarkeit des Netzes 19 ermöglicht dabei, dass sich ein Abschnitt des Netzes 19 radial nach außen bewegt bzw. radial nach außen ausgelenkt wird, wobei das Netz 19 abschnittsweise der Blutbewegung folgt. Ein Blutfluss durch die Maschen des Netzes 19 wird dabei behindert. Vorzugsweise ist die Ausweitbarkeit des Netzes 19 derart eingestellt, dass das Netz 19 hinsichtlich seiner Auslenkung durch die Blutströmung in das Aneurysma 31 eine Trägheit bzw. einen Widerstand zur Blutströmung aufweist. Das Netz 19 folgt also dem Blutdruckanstieg während der Systole durch eine radiale Bewegung nach außen. Der Blutdurchfluss durch die Maschen des Netzes 19 in das Aneurysma 31 wird gleichzeitig reduziert. Die abschnittsweise radiale Auslenkung des Netzes 19 unter Einfluss des systolischen Blutdruckanstiegs ist in Figur 12 dargestellt. Die Druckübertragung erfolgt nur bis zu einem bestimmten Grad, so dass es zur Gerinnselbildung und einer verringerten Degenerierung der Zellen kommt. Durch die Möglichkeit, die Ausweitbarkeit des Netzes 19 einstellen zu können bspw. durch Wahl des Flechtwinkels, kann die Druckübertragung geregelt werden.

Die Auslenkung des Netzes 19 in das Aneurysma 31 hat mehrere Vorteile. Einerseits ermöglicht das flexible Netz 19 die Übertragung der Druckwelle in das Aneurysma. Zwar wird eine Blutströmung in das Aneurysma behindert. Der Druck P wird hingegen in das Blutvolumen des Aneurysmas 31 übertragen. Dadurch wird eine periodische Belastung der Zellen der Aneurysmenwand 34 aufrechterhalten, wodurch einer Degenerierung der Zellen entgegengewirkt wird. Gleichzeitig wird der direkte Blutdurchfluss also die Durchgangsströmung F_{D} reduziert, wodurch die Endotelialisierung des rohrförmigen Körpers 10 gefördert wird. Durch die reduzierte Durchgangsströmung F_{D} wird die Ansiedlung von Endotelzellen und Anhaftung der Endotelzellen am rohrförmigen Körper 10 erleichtert. Insbesondere das Zuwachsen der Maschen der Gitterstrukturen 11, 12 wird erleichtert, da durch die Maschen der ersten Gitterstruktur 11 bzw. des Netzes 19 keine oder eine nur geringfügige Durchgangsströmung F_{D} besteht.

Im Allgemeinen hat sich gezeigt, dass es vorteilhaft ist, eine Gitterstruktur bereitzustellen, die derart flexibel ist, dass sie zumindest abschnittsweise in das Aneurysma 31 ausgelenkt werden kann. Dies ist beispielsweise durch eine Gitterstruktur ermöglicht, die einen vergleichsweise kleinen Flechtwinkel aufweist. Eine derartige Gitterstruktur erfährt jedoch eine erhöhte Verjüngung R, wenn das Blutgefäß 30, beispielsweise in Folge des Pulsschlages, einer Streckung E unterliegt. Diesen Effekt zeigen Gitterstrukturen mit einem großen Flechtwinkel nicht. Konkret ermöglicht eine Gitterstruktur mit einem großen Flechtwinkel eine hohe Streckung, ohne signifikante Änderung des Querschnittsdurchmessers. Derartige Gitterstrukturen ermöglichen jedoch keine abschnittsweise Auswölbung, beispielsweise in ein Aneurysma 31.

Mit der erfindungsgemäßen Vorrichtung werden beide Effekte erzielt, wobei die Funktionen unterschiedlichen Gitterstrukturen 11, 12 zugeordnet sind. Die zweite Gitterstruktur 12 bzw. der Träger 18 ermöglicht die genaue und sichere Positionierung des rohrförmigen Körpers 10 im Blutgefäß. Das Netz 19 hingegen ermöglicht die Beeinflussung der Blutströmung in das Aneurysma 31, wobei gleichzeitig eine Übertragung des Blutdrucks ermöglicht ist. Somit wird die postoperative Gefahr einer Ruptur des Aneurysmas 31 reduziert.

In Figur 7 ist ein weiteres Ausführungsbeispiel der medizinischen Vorrichtung gezeigt, wobei das Netz 19 bzw. die erste Gitterstruktur 11 unterschiedliche Flechtwinkel aufweist. Insbesondere umfasst die erste Gitterstruktur 11 einen mittleren Abschnitt 111 und zwei Randabschnitte 116, wobei der mittlere Abschnitt 111 zwischen den Randabschnitten 116 angeordnet ist. Im mittleren Abschnitt 111 weist die erste Gitterstruktur 11 einen kleineren Flechtwinkel als in den Randabschnitten 116 auf. Der vergleichsweise größere Flechtwinkel in den Randabschnitten 116 führt dazu, dass die Randabschnitte 116 die Streckung E des Blutgefäßes 30 kompensieren, weil diese sich ohne signifikante Durchmesseränderung strecken lassen. Insbesondere bilden sich zwischen dem mittleren Abschnitt 111 und den Randabschnitten 116 Übergangsabschnitte 113 aus, die durch die punktierten Linien in Figur 7 hervorgehoben sind. In den Übergangsabschnitten 113 erfolgt eine Verkürzung K, die gegenläufig zur Streckung E des Blutgefäßes 30 orientiert ist. Der mittlere Abschnitt 111 weist gegenüber den Randabschnitten 116 eine höhere Ausweitbarkeit auf, so dass der mittlere Abschnitt 111 eine stärkere Verkürzung unter Einfluss eines systolischen Blutdruckanstiegs aufweisen kann als die Randabschnitte 116. Der mittlere Abschnitt 111 kann also dem Blutdruckanstieg folgen, so dass sich das Netz 19 bzw. die erste Gitterstruktur 11 im mittleren Abschnitt 111 in das Aneurysma 31 wölben bzw. eine Auswölbung B bilden kann. Aus Gründe der Übersichtlichkeit ist der Träger 18 bzw. die zweite Gitterstruktur 12 in Figur 7 nicht dargestellt.

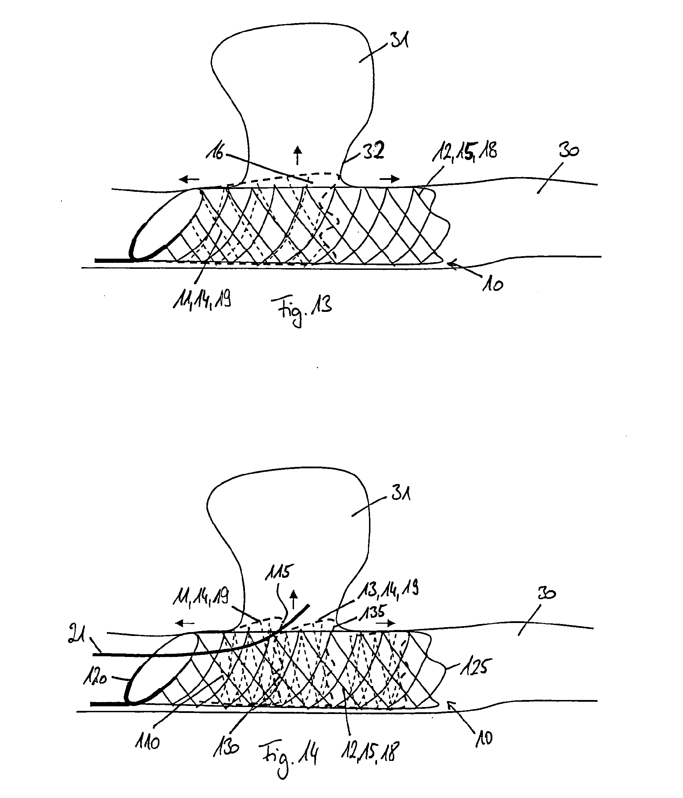

Ein weiteres Ausführungsbeispiel ist in Figur 13 dargestellt, wobei vorgesehen ist, dass das Netz 19 gegenüber dem Träger 18 eine kürzere Längserstreckung aufweist. Insbesondere im implantierten Zustand innerhalb des Blutgefäßes 30 weist das Netz 19 bzw. die erste Gitterstruktur 11 eine wesentlich kürzere Längserstreckung als der Träger 18 bzw. die zweite Gitterstruktur 12 auf. Vorzugsweise ist der rohrförmige Körper 10 dabei derart im Blutgefäß 30 positioniert, dass das distale Ende 115 der ersten Gitterstruktur 11 bzw. des Netzes 19 im Bereich des Aneurysmenhalses 32 angeordnet ist. Das distale Ende der ersten Gitterstruktur 115 bildet dabei ein freies Ende. Das proximale Ende 110 der ersten Gitterstruktur 11 ist hingegen mit dem proximalen Ende 120 der zweiten Gitterstruktur 12 verbunden. Das frei Ende bzw. distale Ende 115 der ersten Gitterstruktur 11 kann sich radial nach außen aufweiten. Dabei wird das distale Ende 115 der ersten Gitterstruktur 11 bzw. des Netzes 19 in das Aneurysma 31 bzw. in den Bereich des Aneurysmenhalses 32 radial nach außen ausgelenkt. Mit anderen Worten kann das Netz 19 abschnittsweise in das Aneurysma 31 ausgelenkt werden. Das Netz 19 bewegt sich daher bei einem systolischen Blutdruckanstieg mit dem Blutdruckanstieg in das Aneurysma 31 hinein, so dass die Durchgangsströmung F_{D} durch die Maschen des Netzes 19 reduziert sind. Das distale Ende ist länger und sichert die Anhaftung.

Ein weiteres Ausführungsbeispiel ist in Figur 14 dargestellt, wobei der rohrförmige Körper 10 eine Umfangswandung umfasst, die eine Innenschicht 15 und eine Außenschicht 14 aufweist. Die Innenschicht 15 ist durch die zweite Gitterstruktur 12 gebildet, die als Träger 18 ausgebildet ist. Die Außenschicht 14 weit die erste Gitterstruktur 11 und eine dritte Gitterstruktur 13 auf, wobei die erste Gitterstruktur 11 und die dritte Gitterstruktur 13 jeweils ein Netz 19 bilden. Die erste und dritte Gitterstruktur 11, 13 sind jeweils mit ihren proximalen Enden 110, 130 mit der zweiten Gitterstruktur 12 verbunden. Die distalen Enden 115, 135 der ersten Gitterstruktur 11 und der dritten Gitterstruktur 13 sind frei angeordnet. Das proximale Ende 130 der dritten Gitterstruktur 13 ist im Wesentlichen auf Höhe des distalen Endes 115 der ersten Gitterstruktur 11 mit der zweiten Gitterstruktur 12 verbunden. Somit ergibt sich im Wesentlichen eine schuppenartige Anordnung der ersten und dritten Gitterstruktur 11, 13. Die erste Gitterstruktur 11 und die dritte Gitterstruktur 13 können sich auch zumindest abschnittsweise überlappen oder beabstandet angeordnet sein.

Die erste Gitterstruktur 11 und die dritte Gitterstruktur 13 bzw. die Netze 19 können eine klappenartige Funktion aufweisen. Durch die freie Beweglichkeit der distalen Enden 115, 135 ermöglichen die Netze 19 bzw. die erste und dritte Gitterstruktur 11, 13 eine gewisse Druckübertragung in das Aneurysma 31. Gleichzeitig ermöglicht die Klappenfunktion der Netze 19 die Zufuhr von zusätzlichen Behandlungsvorrichtungen in das Aneurysma 31. Derartige Behandlungsvorrichtungen können beispielsweise einen Coilkatheter 21 umfassen.

Grundsätzlich ist die Erfindung nicht auf zwei Netze 19 bzw. zwei Gitterstrukturen 11, 13 eingeschränkt, die die Außenschicht 14 bilden. Vielmehr ist im Rahmen der Erfindung auch vorgesehen, dass drei oder mehr Netze 19 bzw. Gitterstrukturen 11, 13 die Außenschicht 14 der Umfangswandung des rohrförmigen Körpers 10 bilden.

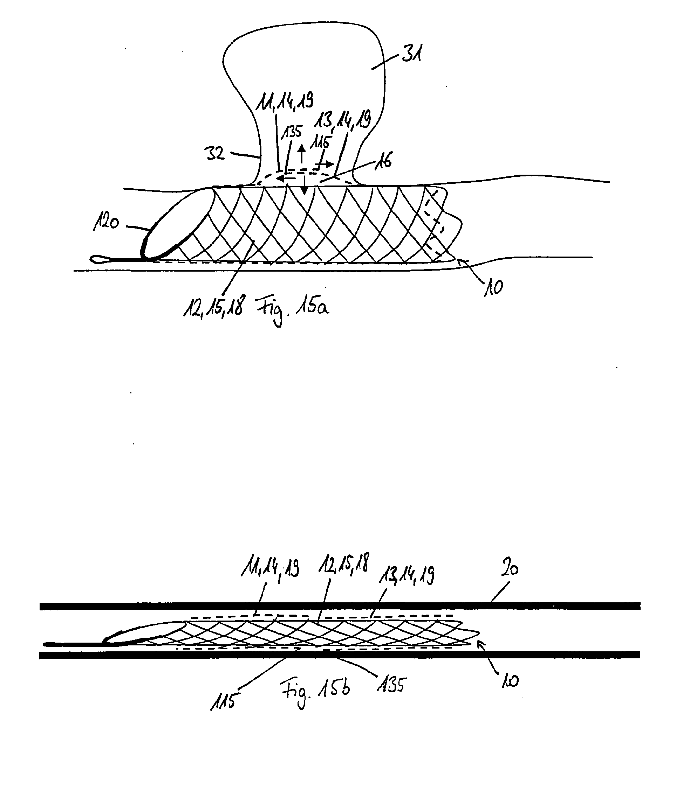

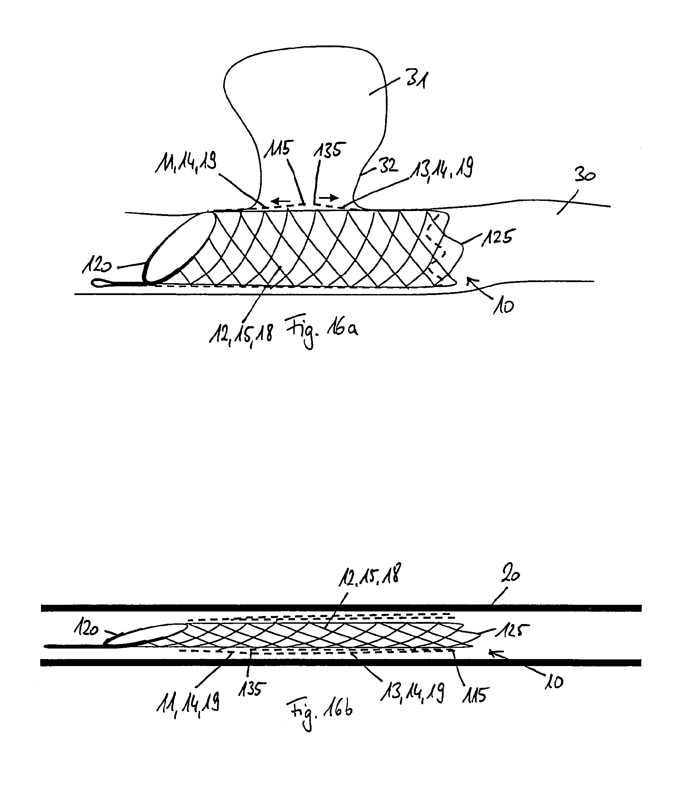

In Figur 15a ist ein weiteres Ausführungsbeispiel gezeigt, das sich von dem Ausführungsbeispiel gemäß Figur 14 dadurch unterscheidet, dass die erste Gitterstruktur 11 und die dritte Gitterstruktur 13 im Wesentlichen entgegengesetzt ausgerichtet sind. Konkret ist bei dem Ausführungsbeispiel gemäß Figur 15a vorgesehen, dass die erste Gitterstruktur 11 ein proximales Ende 110 umfasst, das mit dem proximalen Ende 120 der zweiten Gitterstruktur 12 verbunden ist. Das distale Ende 115 der ersten Gitterstruktur 11 ist frei angeordnet. Die dritte Gitterstruktur 13 umfasst hingegen ein distales Ende 135, das mit dem distalen Ende 125 der zweiten Gitterstruktur 12 verbunden ist. Das proximale Ende 130 der dritten Gitterstruktur 13 ist frei angeordnet. Ferner ist bei dem Ausführungsbeispiel gemäß Figur 15a vorgesehen, dass sich die freien Enden, also das distale 115 der ersten Gitterstruktur 11 und das proximale Ende 130 der dritten Gitterstruktur 13 überlappen. Dies gilt zumindest für den expandierten bzw. implantierten Zustand des rohrförmigen Körpers 10. Alternativ kann auch vorgesehen sein, dass sich die erste und dritte Gitterstruktur 11, 13 im expandierten bzw. implantierten Zustand des rohrförmigen Körpers 10 nicht überlappen, sondern vielmehr fluchtend zueinander angeordnet sind, wie in Figur 16a dargestellt.

Bei dem Ausführungsbeispiel gemäß Figur 15a ist ferner vorteilhaft vorgesehen, dass sich die Netze 19 bzw. die erste und dritte Gitterstruktur 11, 13 im expandierten Zustand überlappen, im komprimierten Zustand des rohrförmigen Körpers 10 hingegen fluchtend zueinander angeordnet sind. Dazu ist vorteilhaft vorgesehen, dass der Träger 18 bzw. die zweite Gitterstruktur 12 bei der Expansion des rohrförmigen Körpers 10 stärker verkürzt wird, als die Netze 19. Wenigstens ein Netz 19 oder mehrere Netze 19 weisen insbesondere einen kleineren Flechtwinkel als der Träger 18 auf, so dass sich der Träger 18 bei der Expansion des rohrförmigen Körpers 10 stark verkürzt wodurch sich im expandierten Zustand die Überlappung der Netze 19 einstellt. Durch die Überlappung wird sichergestellt, dass der Aneurysmenhals 32 vollständig bedeckt ist. Gleichzeitig ermöglicht die Beweglichkeit der freien Enden der ersten und dritten Gitterstruktur 11, 13 eine Relativbewegung der beiden Gitterstrukturen 11, 13 bzw. Netze 19 zueinander, so dass eine effiziente Druckübertragung in das Aneurysma 31 ermöglicht ist. Gleichzeitig wird durch die abschnittsweise Überlappung der ersten und dritten Gitterstruktur 11, 13 die Feinmaschigkeit im Bereich des Aneurysmenhalses 32 erhöht und die Blutströmung in das Aneurysma 31 vorteilhaft beeinflusst. Um eine derartige Feinmaschigkeit mit einem einzigen Netz 19 bzw. einer einzigen Gitterstruktur 11 zu erreichen, ist hingegen ein vergleichsweise größeres Zuführsystem 20 erforderlich.

Die fluchtende Anordnung der Netze 19 innerhalb des Zuführsystems 20, also im komprimierten Zustand, reduziert den Crimpdurchmesser, so dass kleine Zuführsysteme 20 einsetzbar sind (Figur 15b).

Bei dem Ausführungsbeispiel gemäß Figur 16a ist vorgesehen, dass die gegenüber angeordneten Netze 19 bzw. die erste und dritte Gitterstruktur 11, 13 im Unterschied zu dem Ausführungsbeispiel gemäß Figur 15a einen vergleichsweise größeren Flechtwinkel aufweisen. Dadurch wird die Feinmaschigkeit der einzelnen Netze 19 bzw. der ersten und dritten Gitterstruktur 11, 13 erhöht. Eine derartige Konfiguration bewirkt, dass sich die Netze 19 bei der Expansion stark verkürzen. Um eine genaue Positionierung des rohrförmigen Körpers 10 im Blutgefäß zu ermöglichen, weist der Träger 18 bzw. die zweite Gitterstruktur 12 hingegen vorteilhafterweise einen vergleichsweise kleinen Flechtwinkel auf, so dass der Foreshorteningeffekt reduziert ist.

Im expandierten bzw. implantierten Zustand des rohrförmigen Körpers 10 sind die erste Gitterstruktur 11 und die dritte Gitterstruktur 13 bzw. die beiden Netze 19 vorzugsweise fluchtend zueinander angeordnet. Dabei können sich die freien Enden der ersten und dritten Gitterstruktur 11, 13 berühren, so dass das Aneurysma 31 bzw. der Aneurysmenhals 32 vollständig durch die erste und dritte Gitterstruktur 11, 13 abgedeckt ist. Im komprimierten Zustand innerhalb des Zuführsystems 20 sind die erste und dritte Gitterstruktur 11, 13 hingegen überlagert, wie in Figur 16b dargestellt. Um trotz der Überlagerung bzw. Überlappung der ersten und dritten Gitterstruktur 11, 13 im Zuführsystem 20 einen möglichst kleinen komprimierten Querschnittsdurchmesser zu erreichen, ist vorgesehen, dass die Netze 19 bzw. die erste und dritte Gitterstruktur 11, 13 eine kleine Anzahl an Drähten aufweist. Durch den vergleichsweise großen Flechtwinkel, den die erste und dritte Gitterstruktur 11, 13 im Blutgefäß 30 einnehmen, wird auch bei einer geringen Anzahl von Drähten eine hohe Feinmaschigkeit sichergestellt. Ferner wird durch die geringe Anzahl an Drähten der Querschnittsdurchmesser im komprimierten Zustand des rohrförmigen Körpers 10 reduziert. Die Netze können auch innen angeordnet sein.

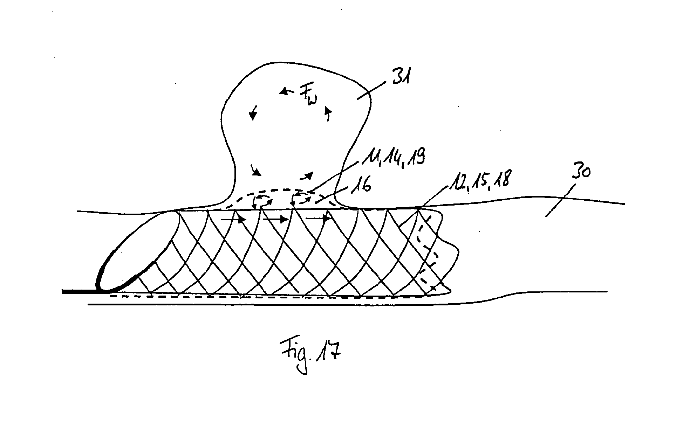

Wie zuvor bereits beschrieben, ermöglicht die Einstellung unterschiedlicher Flechtwinkel in separaten Schichten 14, 15 des rohrförmigen Körpers die Ausbildung eines Spalts 16 zwischen den Schichten 14, 15. Insbesondere kann sich das Netz 19 im expandierten Zustand des rohrförmigen Körpers 10 vom Träger 18 abheben. Die Ausbildung eines Spalts bei einer geeignet konfigurierten medizinischen Vorrichtung ist in Figur 17 dargestellt. Der Spalt 16 ermöglicht eine zusätzliche Beeinflussung der Strömungsverhältnisse im Aneurysma 31. Durch den Spalt 16 bzw. den Abstand zwischen dem Netz 19 und dem Träger 18 entsteht im Wesentlichen ein Polster, in dem Strömungswirbel auftreten, die durch Schubspannungen zwischen dem Blut innerhalb des Polsters bzw. Spalts 16 und der Gefäßströmung F_{G} bewirkt sind. Durch Strömungswirbel im Spalt 16 entsteht ein Energieverlust, wodurch die Strömungsgeschwindigkeiten innerhalb des Aneurysmas 31 reduziert werden. Insbesondere bewirkt die Gefäßströmung F_{G} keine direkte Wirbelströmung F_{W} im Aneurysma, sondern zunächst Strömungswirbel im Spalt 16. Zwar ist ein Blutaustausch zwischen dem Blutgefäß 30 und dem Aneurysma 31 ermöglicht, die Strömungsgeschwindigkeiten sind jedoch reduziert. Gleichzeitig ermöglicht die Ausweitbarkeit des Netzes 19 eine Druckübertragung in das Aneurysma 31, so dass zur Erhaltung der Zellen der Aneurysmenwand 34 einerseits über das Blut Nährstoffe an die Zellen gelangen und andererseits eine mechanische Beanspruchung beibehalten wird, die der Degenerierung entgegenwirkt. Gleichzeitig kann das Netz eine reduzierte bzw. mäßige Druckübertragung ermöglichen.

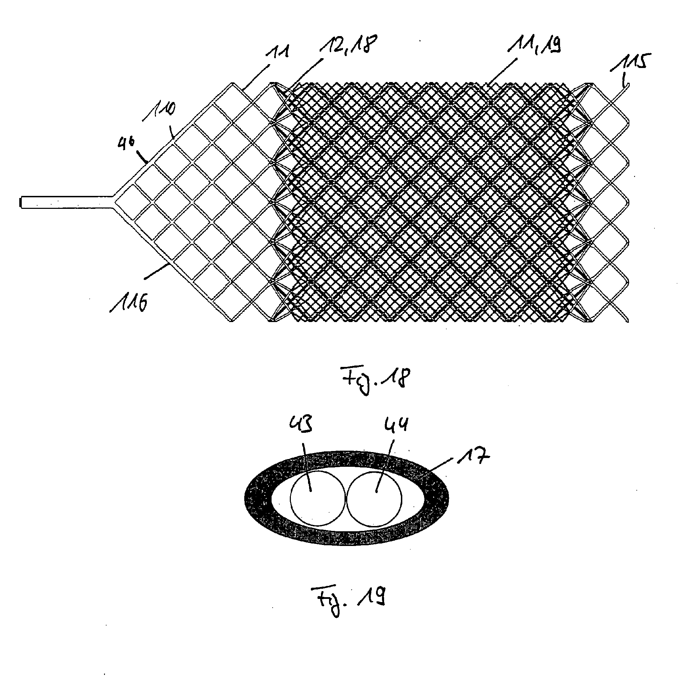

Ein Ausführungsbeispiel eines Gesamtsystems bzw. einer Vorrichtung mit Träger 18 und Netz 19 ist in Figur 18 dargestellt. Die Vorrichtung gemäß Figur 18 bildet ein wieder einziehbares Geflecht aufgrund der schrägen Spitze im proximalen Bereich der Vorrichtung. Bei der Vorrichtung handelt es sich um einen Stent, insbesondere um einen Aneurysmastent. Der Grundaufbau der Vorrichtung, insbesondere im Bereich der schrägen Spitze ist in der auf die Anmelderin zurückgehenden DE 10 2009 056 450 offenbart. Bei dem Grundaufbau handelt es sich um ein Geflecht aus Drahtelementen mit einer Reihe von Endmaschen bzw. Endschlaufen, die ein axiales Geflechtende begrenzen, wobei die Endmaschen äußere Drahtelemente umfassen, die eine Abschlusskante 46 des Geflechts bilden und in innere Drahtelemente übergehen, die innerhalb des Geflechts angeordnet sind. Ein erster Abschnitt der Abschlusskante 46 und ein zweiter Abschnitt der Abschlusskante 46 weist jeweils mehrere äußere Drahtelemente auf, die zusammen einen umlaufenden Rand der Abschlusskante 46 bilden. Der Rand ist derart angepasst, dass das axiale Geflechtende des Hohlkörpers in ein Zuführsystem einziehbar ist. Die äußeren Drahtelemente des ersten Abschnitts zur Bildung der Abschlusskante 46 sind dieser einander unmittelbar nachgeordnet und weisen jeweils eine erste Axialkomponente auf, die in Längsrichtung des Hohlkörpers verläuft. Die äußeren Drahtelemente des zweiten Abschnitts zur Bildung der Abschlusskante 46 sind entlang dieser einander unmittelbar nachgeordnet und weisen jeweils eine zweite Axialkomponente auf, die in Längsrichtung des Hohlkörpers verläuft und der ersten Axialkomponente entgegen gesetzt ist. Beide Axialkomponenten sind auf die selbe Umlaufrichtung des Randes bezogen.

Dieser Aufbau des proximalen Endes der Vorrichtung gilt für alle Ausführungsbeispiele dieser Anmeldung, bei dem die Vorrichtung eine in proximaler Richtung weisende schräge Spitze aufweist.

Bei dem Ausführungsbeispiel gemäß Figur 18 ist die erste Gitterstruktur 11 des Netzes 19 kongruent, d.h. deckungsgleich über der zweiten Gitterstruktur 12 des Trägers 18 angeordnet. Die zweite Gitterstruktur 12 des Trägers 18 ist daher nur im Bereich des Netzes 19 sichtbar, wo die Stränge bzw. einzelnen Drähte der beiden Gitterstrukturen 11, 12 nicht deckungsgleich sind. Die Stränge bzw. Drähte der zweiten Gitterstruktur 12 des Trägers 18 sind im Bereich des Netzes 19 schwarz gekennzeichnet und weisen einen größeren Durchmesser auf als die Drähte des Netzes 19.

Im Bereich der proximalen Spitze bzw. am proximalen Ende 110 der ersten Gitterstruktur 11 sowie am distalen Ende 115 der ersten Gitterstruktur 11 sind die beiden Gitterstrukturen 11, 12 kongruent, so dass nur die Figur 18 oben dargestellte erste Gitterstruktur 11 sichtbar ist. Die zweite Gitterstruktur 12 ist in der abgewickelten Darstellung unter der ersten Gitterstruktur 11 angeordnet. Im rohrförmigen (dreidimensionalen) Körper 10 ist die erste Gitterstruktur 11 mit dem Netz 19 radial außen und die zweite Gitterstruktur 12 des Trägers 18 radial innen angeordnet.

Der Übergang vom Netz 19 zu den Strängen im Bereich der Spitze der ersten Gitterstruktur 11 erfolgt dergestalt, dass jeweils vier Einzeldrähte des Netzes 19 bzw. vier Einzelstränge des Netzes 19 zu zwei Strängen zusammengefasst sind, die wiederum in proximaler Richtung jeweils zu einem Strang zusammengefasst sind, aus dem die Gitterstruktur der Spitze gebildet ist. Eine andere Anzahl von Drähten bzw. Strängen, die jeweils sukzessiv miteinander kombiniert bzw. zusammengefasst sind, ist möglich. Die in proximaler Richtung geführten Stränge verlaufen in die Abschlusskante 46 und sind dort gemäß DE 10 2009 056 450 verbunden.

Der Bereich der Spitze des Trägers 19 ist entsprechend aufgebaut, wobei die Gitterstruktur 12 des Trägers 18 aus Einzeldrähten mit einem größerem Durchmesser als die Einzeldrähte der ersten Gitterstruktur 11 gebildet ist. Alternativ kann die zweite Gitterstruktur 12 auch aus mehreren Strängen jeweils bestehend aus Einzeldrähten gebildet sein.

Im Bereich der Spitze verlaufen also die Stränge bzw. Drähte der beiden Gitterstrukturen 11, 12 parallel und sind deckungsgleich. Dasselbe gilt für das distale Ende 115.

Im Bereich des Netzes 19 verlaufen die Drähte bzw. Stränge ebenfalls parallel, sind aber nicht deckungsgleich. Vielmehr überlappen die Drähte des Netzes 19 die vom Träger 18 gebildeten Gitterzellen.

Die punktuelle Verbindung zwischen der ersten Gitterstruktur 11 und der zweiten Gitterstruktur 12 erfolgt bei dem Ausführungsbeispiel gemäß Figur 18 außerhalb der beiden Gitterstrukturen 11, 12. Konkret sind die beiden Gitterstrukturen 11, 12 im Bereich der beiden gemeinsamen Endstränge 43, 44 verbunden, in denen alle Drähte der jeweiligen Gitterstrukturen 11, 12 zusammengeführt sind. Die beiden Endstränge 43, 44 sind in Figur 19 im Querschnitt dargestellt. Die Verbindung erfolgt durch die Hülse 17. Anstelle der Hülse 17 können die beiden Endstränge 43, 44 anderweitig verbunden sein. Beispielsweise können die dünneren Drähte der ersten Gitterstruktur 11, die im Ausführungsbeispiel gemäß Figur 18 die Außenlage bildet, auf den dickeren Drähten des Träger 18 angebracht und z.B. verdrillt sein. Beispielsweise sind die dickeren Drähte des Trägers 18 in einem Innenstrang verdrillt. Die dünneren Drähte des Netzes 19 bzw. der ersten Gitterstruktur 11 sind radial außen auf dem Innenstrang verdrillt. Andere Verbindungsarten sind möglich. Zusätzlich oder alternativ können die Drähte durch eine Hülse verbunden sein, wie beispielsweise durch die Hülse 17 gemäß Figur 19. Die Hülse kann auf die Drähte gecrimpt und/oder mit den Drähten verschweißt sein. Die Drähte können dabei wie vorstehend beschrieben verdrillt und verbunden sein. Alternativ können die Drähte auch lose nebeneinander angeordnet sein. Die Stränge beider Gitterstrukturen 11, 12 können auch seitlich nebeneinander angeordnet sein, wobei die Hülse 17 beide Stränge umschließt und verbindet, wie in Figur 19 dargestellt. Die Hülse 17 kann im Querschnitt kreisförmig oder oval sein, wie im Beispiel gemäß Figur 19 dargestellt. Die ovale Kontur begünstigt die Apposition an die Gefäßwand.

Durch die punktuelle Verbindung der beiden Gitterstrukturen 11, 12 außerhalb der Fläche der Gitterstrukturen 11, 12 wird erreicht, dass die Gitterstrukturen 11, 12 lose übereinander angeordnet und damit relativ zueinander beweglich sind. Gleichzeitig sind die beiden Gitterstrukturen 11, 12 ausgerichtet, so dass die Muster der Gitterstrukturen 11, 12 in Kombination miteinander ein übergeordnetes gemeinsames Muster bilden, wie in Figur 18 gut erkennbar.

Die Verbindung zwischen den beiden Gitterstrukturen 11, 12 bzw. den beiden Geflechten kann auch im Bereich der Schlaufen stattfinden. Z.B. können die dünnen Drähte des Netzes 19 um die dickeren Drähte des Trägers 18 gewickelt bzw. mit diesen verdrillt sein. Dies gilt für jede Drahtanzahl. Beispielsweise besteht jede Schlaufe des Netzes 19 aus zwei Drähten, die um den einzigen Draht der Schlaufe des Trägers 18 gewickelt sind. Eine andere Anzahl von Drähten bzw. Drahtkombinationen ist möglich. Die Verbindung der Drähte durch Hülsen ist auch möglich.

Weitere Möglichkeiten der punktuellen Verbindung der beiden Gitterstrukturen 11, 12 besteht darin, dass die Verbindung nur im Bereich der Abschlusskante 46, beispielswerise durch Verdrillen erfolgt. Eine weitere punktuelle Verbindung kann dadurch erreicht werden, dass die Drähte der beiden Gitterstrukturen 11, 12 im gesamten Schrägbereich bzw. im gesamten Bereich der Spitze verdrillt oder anderweitig verbunden, beispielsweise verschweißt sind. Der Bereich der Spitze endet dort, wo die beiden Gitterstrukturen 11, 12 in den Bereich der geschlossenen zylindrischen Mantelfläche übergehen. Das distale Ende kann in entsprechender Weise eine punktuelle Verbindung zwischen den beiden Gitterstrukturen 11, 12 aufweisen.

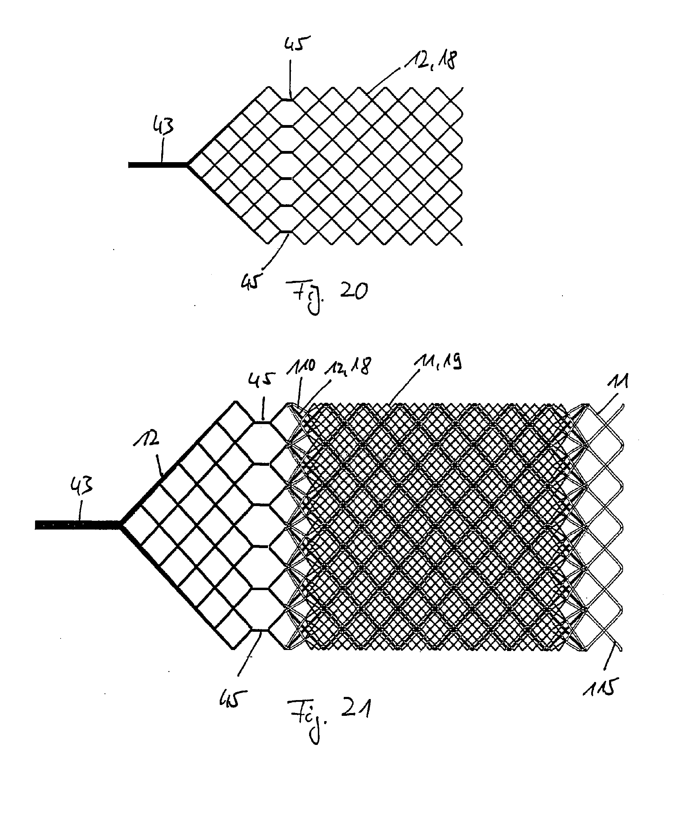

Ein weiteres Ausführungsbeispiel für eine erfindungsgemäße Vorrichtung ist in Figur 21 gezeigt, bei der ein Träger 18 gemäß Figur 20 verwendet ist. Der Träger 18 gemäß Figur 20 ist dazu angepasst, in einem mittleren Bereich mit einem weiteren Geflecht, insbesondere einer weiteren Gitterstruktur verbunden zu sein. Dazu weist die zweite Gitterstruktur 12 des Trägers 18 Festankerungsstellen 45 auf, an denen die erste Gitterstruktur 11 mit dem Netz 19 befestigt werden kann. Dabei entspricht der Grundaufbau der Gitterstruktur 12 des Trägers 18 dem Grundaufbau gemäß Figur 18 bzw. DE 10 2009 056 450, zumindest was den Bereich der Spitze angeht.

Im Bereich der Festankerungsstellen 45 sind die Drähte des Trägers 18 verdrillt und verlaufen im Wesentlichen parallel zur Längsachse der Vorrichtung.

Die kombinierte Struktur ist in Figur 21 dargestellt, wobei die erste Gitterstruktur 11 mit dem Netz 19 im rohrförmigen (dreidimensionalen) Körper 10 radial außen und der Träger 18 radial innen angeordnet ist. Die erste Gitterstruktur 11 mit dem Netz 19 überlappt die zweite Gitterstruktur 12 teilweisen, wobei im Bereich der Spitze bzw. Abschlusskante 46 keine Überlappung stattfindet. Deshalb ist in Figur 21 im Bereich der Spitze die Gitterstruktur 12 des Trägers 18 sichtbar. Wie bei dem Ausführungsbeispiel gemäß Figur 18 setzt sich die Gitterstruktur 12 des Trägers 18 im Bereich des Netzes 19 fort und ist durch die dickeren Drähte oder Stränge, die schwarz gekennzeichnet sind, gut unter dem Netz 19 sichtbar. Am distalen Ende 115 überdecken die Endschlaufen der ersten Gitterstruktur 11 das distale Ende der zweiten Gitterstruktur 12 des Trägers 18.

Die punktuelle Verbindung der beiden Gitterstrukturen 11, 12 erfolgt dadurch, dass im Bereich der verdrillten Elemente des Trägers bzw. der Festankerungsstellen 45 die Drähte der Netzstruktur 19 zusammenlaufen. Im Bereich der Festankerungsstellen 45 verlaufen die Drähte beider Gitterstrukturen 11, 12 parallel zueinander und sind parallel zur Achse des Stents orientiert. Die Drähte können im Bereich der Festankerungsstellen 45 verdrillt oder anderweitig verbunden sein, beispielsweise durch Schweißen, Kleben, Löten oder durch ein separates Mittel, wie eine Verbindungshülse, insbesondere eine c-förmige Verbindungshülse. Die c-förmige Verbindungshülse hat den Vorteil, dass die beiden Systeme bzw. Gitterstrukturen 11, 12 bei der Herstellung zunächst überlappt werden können und dann durch die Hülse verbunden werden. Alternativ können die dickeren Drähte des Trägers 18 von den dünneren Drähten des Netzes 19 umwickelt bzw. mit diesen verdrillt werden und zwar im Bereich der Festankerungsstellen 45.

Alternativ kann die Verbindung auch an Überkreuzungen des Trägers 19 erfolgen. Diesen Ausführungsformen ist gemeinsam, dass die Verbindung der beiden Gitterstrukturen 11, 12 punktuell, d.h. nicht über die gesamte Fläche der beiden Gitterstrukturen erfolgt, sondern nur in einem begrenzten Bereich. Bei der punktuellen Verbindung gemäß Figur 21 handelt es sich um eine Linienverbindung in Umfangsrichtung, die sich aus einzelnen Verbindungspunkten bzw. Verbindungsstellen zusammensetzt. Die Verbindungspunkte sind beispielsweise an den Festankerungsstellen 45 gebildet, wo die beiden Gitterstrukturen lokal begrenzt miteinander verbunden sind.

Das distale Ende des Netzes 19 kann offene Enden aufweisen. Der Vorteil dabei ist die einfache Herstellbarkeit. Das distale Ende kann aber auch geschlossene Schlaufen aufweisen. Zusätzlich zu der proximalen Verbindung im Bereich der Festankerungsstellen 45 kann auch am distalen Ende des Netzes eine punktuelle Verbindung erfolgen.

Die Erfindung eignet sich für endovaskuläre Eingriffe, insbesondere für die Behandlung von Aneurysmen in Blutgefäßen. Vorzugsweise ist die medizinische Vorrichtung dazu als Stent ausgebildet. Dabei ist die Erfindung nicht auf eine medizinische Vorrichtung oder einen Stent eingeschränkt, der geflochtene Gitterstrukturen umfasst. Vielmehr können die Gitterstrukturen auch durch Schneiden einer entsprechenden Struktur aus einem Vollmaterial, insbesondere einem rohrförmigen Vollmaterial, gebildet sein. Die Verwendung von Gittergeflechten bzw. Drahtgeflechten ist im Hinblick auf die bevorzugte hohe Feinmaschigkeit vorteilhaft.

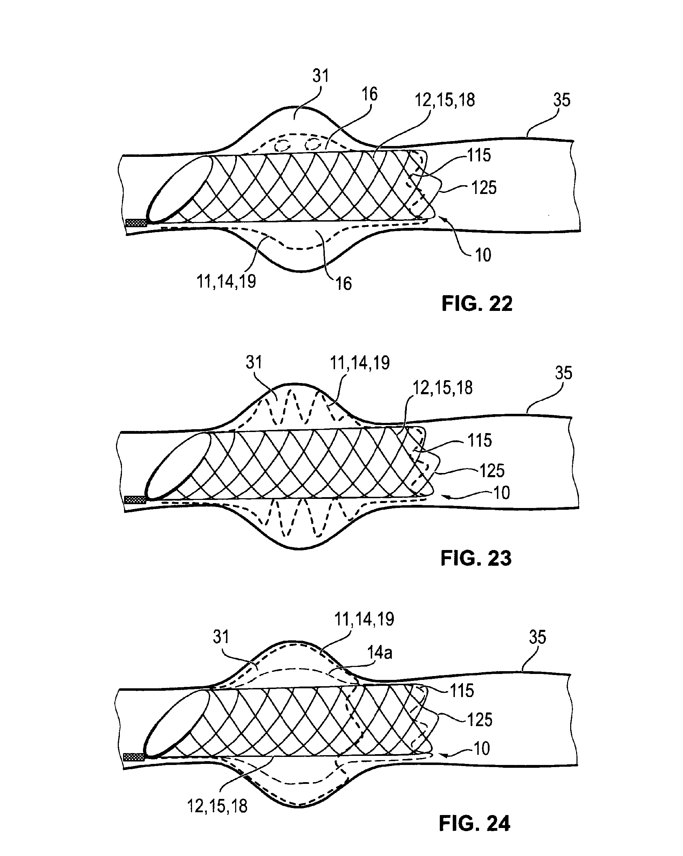

In den Figuren 22 und 23 ist dargestellt, dass sich das Mehrfachgeflecht mit in axialer - und radialer - Richtung relativbeweglichem Außengeflecht bzw. relativbeweglicher Außenschicht 14 zur Behandlung unterschiedlicher Arten von Aneurysmen eignet. Wie dargestellt, eignet sich das Mehrfachgeflecht zur Behandlung fusiformer Aneurysmen, die sich spindelförmig auf dem gesamten Gefäßumfang ausbilden. Bei einem fusiformen Aneurysma bildet die Innenschicht 15 bzw. die zweite Gitterstruktur 12, wie vorstehend beschrieben, den Träger 18, der das System im Gefäß positioniert. Die Innenschicht 15 liegt, wie in Fig. 22 gezeigt, stromauf- und stromabwärts des zu behandelnden Aneurysmas 31 an der Gefäßwand 35 an. Die Außenschicht 14 weitet sich gleichmäßig auf dem gesamten Umfang des rohrförmigen Körpers 10 auf und hebt sich von diesem ab, wie in Fig. 22 gezeigt. Dadurch bildet sich ein ringförmiger Spalt 16 im Bereich des fusiformen Aneurysmas, der für die Verwirbelung der Blutströmung in diesem Bereich sorgt. Die lokal begrenzte Befestigung der Außenschicht 14 einerseits und die geringe Steifigkeit der Außenschicht 14 andererseits ermöglichen die spindelförmige Aufweitung der Außenschicht 14, so dass die Außenschicht 14 zumindest teilweise in das fusiforme Aneurysma hineinragt. Die Möglichkeiten der lokal begrenzten Befestigung sind vorstehend beschrieben und werden im Zusammenhang mit diesem Ausführungsbeispiel offenbart. Die Steifigkeit der Außenschicht 14 ist geringer als die Steifigkeit der Innenschicht 15. Die Außenschicht 14 ist flexibler als die Innenschit 15.

Bei einem seitlichen Aneurysma (sakkuläres Aneurysma) bewegt sich die Außenschicht 14 seitlich von der Innenschicht 15 weg, wie beispielsweise in Fig. 12 dargestellt. Die Außenschicht 14 wird also von der Seite, auf der sich kein Aneurysma befindet, weggedrückt und wölbt sich in den Hals des Aneurysmas hinein.

Die im Zusammenhang mit Figur 22 beschriebene Funktionsweise des Mehrfachgeflechts wird in Zusammenhang mit allen Ausführungsbeispielen offenbart und beansprucht.

Die Auswölbung bzw. Ausweitung der Außenschicht 14 bzw. des äußeren Geflechts kann sich durch eine entsprechende Konditionierung im Rahmen einer Wärmebehandlung automatisch einstellen, wenn der rohrförmige Körper 10 aus dem Katheter entlassen wird. Das bedeutet, dass das äußere Geflecht bzw. die Außenschicht 14 im Ruhezustand ausgeweitet ist, sich also von der Innenschicht 15 abhebt. Dabei kann die Ausweitung des äußeren Geflechts einem mittleren Bereich des äußeren Geflechts aufgeprägt sein bzw. ausschließlich einem mittleren Bereich des äußeren Geflechts aufgeprägt sein. Die Form der Ausweitung kann, wie in Fig. 22 dargestellt, zur Behandlung eines fusiformen Aneurysmas angepasst sein und sich ringförmig um den rohrförmigen Körper 10 erstrecken bzw. eine Spindelform aufweisen. Alternativ kann die Form der Ausweitung an die Behandlung eines sakkulären Aneurysmas angepasst sein und sich nur seitlich bzw. nur auf einer Seite des Körpers 10 ausbilden. Die Außenschicht 14 bzw. das äußere Geflecht kann beispielsweise auf einem entsprechend gewölbten Dorn geformt und wärmebehandelt werden. Die Geflechtenden bzw. die Endbereiche des Geflechts der Außenschicht 14 bleiben in Kontakt mit dem Geflecht der Innenschicht 15. Das bedeutet, dass die Endbereiche der Außenschicht 14 sich auf derselben Ebene bzw. derselben Mantelfläche befinden, wie das Geflecht der Innenschicht 15. Im mittleren Bereich bzw. allgemein zwischen den Endbereichen hebt sich das Geflecht der Außenschicht 14 von der Innenschicht 15 ab.

Alternativ oder zusätzlich kann sich die Ausweitung, wie vorstehend beschrieben, durch den unterschiedlichen Flechtwinkel zwischen der Innenschicht 15 und der Außenschicht 14 ergeben bzw. zwischen den jeweiligen Geflechten.

Die vorstehend beschriebene zwangsweise Ausweitung der Außenschicht 14 durch eine entsprechende Wärmebehandlung wird im Zusammenhang mit allen Ausführungsbeispielen als mögliche Option offenbart und beansprucht. Ferner wird in diesem Zusammenhang offenbart und beansprucht, dass zumindest die Außenschicht 14 bzw. das Geflecht der Außenschicht 14 aus einem Formgedächtniswerkstoff hergestellt ist, beispielsweise aus Nitinol oder einem anderen im Bereich der Medizintechnik üblicherweise verwendeten Formgedächtniswerkstoff, der eine wärmeinduzierte Formänderung ermöglicht. Alternativ kann die erzwungene Formänderung auch durch eine elastische Verformung des Geflechts der Außenschicht 14 erreicht werden.

Wie vorstehend erläutert, kann sich das Geflecht der Außenschicht 14 bzw. das äußere Geflecht von der Innenschicht 15 abheben, weil die Außenschicht 14 relativ weich bzw. flexibler als die Innenschicht 15 ist und sich in der Strömung bewegen kann. Alternativ kann das Geflecht der Außenschicht 14 eine stabile Struktur aufweisen. Insbesondere kann das Geflecht der Außenschicht 14 Verstärkungsdrähte aufweisen, die die radiale Beweglichkeit des Geflechts des Geflechts der Außenschicht 14 einschränken. Dadurch bildet sich ein stabiler Spalt zwischen der Außenschicht 14 und der Innenschicht 15, wobei sich das Geflecht der Außenschicht 14 nicht oder nur wenig in der Blutströmung bewegt. Bei dieser Ausführungsform entspricht die radiale Stabilität bzw. Steifigkeit der Außenschicht 14 in etwa der radialen Stabilität bzw. Steifigkeit der Innenschicht 15. Das bedeutet, dass in etwa dieselben Kräfte zur Aufweitung der Außenschicht 14 und zur Aufweitung der Innenschicht 15 erforderlich sind. Das stabile Außengeflecht bzw. die stabile Außenschicht 14 eignet sich insbesondere im Zusammenhang mit der vorstehend beschriebenen Ausführungsform, bei der die Form der Außenschicht 14 im Ruhezustand radial nach außen gewölbt ist bzw. bei der die Wölbung nach außen aufgeprägt und der Spalt 16 zwischen der Außenschicht 14 und der Innenschicht 15 sich beim Entlassen aus dem Katheter automatisch bildet. Die automatische Ausbildung des Spaltes 16 bedeutet generell, dass die Aufweitung der Außenschicht 14 durch innere Kräfte oder zumindest überwiegend durch innere Kräfte der Außenschicht 14 bewirkt wird. Dieser Vorgang kann durch die von der Blutströmung aufgebrachten äußeren Kräfte unterstützt werden. Die Aufweitung ist aber bei dieser Ausführungsform in erster Linie auf die Eigenschaften des Formgedächtniswerkstoffes und die damit verbundenen inneren Kräfte zurückzuführen.

In Figur 23 ist ein weiteres Ausführungsbeispiel der Erfindung gezeigt, bei dem das Geflecht der Außenschicht 14 bzw. generell die Außenschicht 14 eine im Querschnitt wellenförmige Kontur aufweist. Allgemein variiert der Abstand zwischen der Außen- und Innenschicht 14, 15, wobei der Abstand abwechselnd zuund abnimmt. Die wellenförmige Kontur verlangsamt besonders effektiv die Strömung im Aneurysma 31. Die wellenförmige Struktur kann wenigstens zwei Wellenberge bzw. -spitzen und ein dazwischen liegendes Wellental aufweisen. Bei dem Ausführungsbeispiel gemäß Figur 23 sind vier Spitzen der wellenförmigen Kontur ausgebildet. Eine andere Anzahl von Spitzen bzw. Wellenbergen ist möglich. Wie in Fig. 23 zu erkennen, sind die Wellenberge unterschiedlich hoch. In proximaler und distaler Richtung des rohrförmigen Körpers 10 nimmt die Höhe der Wellenberge ab. Im mittleren Bereich der wellenförmigen Kontur sind die Wellenberge am höchsten. Die äußere Kontur der Wellenberge, die durch deren Spitzen bzw. von der Innenschicht 15 am weitesten entfernten Abschnitte bestimmt ist, entspricht in etwa der Form des zu behandelnden Aneurysmas. Die Außenkontur (Hüllfläche) der wellenförmigen Kontur ist an die Aneurysmenform angepasst.

Die Wellentäler können zumindest teilweise von der Innenschicht 15 beabstandet sein. Alle Wellentäler können von der Innenschicht 15 beabstandet sein. Alternativ können die Wellentäler zumindest teilweise die Innenschicht 15 berühren. Alle Wellentäler können die Innenschicht 15 berühren.

Die Kontaktstellen zwischen den Wellentälern und der Innenschicht 15 können durch Verbindungen zwischen den Geflechten der Außenschicht 14 und der Innenschicht 15 hergestellt sein. Dies gilt für einen Teil der Wellentäler oder für alle Wellentäler. Das Geflecht der Außenschicht 14 kann an einigen, in axialer Richtung voneinander beabstandeten Stellen mit dem Geflecht der Innenschicht 15 verbunden sein. Dies kann beispielsweise durch Hülsen, Crimphülsen oder durch andere Verbindungstechniken, beispielsweise stoffschlüssige Verbindungen erreicht werden. Die Hülsen können beispielsweise C-förmig sein und nachträglich, d.h. nach dem Flechten zur Verbindung der beiden Geflechte bzw. der Mehrfachgeflechte angebracht werden.

Alternativ oder zusätzlich kann die wellenförmige Struktur durch eine Wärmebehandlung vorkonditioniert werden, so das sich die wellenförmige Struktur im Ruhezustand einstellt. Hierfür wird ein an sich bekannter Formgedächtniswerkstoff, wie Nitinol, verwendet. Eine rein mechanische Formgebung ist möglich, wobei sich die Wellenform im Katheter streckt und beim Entlassen wieder den wellenförmigen Ruhezustand einnimmt.

Eine besonders wichtige Ausgestaltung dieses Ausführungsbeispiels, auf das die Erfindung aber nicht eingeschränkt ist, besteht darin, dass diejenigen Bereiche, die radial nach außen expandieren sollen, eine andere Flechtstruktur aufweisen, als diejenigen Bereiche der wellenförmigen Kontur bzw. der wellenförmigen Struktur, die die Wellentäler bilden, insbesondere diejenigen Bereiche, die in Kontakt mit dem Geflecht der Innenschicht 15 bleiben sollen. Beispielsweise kann der Flechtwinkel in den Bereichen, die sich abheben sollen, insbesondere im Bereich der Wellenberge kleiner sein, als der Flechtwinkel der Bereiche, die sich nicht abheben sollen, insbesondere im Bereich der Wellentäler. Ein Geflecht, das einen relativ kleinen Flechtwinkel aufweist, lässt sich vergleichsweise leicht ausweiten. Beim Ausweiten erhöht sich der Flechtwinkel aufgrund der axialen Komprimierung bei der Ausweitung. Diejenigen Bereiche, die radial stabiler sind und sich daher weniger gut ausweiten lassen, weisen einen größeren Flechtwinkel auf. Dies sind die Bereiche, in denen sich die Wellentäler der Struktur einstellen. Beispielsweise kann der Flechtwinkel im Bereich der Wellentäler größer als 45° und im Bereich der Wellenberge kleiner als 45° sein.

Die Bereiche mit kleinem Flechtwinkel bzw. kleinerem Flechtwinkel als in anderen Bereichen gehen mit einer geringeren axialen Verkürzung bei der Expansion einher. Dies bedeutet eine größere radiale Ausweitung bei geringerer axialer Kompression. Das führt dazu, dass sich bei einer axialen Komprimierung der Innenschicht die Bereiche mit dem kleinen Flechtwinkel (Wellenberge) nach Außen ausweiten müssen, damit die Struktur sich zugleich verkürzt. Die Außenschicht muss sich tatsächlich verkürzen, wenn sie distal mit der Innenschicht verbunden ist oder wenn die Innenschicht sie nach außen an die Gefäßwand drückt und daher blockiert.

Die vorstehend genannten Möglichkeiten zur Ausbildung der wellenförmigen Struktur können teilweise oder auch alle miteinander kombiniert sein. Beispielsweise kann sich die wellenförmige Struktur aus einer Kombination aus Wärmebehandlung (Vorprägung der Kontur) und/oder unterschiedlichen Flechtwinkeln und/oder mechanischen Verbindungen zwischen der Innen- und der Außenschicht 15, 14 ergeben.

Im Zusammenhang mit der Versetzung der Außenschicht 14 bezogen auf die Innenschicht 15 wird offenbart, dass die Geflechte auch im Ruhezustand voneinander versetzt sein können. Das bedeutet, dass das proximale Ende des einen Geflechts in axialer Richtung versetzt zum proximalen Ende des anderen Geflechts angeordnet ist. Zusätzlich oder alternativ können auch die distalen Geflechtenden der beiden Geflechte bzw. der Mehrfachgeflechte in axialer Richtung im Ruhezustand voneinander versetzt sein. Konkret können das proximale Ende des Geflechts der Außenschicht 14 und/oder das distale Ende des Geflechts der Außenschicht 14 jeweils bezogen auf die proximalen bzw. distalen Enden des Geflechts der Innenschicht 15 axial nach innen versetzt angeordnet sein.

Das Geflecht der Außenschicht 14 kann mindestens 1,5mal so viele Drähte aufweisen wie das Geflecht der Innenschicht 15, insbesondere mindestens 2mal so viele Drähte, insbesondere mindestens 3mal so viele Drähte, insbesondere mindestens 4mal so viele Drähte wie das Geflecht der Innenschicht 15. Diese Drahtanzahlbereiche werden im Zusammenhang mit allen Ausführungsbeispielen offenbart und beansprucht.

Die wellenförmige Struktur hat den Vorteil der hohen axialen Komprimierbarkeit. Dadurch lässt sich das Geflecht der Außenschicht 14 gut an verschiedene Aneurysmenlängen und -breiten anpassen.

Der Spalt 16, der sich zumindest im Gebrauch zwischen dem Geflecht der Innenschicht 15 und dem Geflecht der Außenschicht 14 einstellt, geht am proximalen und/oder distalen Ende des Spalts 16 kontinuierlich in die Mantelfläche des rohrförmigen Körpers 10 über. Dies ist beispielsweise in Fig. 12 oder in Fig. 22 dargestellt. Dasselbe gilt im Fall der Behandlung eines sakkulären Aneurysmas in Umfangsrichtung. Auch hier geht der Spalt bzw. der abgehobene Geflechtbereich der Außenschicht 14 kontinuierlich in die Mantelfläche des Körpers 10 über. Etwas anderes ist der Fall bei der Behandlung eines fusiformen Aneurysmas, bei der sich ein in Umfangsrichtung erstreckender ringförmiger Spalt 16 zwischen der Innenschicht 15 und der Außenschicht 14 ausbildet bzw. vorkonditioniert ist. Die maximale Spaltbreite, d.h. der Abstand zwischen der Innenschicht 15 und der Außenschicht 14 beträgt 50% des expandierten Durchmessers des rohrförmigen Körpers. Der expandierte Durchmesser bezieht sich auf den freien Rohrkörper, auf den keine äußeren Kräfte wirken, der also nicht im Gefäß angeordnet ist. Die minimale Spaltbreite beträgt 5% des expandierten Durchmessers des rohrförmigen Körpers 10. Auch hier gilt die gleiche Definition des expandierten Durchmessers, wie vorstehend beschrieben. Zwischenwerte des vorstehend genannten Maximalbereichs sind möglich. Beispielsweise kann die Spaltbreite höchstens 45% des expandierten Durchmessers, insbesondere höchstens 40%, insbesondere höchstens 35%, insbesondere höchstens 30%, insbesondere höchstens 25%, insbesondere höchstens 20%, insbesondere höchstens 15%, insbesondere höchstens 10% des expandierten Durchmessers betragen. Der untere Spaltbereich kann wenigstens 5%, insbesondere wenigstens 10%, insbesondere wenigstens 15%, insbesondere wenigstens 20% des expandierten Durchmessers des rohrförmigen Körpers 10 betragen. Die vorstehend genannten Unter- und Obergrenzen können miteinander kombiniert werden.

Die Vorrichtung kann mehrere Lagen aufweisen, die sich auf dem Umfang des Körpers 10 schichtartig erstrecken und einander überlappen, insbesondere teilweise überlappen. Zwischen den einzelnen Lagen ist jeweils ein Spalt 16 gebildet. Bei drei Lagen bilden sich 2 Spalte, bei 4 Lagen 3 Spalte usw.

Besonders bei der Behandlung fusiformer Aneurysmen ist es vorteilhaft, wenn die äußere Lage bzw. die Außenschicht 14 nah an der Innenwandung des Aneurysmas liegt, oder sogar in Kontakt mit dieser kommt. Die Gitterstruktur der äußeren Lage stabilisiert dadurch die Wandung des Aneurysmas und verlangsamt die Strömung in der Nähe der Wandung.

Zusätzlich kann eine Zwischenlage bzw. Zwischenschicht 14a vorgesehen sein, die im Gebrauch von der inneren Lage bzw. der Innenschicht 15 und der äußeren Lage bzw. der Außenschicht 14 beabstandet ist. Die Zwischenlage ist im Gebrauch im Aneurysma angeordnet. Die innere Lage fluchtet mit dem Hals des Aneurysmas. Es können mehrere Zwischenlagen bzw. Zwischenschichten 14a vorgesehen sein, die in das Anaurysma hineinragen und die Strömung progressiv verlangsamen. Diese Ausführung eignet sich insbesondere für fusiformen Aneurysmen, weil diese schwer mit Coils gefüllt werden können. Dies bietet eine einfache Möglichkeit, Material in Form von Geflechtschichten in das Aneurysma einzubringen.

Die Geflechtschichten, welche in das Aneurysma hineinragen, bilden äußere Schichten, die als thrombenbildende Schichten zum Verschluss des Aneurysmas beitragen.

Die unterschiedliche Aufweitung bzw. der unterschiedliche Abhebungsgrad der Schichten und somit die Spaltbildung zwischen den einzelnen Schichten kann durch verschiedene Flechtwinkel der Schichten erreicht werden. Zusätzlich oder alternative kann die Spaltbildung bei Verwendung eines Formgedächtniswerkstoffes erreicht werden, indem den Schichten unterschiedliche Durchmesser durch eine geeignete, an sich bekannte Wärmebehandlung aufgeprägt werden.

Bei dem Beispiel gemäß Figur 24 ist die Außenschicht 14 zumindest teilweise in Kontakt mit der Aneurysmawand. Dabei befindet sich das distale Ende der Außenschicht 14 im Aneurysma oder zumindest am Ende des Aneurysmahalses. Der Vorteil dieser Konfiguration, bei der sich die Außenschicht 14 nicht über den Aneurysmahals hinaus in das Gefäß erstreckt, besteht darin, dass das distale Ende der Außenschicht 14 frei expandieren und die gesamte Außenschicht sich sicher an die Wandung des Aneurysma anlegen kann. Konstruktiv wird dies dadurch erreicht, dass die Außenschicht 14 axial kürzer als der Körper 10 und/oder eine optionale Zwischenschicht 14a ist. Die Außenschicht 14 kann um wenigstens 10%, insbesondere um wenigstens 20%, insbesondere um wenigstens 30%, insbesondere um wenigstens 40%, insbesondere um wenigstens 50%, insbesondere um wenigstens 60%, insbesondere um wenigstens 70% kürzer als der Körper 10 sein.

Das distale Ende der äußeren Lage bzw. der Außenschicht 14 kann sich alternativ wie die mittlere Lage distal über den Aneurysmahals hinaus erstrecken. Dabei wird die Lage vom inneren Geflecht bzw. der Innenschicht 15 gegen die Gefäßwand gedrückt und damit fixiert. Wenn das distale Ende durch die innere Lage bzw. Innenschicht 15 fixiert ist, wird die Expansion der äußeren Lage bzw. außenschicht 14 in das Aneurysma hinein stärker vom Flechtwinkel bestimmt.

Wenn also ein definierter Spalt 16 eingestellt werden soll, sind das distale und proximalen Ende zu blockieren. Hierzu wird eine entprechend lange Außenschicht 14 vorgesehen. Wenn die Expansion frei sein soll, um sich an die Wandung anzulegen, ist die Länge der Außenschicht 14 so zu wählen, dass keine Blockierung der Außenschicht 14 erfolgt.

In der Regel sind nur die proximalen Enden der Lagen bzw Schichten 14, 14a, 15 miteinander verbunden. Die Blockierung der distalen Ende, wenn erwünscht, erfolgt im Gebrauch durch Reibung. Diese Konstruktion ist technisch einfach. Das Geflecht verzerrt sich nur wenig. Wenn die Blockierung am distalen Ende durch Reibung erfolgt, können sich die Geflechte bei der Positionierung relativ zueinander verschieben und zwar am distalen Ende, was die Gefahr der Verzerrung reduziert und die Anpassung an die Anatomie begünstigt.

Wenn hingegen das distale Ende der Schichten verbunden ist, wird eine sichere Blockierung erreicht und die Spaltbildung ist präzise durch die unterschiedlichen Flechtwinkel steuerbar.

Diese Ausführungen beziehen sich auf den Fall sowohl, dass der Körper 10 aus nur 2 Lagen, der Innenschicht 15 und der Außenschicht 14, oder aus mehr als 2 oder 3 usw. Lagen besteht.

### Bezugszeichenliste

- 10: rohrförmiger Körper
- 11: erste Gitterstruktur
- 12: zweite Gitterstruktur
- 13: dritte Gitterstruktur
- 14: Außenschicht
- 14a: Zwischenschicht
- 15: Innenschicht
- 16: Spalt
- 17: Verbindungshülse
- 18: Träger
- 19: Netz
- 20: Zuführsystem
- 21: Coilkatheter
- 30: Blutgefäß
- 31: Aneurysma
- 32: Aneurysmenhals
- 34: Aneurysmenwand
- 35: Gefäßwand
- 36: Anströmbereich
- 40: herkömmlicher Aneurysmenstent
- 41: erster Draht
- 42: zweiter Draht
- 43: Endstrang der ersten Gitterstruktur 11
- 44: Endstrang der zweiten Gitterstruktur 12
- 45: Festankerungsstellen
- 46: Abschlusskante
- 110: proximales Ende der ersten Gitterstruktur 11
- 111: mittlerer Abschnitt
- 112: Draht der ersten Gitterstruktur 11
- 113: Übergangsabschnitt
- 115: distales Ende der ersten Gitterstruktur 11
- 116: Randabschnitt
- 120: proximales Ende der zweiten Gitterstruktur 12
- 122: Draht der zweiten Gitterstruktur 12
- 125: distales Ende zweiten Gitterstruktur 12
- 130: proximales Ende dritten Gitterstruktur 13
- 135: distales Ende dritten Gitterstruktur 13
- F_{G}: Gefäßströmung
- F_{W}: Wirbelströmung
- F_{D}: Durchgangsströmung
- B: Auswölbung
- E: Streckung des Blutgefäßes 30
- K: Verkürzung des Übergangsabschnitts 113
- P: Druck
- W: Aufweitung des Blutgefäßes 30
- R: Verjüngung

## Patentansprüche

1. Medizinische Vorrichtung zur Behandlung von Aneurysmen mit einem zumindest abschnittsweise rohrförmigen Körper (10), der von einem komprimierten Zustand in einen expandierten Zustand überführbar ist und eine Umfangswandung mit wenigstens einer ersten Gitterstruktur (11) und einer zweiten Gitterstruktur (12) umfasst, wobei die erste Gitterstruktur (11) und die zweite Gitterstruktur (12) separate Schichten (14, 15) der Umfangswandung bilden, die koaxial ineinander angeordnet und zumindest punktuell miteinander verbunden sind derart, dass die erste Gitterstruktur (11) und die zweite Gitterstruktur (12) wenigstens abschnittsweise relativ zueinander bewegbar sind,
**dadurchgekennzeichnet, dass**
die erste Gitterstruktur (11) und/oder die zweite Gitterstruktur (12) jeweils aus miteinander verflochtenen Drähten (112, 122) gebildet ist, wobei die Drähte (112) der ersten Gitterstruktur (11) einen kleineren Querschnittsdurchmesser als die Drähte (122) der zweiten Gitterstruktur (12) aufweisen.

2. Medizinische Vorrichtung nach Anspruch 1,
**dadurchgekennzeichnet, dass**
die erste Gitterstruktur (11) ein proximales Ende (110) aufweist, das mit einem proximalen Ende (120) der zweiten Gitterstruktur (12) verbunden ist derart, dass jeweils den proximalen Enden (110, 120) gegenüber angeordnete distale Enden (115, 125) der ersten und zweiten Gitterstruktur (11, 12) relativ zueinander bewegbar sind.

3. Medizinische Vorrichtung nach Anspruch 1 oder 2,
**dadurchgekennzeichnet, dass**
die erste Gitterstruktur (11) und die zweite Gitterstruktur (12) in einem Herstellzustand zumindest abschnittsweise gleiche oder voneinander verschiedene Flechtwinkel aufweisen.

4. Medizinische Vorrichtung nach Anspruch 3,
**dadurchgekennzeichnet, dass**
der Flechtwinkel der ersten Gitterstruktur (11) und/oder der zweiten Gitterstruktur (12) höchstens 70°, insbesondere höchstens 65°, insbesondere höchstens 60°, insbesondere höchstens 59°, insbesondere höchstens 57°, insbesondere höchstens 55°, insbesondere höchstens 52°, insbesondere höchstens 50°, beträgt.

5. Medizinische Vorrichtung nach einem der Ansprüche 1-4,
**dadurch gekennzeichnet, dass**
zwischen der ersten Gitterstruktur (11) und der zweiten Gitterstruktur (12) in einem radial expandierten Zustand des rohrförmigen Körpers (10) zumindest abschnittsweise ein Spalt (16) ausgebildet ist.

6. Medizinische Vorrichtung nach einem der Ansprüche 1-5,
**dadurch gekennzeichnet, dass**
die erste Gitterstruktur (11) und die zweite Gitterstruktur (12) jeweils geschlossene Maschen aufweisen, wobei die Größe der Maschen der ersten Gitterstruktur (11) von der Größe der Maschen der zweiten Gitterstruktur (12) verschieden ist.

7. Medizinische Vorrichtung nach einem der Ansprüche 1 - 6,
**dadurch gekennzeichnet, dass**
die erste Gitterstruktur (11) eine größere Anzahl an Drähten (112) als die zweite Gitterstruktur (12) aufweist und/oder dass die erste Gitterstruktur (11) eine Außenschicht (14) und die zweite Gitterstruktur (12) eine Innenschicht (15) des rohrförmigen Körpers (10) bildet.

8. Medizinische Vorrichtung nach einem der Ansprüche 1-7,
**dadurch gekennzeichnet, dass**
die erste Gitterstruktur (11) eine axiale Längserstreckung aufweist, die kleiner als eine axiale Längserstreckung der zweiten Gitterstruktur (12) ist derart, das die erste Gitterstruktur (11) die zweite Gitterstruktur (12) abschnittsweise, insbesondere um wenigstens 20 %, insbesondere um wenigstens 30 %, insbesondere um wenigstens 40 %, insbesondere um wenigstens 50 %, insbesondere um wenigstens 60 %, überdeckt.

9. Medizinische Vorrichtung nach einem der Ansprüche 1-8,
**dadurch gekennzeichnet, dass**
der rohrförmige Körper (10) eine dritte Gitterstruktur (13) aufweist, die gemeinsam mit der ersten Gitterstruktur (11) die Außenschicht (14) des rohrförmigen Körpers (10) bildet.

10. Medizinische Vorrichtung nach Anspruch 9,
**dadurch gekennzeichnet, dass**
die erste Gitterstruktur (11) an einem proximalen Ende (110) und die dritte Gitterstruktur (13) an einem distalen Ende (135) mit der zweiten Gitterstruktur (12) verbunden ist, die die Innenschicht (15) des rohrförmigen Körpers (10) bildet und/oder sich die erste Gitterstruktur (11) und die dritte Gitterstruktur (13) in einem radial komprimierten Zustand oder einem radial expandierten Zustand zumindest abschnittsweise überlappen.

11. Medizinische Vorrichtung nach einem der Anspruch 9 oder 10,
**dadurch gekennzeichnet, dass**
die erste Gitterstruktur (11) und die dritte Gitterstruktur (13) jeweils ein proximales Ende (110, 130) umfassen, das mit der zweiten Gitterstruktur (12) verbunden ist, wobei das proximale Ende (110) der ersten Gitterstruktur (11) von dem proximalen Ende (130) der der dritten Gitterstruktur (13) beabstandet angeordnet sind.

12. Medizinische Vorrichtung nach einem der Ansprüche 1-11,
**dadurch gekennzeichnet, dass**
die erste Gitterstruktur (11) einen mittleren Abschnitt (111) und zwei den mittleren Abschnitt begrenzende Randabschnitte (116) umfasst, wobei die erste Gitterstruktur (11) im mittleren Abschnitt (111) einen kleineren Flechtwinkel als in den Randabschnitten (116) aufweist.

13. Medizinische Vorrichtung nach einem der Ansprüche 7 - 14,
**dadurch gekennzeichnet, dass**
der Abstand zwischen der Außenschicht (14) und der Innenschicht (15) im expandierten Zustand des Körpers (10) variiert, wobei der Abstand zumindest abschnittsweise abwechselnd ab- und zunimmt.

14. Medizinische Vorrichtung nach Anspruch 13,
**dadurch gekennzeichnet, dass**
die Außenschicht (14) im expandierten Zustand des Körpers 10 zumindest abschnittsweise eine wellenförmige Kontur aufweist, und/oder dass die Außenschicht (14) alternierend angeordnete Wellenberge und Wellentäler aufweist, wobei zumindest ein Teil der, insbesondere alle, Wellentäler mit der Innenschicht (15) verbunden sind und/oder vorgeformt, insbesondere durch eine Wärmebehandlung vorgeformt, sind und/oder einen anderen Flechtwinkel als die Wellenberge aufweisen.

15. System für medizinische Anwendungen mit einer Vorrichtung nach Anspruch 1 und einem Zuführsystem (20), das ein flexibles Zuführelement, insbesondere einen Führungsdraht umfasst, wobei das Zuführelement mit der Vorrichtung gekoppelt oder koppelbar ist.

## Claims

1. A medical device for treatment of aneurysms, having a tubular body (10) in at least some sections, this body being convertible from a compressed state into an expanded state, and comprising a circumferential wall having at least one first lattice structure (11) and a second lattice structure (12), the first lattice structure (11) and the second lattice structure (12) forming separate layers (14, 15) of the circumferential wall, which are arranged coaxially one inside the other and are connected to one another in at least some points such that the first lattice structure (11) and the second lattice structure (12) can be moved in at least some sections relative to one another, **characterized in that** the first lattice structure (11) and/or the second lattice structure (12) is/are each formed from closely intertwined wires (112, 122), wherein the wires (112) of the first lattice structure (11) have a smaller cross-sectional diameter than the wires (122) of the second lattice structure (12).

2. The medical device according to Claim 1, **characterized in that** the first lattice structure (11) has a proximal end (110), which is connected to a proximal end (120) of the second lattice structure (12), such that distal ends (115, 125) of the first and second lattice structures (11, 12) are each movable relative to one another, said distal ends being disposed opposite the proximal ends (110, 120).

3. The medical device according to Claim 1 or 2, **characterized in that** the first lattice structure (11) and the second lattice structure (12) have the same or different angles of twist in at least some sections in an as-manufactured state.

4. The medical device according to Claim 3, **characterized in that** the angle of twist of the first lattice structure (11) and/or of the second lattice structure (12) amounts to at most 70°, at most 65°, in particular at most 60°, in particular at most 59°, in particular at most 57°, in particular at most 55°, in particular at most 52°, in particular at most 50°.

5. The medical device according to any one of Claims 1-4, **characterized in that** a gap (16) is formed in at least some sections between the first lattice structure (11) and the second lattice structure (12) in a radially expanded state of the tubular body (10).

6. The medical device according to any one of Claims 1-5, **characterized in that** the first lattice structure (11) and the second lattice structure (12) each have a closed mesh, wherein the size of the mesh of the first lattice structure (11) is different from the size of the mesh of the second lattice structure (12).

7. The medical device according to any one of Claims 1-6, **characterized in that** the first lattice structure (11) has a greater number of wires (112) than the second lattice structure (12) and/or the first lattice structure (11) forms an outer layer (14) and the second lattice structure (12) forms an inner layer (15) of the tubular body (10).

8. The medical device according to any one of Claims 1-7, **characterized in that** the first lattice structure (11) has an axial longitudinal extent, which is smaller than the axial longitudinal extent of the second lattice structure (12), such that the first lattice structure (11) covers the second lattice structure (12) in some sections, in particular by at least 20%, in particular by at least 30%, in particular by at least 40%, in particular by at least 50%, in particular by at least 60%.

9. The medical device according to any one of Claims 1-8, **characterized in that** the tubular body (10) has a third lattice structure (13), which, together with the first lattice structure (11), forms the outer layer (14) of the tubular body (10).

10. The medical device according to Claim 9, **characterized in that** the first lattice structure (11) is connected to the second lattice structure (12) on a proximal end (110) and the third lattice structure (13) is connected to the second lattice structure on a distal end (135), the second lattice structure forming the inner layer (15) of the tubular body (10) and/or the first lattice structure (11) and the third lattice structure (13) overlapping in at least some sections in a radially compressed state or in a radially expanded state.

11. The medical device according to any one of Claims 9 or 10, **characterized in that** the first lattice structure (11) and the third lattice structure (13) each have a proximal end (110, 130), which is connected to the second lattice structure (12), wherein the proximal end (110) of the first lattice structure (11) is arranged at a distance from the proximal end (130) of the third lattice structure (13).

12. The medical device according to any one of Claims 1-11, **characterized in that** the first lattice structure (11) comprises a central section (111) and two edge sections (116) that border the central section, wherein the first lattice structure (11) has a smaller angle of twist in the central section (111) than in the edge sections (116).

13. The medical device according to any one of Claims 7-14, **characterized in that** the distance between the outer layer (14) and the inner layer (15) varies in the expanded state of the body (10), wherein the distance alternately increases and decreases in at least some sections.

14. The medical device according to Claim 13, **characterized in that** the outer layer (14) has a wavy contour in at least some sections in the expanded state of the body and/or the outer layer (14) has crests and troughs of waves arranged in alternation, wherein at least one part of the troughs, in particular all of the troughs, are connected to the inner layer (15) and/or are preshaped, in particular preshaped by a heat treatment, and/or have a different angle of twist than the crests of the waves.

15. A system for medical applications having a device according to Claim 1 and a supply system (20), which comprises a flexible supply element, in particular a guide wire, wherein the guide element is or can be coupled to the device.

## Revendications

1. Dispositif médical de traitement d'anévrismes, comportant un corps de forme tubulaire au moins par endroits (10) qui peut être passé d'un état comprimé à un état dilaté et comprend une paroi circonférentielle présentant au moins une première structure grillagée (11) et une deuxième structure grillagée (12), la première structure grillagée (11) et la deuxième structure grillagée (12) constituant des couches séparées (14, 15) de la paroi circonférentielle, qui sont disposées coaxialement l'une dans l'autre et sont reliées entre elles du moins ponctuellement de manière à ce que la première structure grillagée (11) et la deuxième structure grillagée (12) soient mobiles du moins par endroits l'une par rapport à l'autre, **caractérisé en ce que** la première structure grillagée (11) et/ou la deuxième structure grillagée (12) sont formées respectivement de fils tressés entre eux (112, 122), les fils (112) de la première structure grillagée (11) ayant un diamètre de section transversale inférieur à celui des fils (122) de la deuxième structure grillagée (12).

2. Dispositif médical selon la revendication 1, **caractérisé en ce que** la première structure grillagée (11) a une extrémité proximale (110) qui est reliée à une extrémité proximale (120) de la deuxième structure grillagée (12) de manière à ce que les extrémités distales (115, 125) disposées respectivement à l'opposé des extrémités proximales (110, 120) des première et deuxième structures grillagées (11, 12) soient mobiles les unes par rapport aux autres.

3. Dispositif médical selon la revendication 1 ou 2, **caractérisé en ce que** la première structure grillagée (11) et la deuxième structure grillagée (12), en état de fabrication, présentent des angles de tressage égaux ou différents du moins par endroits.

4. Dispositif médical selon la revendication 3, **caractérisé en ce que** l'angle de tressage de la première structure grillagée (11) et/ou de la deuxième structure grillagée (12) est au plus de 70°, en particulier au plus de 65°, en particulier au plus de 60°, en particulier au plus de 59°, en particulier au plus de 57°, en particulier au plus de 55°, en particulier au plus de 52°, en particulier au plus de 50°.

5. Dispositif médical selon une des revendications 1 à 4, **caractérisé en ce que**, entre la première structure grillagée (11) et la deuxième structure grillagée (12), en état dilaté radialement du corps de forme tubulaire (10), un intervalle (16) est constitué du moins par endroits.

6. Dispositif médical selon une des revendications 1 à 5, **caractérisé en ce que** la première structure grillagée (11) et la deuxième structure grillagée (12) présentent respectivement des mailles fermées, la taille des mailles de la première structure grillagée (11) étant différente de la taille des mailles de la deuxième structure grillagée (12).

7. Dispositif médical selon une des revendications 1 à 6, **caractérisé en ce que** la première structure grillagée (11) présente un plus grand nombre de fils (112) que la deuxième structure grillagée (12) et/ou que la première structure grillagée (11) forme une couche extérieure (14) et la deuxième structure grillagée (12) une couche intérieure (15) du corps de forme tubulaire (10).

8. Dispositif médical selon une des revendications 1 à 7, **caractérisé en ce que** la première structure grillagée (11) présente une extension longitudinale axiale qui est inférieure à une extension longitudinale axiale de la deuxième structure grillagée (12) de manière à ce que la première structure grillagée (11) recouvre la deuxième structure grillagée (12) par endroits, en particulier à raison d'au moins 20 %, en particulier à raison d'au moins 30 %, en particulier à raison d'au moins 40 %, en particulier à raison d'au moins 50 %, en particulier à raison d'au moins 60 %.

9. Dispositif médical selon une des revendications 1 à 8, **caractérisé en ce que** le corps de forme tubulaire (10) présente une troisième structure grillagée (13) qui constitue avec la première structure grillagée (11) la couche extérieure (14) du corps de forme tubulaire (10).

10. Dispositif médical selon la revendication 9, **caractérisé en ce que** la première structure grillagée (11) est reliée par une extrémité proximale (110) et la troisième structure grillagée (13) par une extrémité distale (135) à la deuxième structure grillagée (12) qui constitue la couche intérieure (15) du corps de forme tubulaire (10) et/ou que la première structure grillagée (11) et la troisième structure grillagée (13), en état comprimé radialement ou en état dilaté radialement, se chevauchent du moins par endroits.

11. Dispositif médical selon une des revendications 9 ou 10, **caractérisé en ce que** la première structure grillagée (11) et la troisième structure grillagée (13) comprennent respectivement une extrémité proximale (110, 130) qui est reliée à la deuxième structure grillagée (12), l'extrémité proximale (110) de la première structure grillagée (11) étant disposée à distance de l'extrémité proximale (130) de celle de la troisième structure grillagée (13).

12. Dispositif médical selon une des revendications 1 à 11, **caractérisé en ce que** la première structure grillagée (11) comprend une section médiane (111) et deux sections périphériques (116) limitant la section médiane, la première structure grillagée (11) présentant dans la section médiane (111) un angle de tressage plus réduit que dans les sections périphériques (116).

13. Dispositif médical selon une des revendications 7 à 14, **caractérisé en ce que** la distance entre la distance entre la couche extérieure (14) et la couche intérieure (15) varie en état dilaté du corps (10), cette distance diminuant et augmentant alternativement du moins par endroits.

14. Dispositif médical selon la revendication 13, **caractérisé en ce que** la couche extérieure (14), en état dilaté du corps, présente, du moins par endroits, un contour de forme ondulée et/ou que la couche extérieure (14) présente des monts et des creux disposés en alternance, au moins une partie des creux, en particulier tous, étant reliés à la couche intérieure (15) et/ou préformés, en particulier préformés par un traitement thermique, et/ou présentant un angle de tressage autre que celui des monts.

15. Système destiné à des applications médicales, comportant un dispositif selon la revendication 1 et un système d'acheminement (20) qui comprend un élément d'acheminement souple, en particulier un fil de guidage, l'élément d'acheminement étant couplé ou pouvant être couplé au dispositif.
